# EUROPEAN PATENT APPLICATION

(11) **EP 4 512 427 A1**
(43) Date of publication of application: **26.02.2025**
(21) Application number: 23306420.3
(22) Date of filing: 25.08.2023
(51) Int. Cl.: A61K 47/68

(54) **ANTIBODY-DRUG CONJUGATES BASED ON MOLECULAR GLUE DEGRADERS AND USES THEREOF**

(71) Applicant: Mablink Bioscience, 69009 Lyon (FR); UNIVERSITE CLAUDE BERNARD - LYON 1, 69100 Villeurbanne (FR); Hospices Civils de Lyon, 69002 Lyon (FR); Institut National de la Santé et de la Recherche Médicale, 75013 Paris (FR); Centre Léon Bérard, 69008 Lyon (FR); Ecole Sup Chimie Phys Electroniq Lyon (CPE Lyon), 69100 Villeurbanne (FR); Centre National de la Recherche Scientifique, 75016 Paris (FR); Institut National des Sciences Appliquees de Lyon (Insa Lyon), 69100 Villeurbanne (FR)
(72) Inventor: JOSEPH, Benoît, 69100 VILLEURBANNE (FR); FOURNET, Guy, 69006 LYON (FR); DUMONTET, Charles, 69200 VENISSIEUX (FR); VIRICEL, Warren, 69280 SAINTE CONSORCE (FR); CONILH, Louise, 69006 LYON (FR)
(74) Representative: Plasseraud IP

(57) **Abstract**

The present disclosure relates to antibody-drug conjugates, wherein the drug is a pyrazolo[1 ,5-a][1 ,3,5]triazine derivative or a pyrazolo[1 ,5-a]pyrimidine derivative. In particular the drug can be a 2-substituted 8-halogeno-N4-(4-(pyridin-2-yl) benzyl) pyrazolo[1 ,5-a][1 ,3,5] triazine-2,4-diamine. Such antibody-drug conjugates are useful in particular in treating proliferative diseases including cancers.

## Description

### Technical field

The present disclosure relates to antibody-drug conjugates, wherein the drug (or "payload") is a molecular glue degrader which is a pyrazolo[1,5-a][1 ,3,5]triazine derivative or a pyrazolo[1 ,5-a]pyrimidine derivative. In particular the drug can be a 2-substituted 8-halogeno-N4-(4-(pyridin-2-yl) benzyl) pyrazolo[1,5-a][1,3,5] triazine-2,4-diamine. Such antibody-drug conjugates are useful in particular in treating proliferative diseases including cancers.

### Background

Antibody-drug conjugates (hereafter referred as "ADCs") are a new class of therapeutics, notably cancer therapeutics. Such ADCs comprise at least an antibody and a payload (e.g. an anticancer or immunostimulatory drug), both covalently bonded by a linker. ADCs combine the specificity of monoclonal antibodies with the potency of highly cytotoxic agents, potentially reducing the severity of side effects by preferentially targeting their payload to the tumour site. An efficient ADC should exhibit high tumor specificity, good cell-killing properties and low systemic toxicity. Within the context of toxicity, antibody used in ADCs needs to bind accurately and efficiency to the targeted antigen, meaning that the suitable target antigen is preferentially or exclusively expressed on targeted cancerous cells.

When designing an ADC, there is a need to covalently attach the final active drug ("payload") to the ligand targeting unit, while allowing the final release of the active drug unit by a selective cleavable enzymatic mechanism after cellular internalization or in the diseased tissue microenvironment. In this regard, several peptidase- and glycosidase-sensitive cleavable linker chemical strategies (associated with self-immolative chemistries) were developed. These cleavable linkers and their corresponding cleavage mechanisms are well known and have been described in several publications (e.g. Bargh JG et al., Chem. Soc. Rev., 2019,48, 4361-4374; Toki et al. J. Org. Chem. 2002, 67, 6, 1866-1872 ; Jeffrey et al. Bioconjugate Chem. 2006, 17, 3, 831-840).

As only a very small fraction of the administered ADC dose ultimately reaches the tumour tissue (in the order of 0.1%), payloads with very high cytotoxicity are required to achieve therapeutic efficacy. As a result, ADC payloads typically have an in vitro inhibitory IC₅₀ concentrations in the pico to low nanomolar range (see Drago et al., Nat Rev Clin Oncol, 2021, 18(6), 327-344). This high potency requirement explains in part why "conventional" ADC payloads belong to the tubulin inhibitor, DNA alkylator or topoisomerase I inhibitor class of drugs.

Molecular glue degraders are a class of molecules that promotes proximity-induced target protein degradation (TPD) in which protein of interest are linked to natural digestive enzymes (typically E3 ubiquitin ligases of the ubiquitin-proteasome system) and are destroyed. Molecular glue degraders are advantageous because they act via transient binding rather than competitive occupancy and dissociate after promoting polyubiquitination of the protein of interest. As a result, a single molecular glue degrader compound can therefore destroy many copies of a targeted protein, thus inactivating disease-relevant proteins. A recent review explaining this approach can be found in Lai and Crews, Nat Rev Drug Discov 2017, 16(2), 1474-1784. It should be noted that in contrast to their high molecular weight heterobifunctional proteolysis targeting chimeras (PROTACs) siblings, molecular glue degraders are smaller, less hindered, and less hydrophobic. As such, they are more amenable to bioconjugation to antibodies and antibody fragments.

ADC payload diversification is expected to play a key role in future ADC development, in order to overcome tumour resistance and propose treatment alternatives to relapsing patients that would be refractory to "conventional" ADC payloads. Therefore, developing ADCs based on "differentiated" molecular glue degrader payloads having high cell-killing potency (low nanomolar IC₅₀) would be highly desirable.

### Summary

The inventors surprisingly found that ADC comprising a pyrazolo[1 ,5-a][1 ,3,5]triazine derivative exhibit valuable pharmaceutical properties, in particular as molecular glue degraders, and are therefore indicated in therapy.

Consequently, in a first aspect, the present disclosure relates to an antibody-drug conjugate formula (I):

Ab-[L-D]p (I),

wherein :
Ab is an antibody or an antibody fragment,
L is a cleavable linker moiety bonded to Ab and D,
p is 1 or more, preferably from 1 to 8
D is a cytotoxic drug moiety of formula (II) bonded to L, 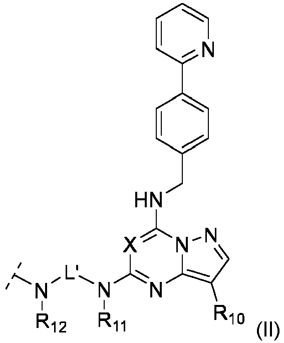 wherein
X is C or N, preferably N;
R10 is selected from the group consisting of H, Halogen, C₁-C₁₂ alkyl, C₁-C₁₂ haloalkyl, C₁-C₁₂ heteroalkyl, aryl having 6 to 10 ring atoms, C₃-C₈ cycloalkyl, heterocycloalkyl having 3 to 10 ring atoms, heteroaryl having 5 to 10 ring atoms, and combination thereof, said alkyl, heteroalkyl, aryl, cycloalkyl, heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl, preferably R10 is a halogen or a C₁-C₁₂ alkyl;
R11 is selected from the group consisting of H and C₁-C₆ alkyl, said alkyl being optionally substituted;
Or R11 is linked to an atom from the linker L' and forms a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl;
L' is a bond or a linker, preferably selected from C₁-C₂₀ alkylene, C₁-C₂₀ heteroalkylene, C₃-C₁₂ cycloalkylene, heterocyclylene having 5 to 10 ring atoms, arylene having 6 to 10 ring atoms, heteroarylene having 5 to 10 ring atoms, and any combination thereof; said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
R12 is selected from the group consisting of H, and C₁-C₁₂ alkyl;
Or R12 is linked to an atom from the linker L' and forms a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl;
   Or R11 and R12, together with the N atoms and the linker L' to which they are bonded, form a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl.;

In a second aspect, the disclosure relates to a pharmaceutical composition comprising an ADC of the present disclosure and a pharmaceutically acceptable carrier.

In a third aspect, the disclosure relates to an ADC of the present disclosure for use as a drug.

In a fourth aspect, the disclosure relates to an ADC of the present disclosure for use in treating cancer.

### Legends of the Figures

Figures 1A and 1B represent the in vitro cytotoxicity assays of halogenated compound 2384 and its isopropyl counterpart 2420 according to example 5.
Figures 2A and 2B represent the in vitro cytotoxicity assays of halogenated compound 2388 and its isopropyl counterpart 2428 according to example 5
Figure 3 represents the in vitro cytotoxicity assays of compound 2384 of the disclosure to the known glue degrader compound CR8 according to example 5.
Figure 4 represents the result of the kinase screening assay of the 2384 compound according example 7. Kinases inhibited at more than 90% are presented in the map as circles whose size is representative of percentage of inhibition.
Figure 5 represents the dose-response inhibition on top 25 selected kinases according to example 7.
Figure 6 represents the in vitro cytotoxicity assays of compounds of the invention in the presence or absence of pevonedistat (non-cytotoxic cullin NEDDylation inhibitor), according to example 8.
Figure 7 represents in vitro mechanistic studies performed on lead compound 2384 of the invention, according to example 9.
Figure 8 represents the in vitro cytotoxicity assays of ADCs based on compounds 2384 and 2420 according to example 12.
Figure 9 represents the in vitro cytotoxicity assays of ADCs based on compounds 2388 and 2428 according to example 12.
Figure 10 represents the in vitro cytotoxicity assays of an ADC based on compound 2384 and the trastuzumab-based commercially approved ADCs (Kadcyla^{®} and Enhertu^{®}) according to example 12.
Figure 11 represents the in vitro cytotoxicity assays of an ADC based on compound 2384 and a non-binding isotype control-based ADC according to example 12.
Figure 12 represents the in vitro cytotoxicity assays of trastuzumab (anti-HER2)-based ADCs based on compounds of the invention, according to example 12.
Figure 13 represents the in vitro cytotoxicity assays of farletuzumab_LALA (anti-FRα)-based ADCs based on compounds of the invention, according to example 12.
Figure 14 represents the in vitro cytotoxicity assays of trastuzumab (anti-HER2) and farletuzumab_LALA (anti-FRo)-based ADCs based on compounds of the invention incorporating dipeptide or glucuronide-cleavable modalities, according to example 12.
Figures 15A, 15B and 15C represents the in vitro bystander cell killing assay of ADCs according to example 13.
Figure 16A and 16B represents the in vivo efficacy of trastuzumab-based ADCs incorporating compounds 2384 and 2388 of the invention, according to example 14.
Figure 17 represents the in vivo efficacy of a trastuzumab-based ADC incorporating compound 2384 of the invention, *versus* the trastuzumab-based commercially approved ADC Enhertu^{®}, according to example 14.
Figure 18 represents the in vivo mouse tolerability of ADCs based on compounds 2384 and 2388 of the invention, according to example 15.

### Detailed description

### Definitions

As used herein, the terms "Cₓ-C_{y} alkyl", by itself or as part of another substituent, refer to a linear or branched alkyl functional group having x to y carbon atoms, preferably 1 to 12 carbon atoms, more preferably 1 to 6 carbon atoms. Suitable alkyl groups include methyl, ethyl, *n*-propyl, *i*-propyl, *n*-butyl, *i*-butyl, *s*-butyl and *t*-butyl, pentyl and its isomers (e.g. *n*-pentyl, *iso*-pentyl), and hexyl and its isomers (e.g. *n*-hexyl, *iso*-hexyl).

As used herein, the terms "C₃-C₁₂ cycloalkyl" refer to a saturated or unsaturated cyclic group having 3 to 12 carbon atoms, preferably 3 to 6. The cycloalkyl can have a single ring or multiple rings fused together. The cycloalkyl can also include spirocyclic rings. Suitable cycloalkyl groups include cyclopropyl, cyclobutyl, cyclopentyl and cyclohexyl.

As used herein, the term "halogen" refers to a fluoro (-F), chloro (-CI), bromo (-Br), or iodo (-I) group.

As used herein, the terms "C₁-C₆ haloalkyl" refer to a C₁-C₆ alkyl as defined herein that is substituted by one or more halogen group as defined herein. Suitable C₁-C₆ haloalkyl groups include trifluoromethyl and dichloromethyl.

As used herein, the terms "C₁-Cₓ heteroalkyl", refer to a straight or branched hydrocarbon chain consisting of 1 to x carbon atoms, preferably 1 to 20 carbon atoms, more preferably 1 to 12 carbon atoms, and at least one heteroatom, preferably from one to three, heteroatoms selected from the group consisting of O, N, Si and S, and wherein the nitrogen and sulfur atoms may optionally be oxidized (for example: a sulfoxide or a sulfone) and the nitrogen heteroatom may optionally be quaternized. The heteroatom(s) O, N and S may be placed at any interior position of the heteroalkyl group or at the position at which the alkyl group is attached to the remainder of the molecule.

As used herein, the terms "C₁-C₆ alkoxy" refer to a -O-alkyl group, wherein the alkyl group is a C₁-C₆ alkyl as defined herein. Suitable C₁-C₆ alkoxy groups include methoxy, ethoxy, propoxy.

As used herein, the terms "C₁-C₆ haloalkoxy" refer to a C₁-C₆ alkoxy group as defined herein, that is substituted by one or more halogen group as defined herein. Suitable haloalkoxy include trifluoromethoxy.

As used herein, the terms "C₁-Cₓ alkylene", used alone or as part of another substituent, refer to a divalent saturated, straight-chained or branched hydrocarbon group having 1 to 12 carbon atoms, preferably 1 to 6.

As used herein, the terms "C₁-Cₓ heteroalkylene", refer to a divalent heteroalkyl as defined above. For heteroalkylene groups, heteroatoms can also occupy either or both of the chain termini.

As used herein, the terms "aryl having 6 to 10 ring atoms" refer to a polyunsaturated, aromatic hydrocarbyl group having a single ring or multiple aromatic rings fused together, containing 6 to 10 ring atoms, wherein at least one ring is aromatic. The aromatic ring may optionally include one to two additional rings (cycloalkyl, heterocyclyl or heteroaryl as defined herein) fused thereto. Suitable aryl groups include phenyl, naphtyl and phenyl ring fused to a heterocyclyl, like benzopyranyl, benzodioxolyl, benzodioxanyl and the like.

As used herein, the terms "heteroaryl having 5 to 10 ring atoms" refer to a polyunsaturated, aromatic ring system having a single ring or multiple aromatic rings fused together or linked covalently, containing 5 to 10 atoms, wherein at least one ring is aromatic and at least one ring atom is a heteroatom selected from N, O and S. The nitrogen and sulfur heteroatoms may optionally be oxidized and the nitrogen heteroatoms may optionally be quaternized. Such rings may be fused to an aryl, cycloalkyl or heterocyclyl ring. Non-limiting examples of such heteroaryl, include: furanyl, thiophenyl, pyrrolyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, indolyl, isoindolyl, benzofuranyl, isobenzofuranyl, benzothiophenyl, isobenzothiophenyl, indazolyl, benzimidazolyl, benzoxazolyl, purinyl, benzothiadiazolyl, quinolinyl, isoquinolinyl, cinnolinyl, quinazolinyl and quinoxalinyl.

As used herein, the terms "heterocyclyl having 3 to 10 ring atoms" refer to a saturated or unsaturated cyclic group having 3 to 10 ring atoms, wherein at least one ring atom is a heteroatom selected from N, O and S. The nitrogen and sulfur heteroatoms may optionally be oxidized and the nitrogen heteroatoms may optionally be quaternized. The heterocycle can include fused or bridged rings as well as spirocyclic rings. Examples of heterocycle include, but are not limited to, tetrahydropyridyl, piperidinyl, morpholinyl, tetrahydrofuranyl, tetrahydrothienyl, piperazinyl, 1-azepanyl, imidazolinyl, 1,4-dioxanyl and the like.

As used herein, the terms "organyl group" refer to any organic substituent group, regardless of functional type, having one free valence at a carbon atom.

As used herein, the terms "optionally substituted" can refer to groups that can be substituted with one or more of the substituents independently selected from: C₁-C₂₀ alkyl, C₁-C₂₀ heteroalkyl, aryl having 6 to 10 ring atoms, C₃-C₈ cycloalkyl, heterocycloalkyl having 3 to 10 ring atoms, heteroaryl having 5 to 10 ring atoms, C₁-C₂₀ haloalkyl,-X, -R', -O⁻, -OR', =O, -SR', -S⁻, - NR'₂, -NR'₃, =NR', -CX₃, -CN, -OCN, -SCN, -NCS, -NO, -NO₂, =N₂, -NRC(=O)R', -C(=O)R', - C(=O)NR'₂, -SO₃⁻, -SOsH, -S(=O)₂R', -OS(=O)₂OR', -S(=O)₂NR', -S(=O)R', -C(=O)R', -C(=S)R', - CO₂R', -CO₂, -C(=S)OR', C(=O)SR', C(=S)SR', C(=O)NR'₂, C(=S)NR'₂, and C(=NR')NR'₂, where each X is independently a halogen: -F, -Cl, -Br, or -I; and each R' is independently -H, -C₁₋C₂₀ alkyl, -C₆-C₁₀ aryl, C₃-C₈ cycloalkyl, heterocycloalkyl having 3 to 10 ring atoms, or heteroaryl having 5 to 10 ring atoms.

The term "connector unit" refers to a component that connects different parts of the compound together, for example, the connector can connect Ab to a spacer, or a spacer to the amide function -CO-NR1-. The connector is a scaffold bearing attachment sites for components of the antibody-drug-conjugate, namely Ab, the spacer, the hydrophobicity masking entity, and/or the amide function -CO-NR1-.

From his knowledge, the one skilled in the art is capable to select a connector which is appropriate. Non-exhaustive listing of connectors includes: aminoacids, for example lysine, glutamic acid, aspartic acid, serine, tyrosine, cysteine, selenocysteine, glycine, homoalanine; amino alcohols; amino aldehydes; polyamines or any combination thereof. Advantageously, the connector unit X₁ and/or X₂ is one or more natural or non-natural aminoacids. In one embodiment, the connector unit X₁ and/or X₂ is selected from glutamic acid, lysine and glycine. The connector units X₁ and X₂ can be independently selected from the group consisting of one or more amino acid(s), one or more N-substituted amino acid, optionally substituted polyether, C₁-C₁₂ alkylene, arylene having 6 to 10 ring atoms, C₃-C₈ cycloalkylene, heterocycloalkylene having 5 to 10 ring atoms, heteroarylene having 5 to 10 ring atoms, C₂-C₁₀ alkenylene, and any combination thereof, said alkylene and alkenylene being optionally interrupted by one or more heteroatoms or chemical groups selected from -O-, -S-, -C(O)-, -NR"-, -C(O)NR"-, -NR"-C(O)-, -NR"-C(O)-NR‴-, -NR"-C(O)-O-, -O-C(O)NR"- and triazole,
and said alkylene, arylene, cycloalkylene, heterocycloalkylene, heteroarylene, and alkenylene being optionally substituted with one or more of the substituents selected from : halogen, oxo, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R‴, -NR"-(CO)-R', and -NR"R‴; R', R" and R‴ being independently selected from H and C₁-C₆ alkyl.

Examples of connector units include optionally substituted polyether, aminoacids, benzyl groups, amines, ketones,

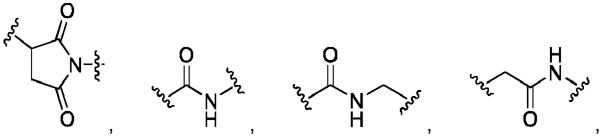

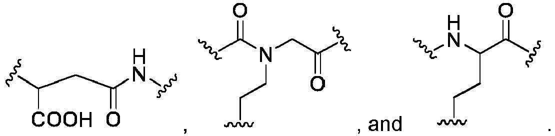

In particular, the connector unit can be divalent or trivalent. For example, X₂ can be a trivalent connector unit when the hydrophobicity masking entity K is present.

The term "aminoacids" refers to natural or non-natural aminoacids. The CO moiety of the -CONR1- or -CONR1'- group can be considered as part of the X₂ connector unit when X₂ consists of one or more aminoacids. Non-exhaustive listing of aminoacids includes lysine, glutamic acid, aspartic acid, serine, tyrosine, cysteine, selenocysteine, glycine, and homoalanine.

A spacer or a linker is a divalent arm that covalently binds two components of the antibody-drug-conjugate, such as the 2 connector units.

Non-exhaustive listing of spacer units or linker includes: alkylene, heteroalkylene (so an alkylene interrupted by at least one heteroatom selected from Si, N, O and S); alkoxy; polyether such as polyalkylene glycol and typically polyethylene glycol; one or more natural or non-natural aminoacids such as glycine, alanine, proline, valine, N-methylglycine; C₃-C₈ heterocyclo; C₃-C₈ carbocyclo; arylene, and any combination thereof, said alkylene, heteroalkylene alkoxy being optionally substituted. For example, a spacer is a divalent linear alkylene group, preferably (CH₂)₄.

For example, the spacer or the linker can be selected from the group consisting of -C₁-C₂₀ alkylene-, -C₁₋C₂₀ heteroalkylene-, -C₃-C₈ carbocyclo-, -O-(C₁ C₈ alkyl)-, -arylene-, -C₁₋C₂₀ alkylene-arylene-, -arylene-C₁-C₂₀ alkylene-, -C₁₋C₂₀ alkylene-(C₃-C₈ carbocyclo)-, -(C₃-C₈ carbocyclo)-C₁-C₂₀ alkylene-, -C₃-C₈ heterocyclo-, -C₁₋C₂₀ alkylene-(C₃-C₈ heterocyclo)-, - (C₃-C₈ heterocyclo)-C₁-C₂₀ alkylene-, -C₁₋C₂₀ alkylene-C(=O)-, -C₁₋C₂₀ heteroalkylene-C(=O)-, - C₃-C₈ carbocyclo-C(=O)-, -O-(C₁-C₈ alkyl)-C(=O)-, -arylene-C(=O)-, -C₁₋C₂₀ alkylene-arylene-C(=O)-, -arylene-C₁-C₂₀ alkylene-C(=O)-, -C₁₋C₂₀ alkylene-(C₃-C₈carbocyclo)-C(=O)-, -(C₃-C₈ carbocyclo)-C₁-C₂₀ alkylene-C(=O)-, -C₃-C₈ heterocyclo-C(=O)-, -C₁₋C₂₀ alkylene-(C₃-C₈heterocyclo)-C(=O)-, -(C₃-C₈ heterocyclo)-C₁-C₂₀ alkylene-C(=O)-, -C₁₋C₂₀ alkylene-NH-, -C₁-C₂₀ heteroalkylene-NH-, -C₃-C₈ carbocyclo-NH-, -O-(C₁-C₈ alkyl)-NH-, -arylene-NH-, -C₁₋C₂₀ alkylene-arylene-NH-, -arylene-C₁-C₂₀ alkylene-NH-, -C₁₋C₂₀ alkylene-(C₃-C₈ carbocyclo)-NH-, - (C₃-C₈ carbocyclo)-C₁-C₂₀ alkylene-NH-, -C₃-C₈heterocyclo-NH-, -C₁₋C₂₀ alkylene-(C₃-C₈ heterocyclo)-NH-, -(C₃-C₈ heterocyclo)-C₁-C₂₀ alkylene-NH-, -C₁₋C₂₀ alkylene-S-, -C₁-C₂₀ heteroalkylene-S -, -C₃-C₈carbocyclo-S -, -O-(C₁-C₈ alkyl)-)-S -, -arylene-S-, -C₁₋C₂₀ alkylene-arylene-S-, -arylene-C₁-C₂₀ alkylene-S-, -C₁₋C₂₀ alkylene-(C₃-C₈ carbocyclo)-S-, -(C₃-C₈ carbocyclo)-C₁-C₂₀ alkylene-S-, -C₃-C₈ heterocyclo-S-, -C₁₋C₂₀ alkylene-(C₃-C₈ heterocyclo)-S-, -(C₃-C₈ heterocyclo)-C₁-C₂₀ alkylene-S-, -C₁₋C₂₀ alkylene-O-C(=O)-, -C₃-C₈ carbocyclo-O-C(=O)-, -O-(C₁-C₈ alkyl)-O-C(=O)-, -arylene-O-C(=O)-, -C₁₋C₂₀ alkylene-arylene-O-C(=O)-, - arylene-C₁-C₂₀ alkylene-O-C(=O)-, -C₁₋C₂₀ alkylene-(C₃-C₈carbocyclo)-O-C(=O)-,-(C₃-C₈ carbocyclo)-C₁-C₂₀ alkylene-O-C(=O)-, -C₃-C₈ heterocyclo-O-C(=O)-, -C₁₋C₂₀ alkylene-(C₃-C₈heterocyclo)-O-C(=O)-, and -(C₃-C₈ heterocyclo)-C₁-C₂₀ alkylene-O-C(=O)-.

In the present disclosure, the dotted bond represents the attachment point of a moiety to the rest of the molecule.

Various embodiments of the disclosure are described herein. It will be recognized that features specified in each embodiment may be combined with other specified features to provide further embodiments.

The present disclosure encompasses the compounds of the present disclosure, their tautomers, enantiomers, diastereomers, racemates or mixtures thereof, and their hydrates, esters, solvates or pharmaceutically acceptable salts.

The terms "pharmaceutically acceptable salts" refers to salts that retain the biological effectiveness and properties of the compounds of this disclosure and, which typically are not biologically or otherwise undesirable. In many cases, the compounds of the disclosure are capable of forming acid and/or base salts by virtue of the presence of amino and/or carboxyl groups or groups similar thereto. Pharmaceutically acceptable acid addition salts can be formed with organic acids and/or inorganic acids. Pharmaceutically acceptable base addition salts can be formed with organic bases and/or inorganic bases.

In many cases, the compounds of the disclosure are capable of forming esters by virtue of the presence of carboxyl groups. Esters include C₁-C₆ alkyl esters.

Any formula given herein is also intended to represent unlabeled as well as isotopically forms of the compounds, like deuterium labeled compounds or ¹⁴C-labeled compounds.

The term "antibody" as used herein is used in the broadest sense and covers monoclonal antibodies, polyclonal antibodies, modified monoclonal and polyclonal antibodies, monospecific antibodies, multispecific antibodies such as bispecific antibodies, antibody fragments and antibody mimetics (Affibody^{®}, Affilin^{®}, Affimer^{®}, Nanofitin^{®}, Cell Penetrating Alphabody^{®}, Anticalin^{®}, Avimer^{®}, Fynomer^{®}, Monobodies or nanoCLAMP^{®}). An example of an antibody is trastuzumab.

The term "antibody" as referred to herein includes whole antibodies and any antigen-binding fragments (i.e., "antigen-binding portion") or single chains thereof.

A naturally occurring "antibody" is a glycoprotein comprising at least two heavy (H) chains and two light (L) chains inter-connected by disulfide bonds. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as V_{L}) and a light chain constant region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The terms "antigen-binding portion" of an antibody (or simply "antigen portion"), as used herein, refers to full length or one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include a Fab fragment, a monovalent fragment consisting of the V_{L}, V_{H}, C_{L} and CH1 domains; a F(ab)₂ fragment, a bivalent fragment comprising two Fab fragments linked by a disulfide bridge at the hinge region; a Fd fragment consisting of the V_{H} and CH1 domains; a Fv fragment consisting of the V_{L} and V_{H} domains of a single arm of an antibody; a dAb fragment (Ward et al., 1989 Nature 341:544-546), which consists of a V_{H} domain; and an isolated complementarity determining region (CDR), or any fusion proteins comprising such antigen-binding portion.

Furthermore, although the two domains of the Fv fragment, V_{L} and V_{H}, are coded for by separate genes, they can be joined, using recombinant methods, by a synthetic linker that enables them to be made as a single chain protein in which the V_{L} and V_{H} regions pair to form monovalent molecules (known as single chain Fv (scFv); see e.g., Bird et al., 1988 Science 242:423-426; and Huston et al., 1988 Proc. Natl. Acad. Sci. 85:5879-5883). Such single chain antibodies are also intended to be encompassed within the term "antigen-binding portion" of an antibody. These antibody fragments are obtained using conventional techniques known to those of skill in the art, and the fragments are screened for utility in the same manner as are intact antibodies.

In specific embodiments, the ligand of the LDC is a chimeric, humanized or human antibody.

The term "human antibody", as used herein, is intended to include antibodies having variable regions in which both the framework and CDR regions are derived from sequences of human origin. Furthermore, if the antibody contains a constant region, the constant region also is derived from such human sequences, e.g., human germline sequences, or mutant versions of human germline sequences or antibody containing consensus framework sequences derived from human framework sequences analysis, for example, as described in Knappik, et al. (2000. J Mol Biol 296, 57-86).

The human antibodies may include amino acid residues not encoded by human sequences (e.g., mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human sequences.

As used herein, "isotype" refers to the antibody class (e.g., IgM, IgE, IgG such as IgG1 or IgG4) that is provided by the heavy chain constant region genes.

The phrases "an antibody recognizing an antigen" and "an antibody specific for an antigen" are used interchangeably herein with the term "an antibody which binds specifically to an antigen".

As used herein, the term "treating" includes reversing, alleviating, inhibiting the progression of, preventing or reducing the likelihood of the disease, disorder, or condition to which such term applies, or one or more symptoms or manifestations of such disease, disorder or condition. Preventing refers to causing a disease, disorder, condition, or symptom or manifestation of such, or worsening of the severity of such, not to occur. Accordingly, the presently disclosed compounds can be administered prophylactically to prevent or reduce the incidence or recurrence of the disease, disorder, or condition.

As used herein, the terms "therapeutically efficient amount" of a compound refer to an amount of the compound that will elicit the biological or medical response of a subject, for example, ameliorate the symptoms, alleviate conditions, slow or delay disease progression, or prevent a disease.

### ADC of formula I

The present disclosure first relates to an ADC compound of formula (I)

Ab-[L-D]p (I),

wherein :
Ab is an antibody or an antibody fragment,
L is a cleavable linker moiety bonded to Ab and D,
p is 1 or more, preferably from 1 to 16, and more preferably from 1 to 8
D is a cytotoxic drug moiety of formula (II) bonded to L, 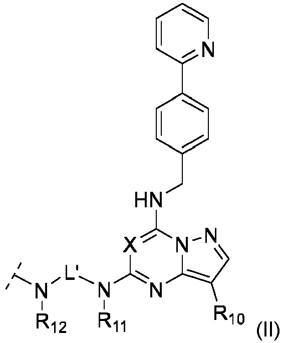 wherein
X is C or N, preferably N;
R10 is selected from the group consisting of H, Halogen, C₁-C₁₂ alkyl, C₁-C₁₂ haloalkyl, C₁-C₁₂ heteroalkyl, aryl having 6 to 10 ring atoms, C₃-C₈ cycloalkyl, heterocycloalkyl having 3 to 10 ring atoms, heteroaryl having 5 to 10 ring atoms, and combination thereof, said alkyl, heteroalkyl, aryl, cycloalkyl, heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl, preferably R10 is a halogen or a C₁-C₁₂ alkyl;
R11 is selected from the group consisting of H and C₁-C₆ alkyl, said alkyl being optionally substituted;
Or R11 is linked to an atom from the linker L' and forms a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl;
L' is a bond or a linker, preferably selected from C₁-C₂₀ alkylene, C₁-C₂₀ heteroalkylene, C₃-C₁₂ cycloalkylene, heterocycloalkylene having 5 to 10 ring atoms, arylene having 6 to 10 ring atoms, heteroarylene having 5 to 10 ring atoms, and any combination thereof; said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
R12 is selected from the group consisting of H, and C₁-C₁₂ alkyl;
Or R12 is linked to an atom from the linker L' and forms a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl;
Or R11 and R12, together with the N atoms and the linker L' to which they are bonded, form a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl.

### Drug D

Preferably X is N.

According to an embodiment, R10 is a halogen, atom, like F, CI, Br or I. For example, R10 can be F or Br. Preferably R10 is Br.

R11 can be H or a C₁-C₆ alkyl. R12 can be H or a C₁-C₆ alkyl.

According to an embodiment, L' is a bond or a linker selected from C₁-C₆ alkylene, C₃-C₈ cycloalkylene, arylene having 6 to 10 ring atoms, -(C₁-C₆ alkylene)-(C₃-C₈ cycloalkylene)-, and -(C₃-C₈ cycloalkylene)-(C₁-C₆ alkylene).

According to an embodiment, R11 is linked to an atom from the linker L' and forms a heterocycloalkyl having 3 to 10 ring atoms.

According to an embodiment, R12 is linked to an atom from the linker L' and forms a heterocycloalkyl having 3 to 10 ring atoms, said heterocycloalkyl being optionally substituted by one or more -OH.

According to another embodiment, R11 and R12, together with the N atoms and the linker L' to which they are bonded, form a heterocycloalkyl having 3 to 10 ring atoms.

According to an embodiment, X is N, R10 is Br, R11 is H, R12 is H, and L' is a C₃-C₆ cycloalkylene.

According to an embodiment, D is a moiety of formula (II) wherein
X is N,
R10 is a halogen atom,
R11 is H or a C₁-C₆ alkyl,
Or R11 is linked to an atom from the linker L' and forms a heterocycloalkyl having 3 to 10 ring atoms,
L' is a bond or a linker selected from C₁-C₆ alkylene, C₃-C₈ cycloalkylene, arylene having 6 to 10 ring atoms, -(C₁-C₆ alkylene)-(C₃-C₈ cycloalkylene)-, and -(C₃-C₈ cycloalkylene)-(C₁-C₆ alkylene), and
R12 is H or a C₁-C₆ alkyl,
Or R12 is linked to an atom from the linker L' and forms a heterocycloalkyl having 3 to 10 ring atoms, said heterocycloalkyl being optionally substituted by one or more -OH,
Or R11 and R12, together with the N atoms and the linker L' to which they are bonded, form a heterocycloalkyl having 3 to 10 ring atoms.

According to an embodiment, D is a moiety of formula (II) wherein X is N, R10 is a halogen atom, R11 is H or C₁-C₆ alkyl, R12 is H or C₁-C₆ alkyl, and L' is linker from C₁-C₆ alkylene, C₃-C₈ cycloalkylene, arylene having 6 to 10 ring atoms, -(C₁-C₆ alkylene)-(C₃-C₈ cycloalkylene)-, and -(C₃-C₈ cycloalkylene)-(C₁-C₆ alkylene).

According to an embodiment, D is a moiety of formula (III) 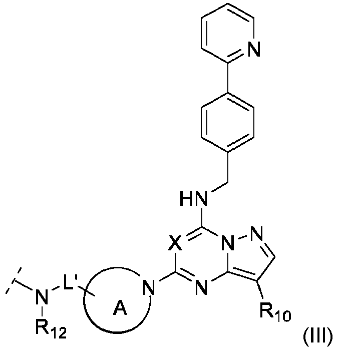 wherein
X is C or N, preferably N;
Ring A is a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl,
L' is a bond or a linker, preferably selected from C₁-C₁₂ alkylene, C₁-C₁₂ heteroalkylene, C₃-C₆ cycloalkylene, heterocyclylene having 5 to 10 ring atoms, arylene having 6 to 10 ring atoms, heteroarylene having 5 to 10 ring atoms, and any combination thereof, and
R12 is selected from the group consisting of H, and C₁-C₁₂ alkyl.

According to an embodiment, D is a moiety of formula (III) wherein X is N, R10 is a halogen atom, Ring A is a a heterocycloalkyl having 3 to 10 ring atoms, L' is a bond or a linker selected from C₁-C₆ alkylene, and R12 is H.

According to an embodiment, D is a moiety of formula (IV) 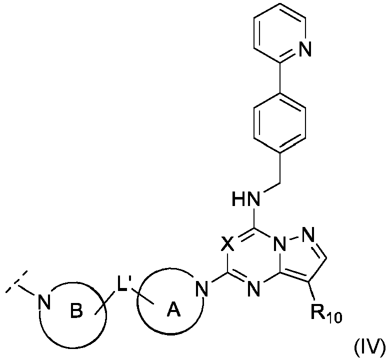 Wherein
X is C or N, preferably N;
Ring A is a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl; and
Ring B is a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl,
L' is a bond or a linker, preferably selected from C₁-C₁₂ alkylene, C₁-C₁₂ heteroalkylene, C₃-C₆ cycloalkylene, heterocyclylene having 5 to 10 ring atoms, arylene having 6 to 10 ring atoms, heteroarylene having 5 to 10 ring atoms, and any combination thereof.

According to an embodiment, D is a moiety of formula (IV) wherein X is N, R10 is a halogen atom, Ring A is a heterocycloalkyl having 3 to 10 ring atoms, Ring B is a heterocycloalkyl having 3 to 10 ring atoms, and L' is a bond.

According to an embodiment, D is a moiety of formula (V) 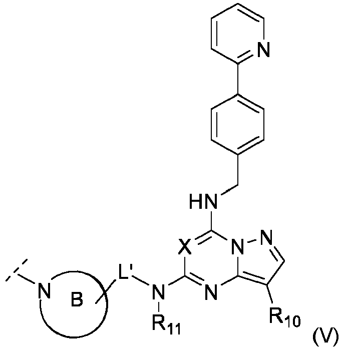 Wherein
X is C or N, preferably N;
R11 is selected from the group consisting of H, and C₁-C₁₂ alkyl,
L' is a bond or a linker, preferably selected from C₁-C₁₂ alkylene, C₁-C₁₂ heteroalkylene, C₃-C₆ cycloalkylene, heterocyclylene having 5 to 10 ring atoms, arylene having 6 to 10 ring atoms, heteroarylene having 5 to 10 ring atoms, and any combination thereof, and
Ring B is a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl.

According to an embodiment, D is a moiety of formula (V) wherein X is N, R10 is a halogen atom, R11 is H, L' is a bond or a C₁-C₆ alkylene, Ring B is a heterocycloalkyl having 3 to 10 ring atoms, said heterocycloalkyl being optionally substituted by one or more -OH.

According to an embodiment, Ring A and/or Ring B, is a heterocycloalkyl having 3 to 10 ring atoms, said heterocycloalkyl being optionally substituted, by one or more substituent selected from C₁-C₁₂ heteroalkyl, heterocyclyl having 5 to 10 ring atoms, and -NR'R"; R', and R" being independently selected from H and C₁-C₆ alkyl.

According to an embodiment, R10 is a halogen, preferably Br.

According to an embodiment, L' is a linker selected from C₁-C₁₂ alkylene, C₃-C₈ cycloalkylene, arylene having 6 to 10 ring atoms, and -(C₁-C₁₂ alkyl)-(C₃-C₈ cycloalkylene).

According to an embodiment, the drug D is selected from

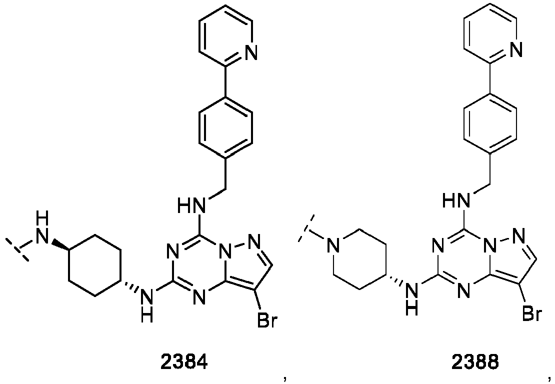

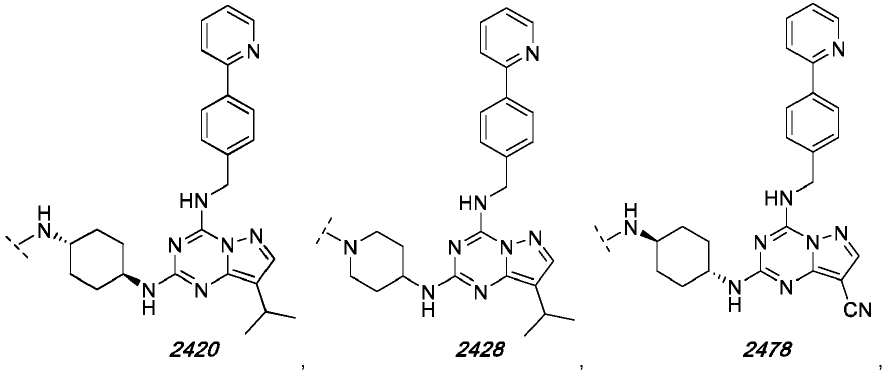

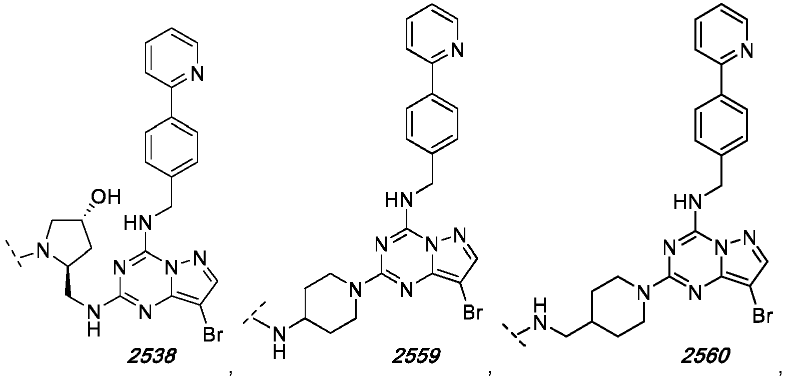

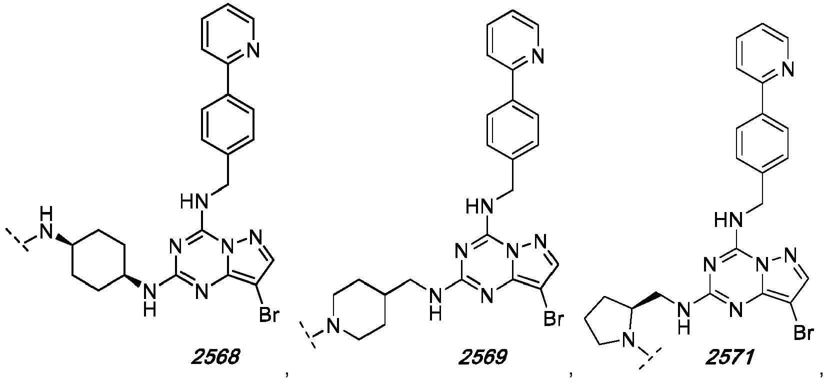

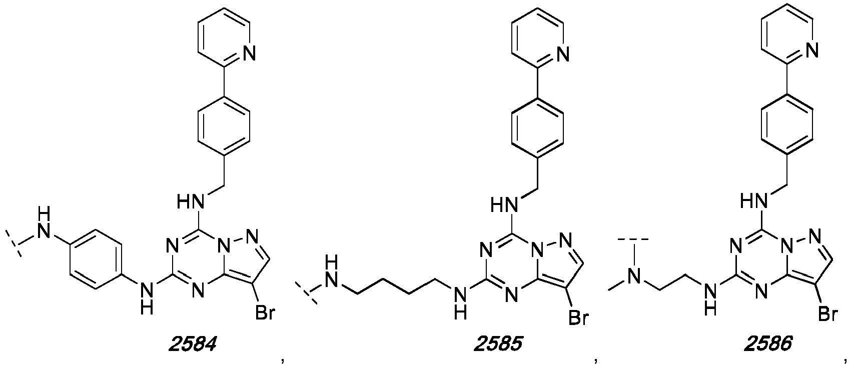

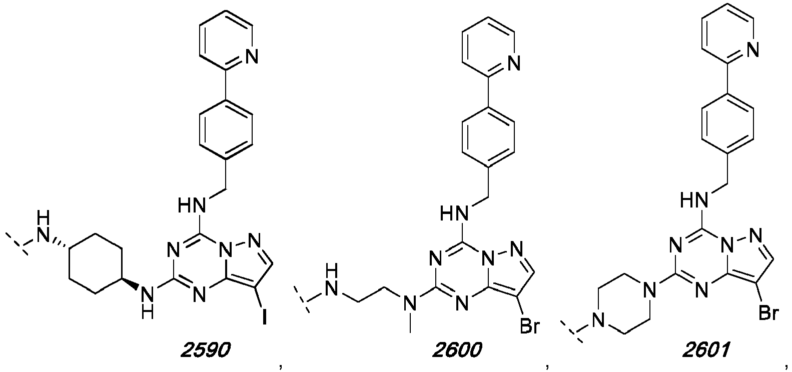

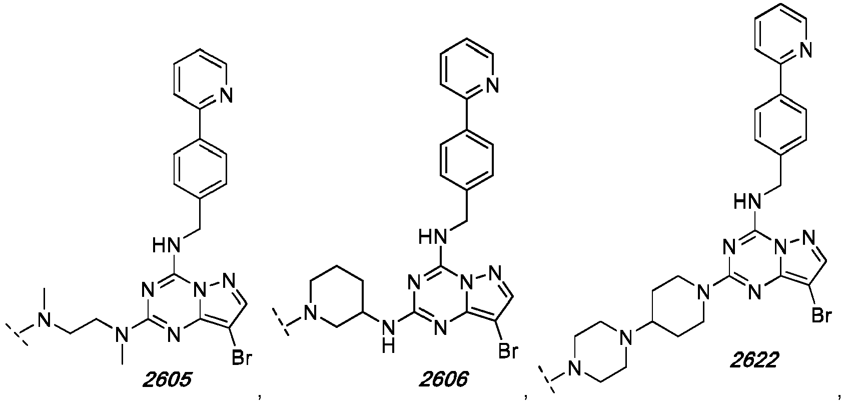

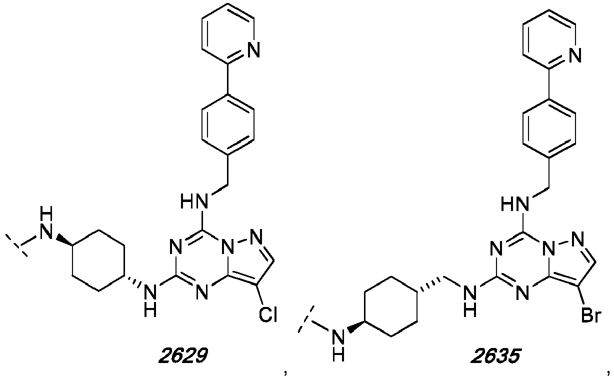

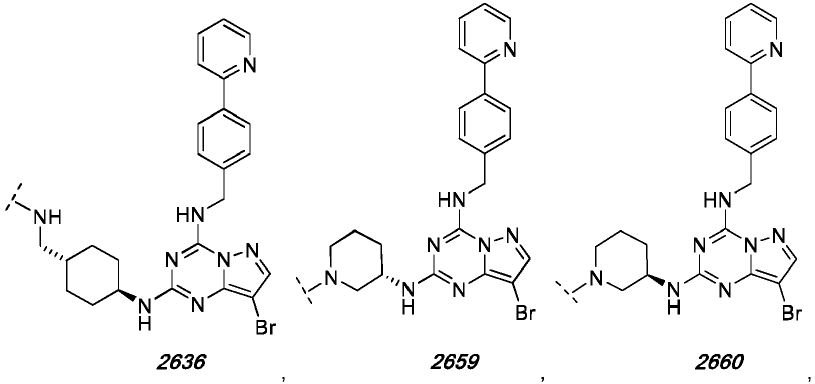

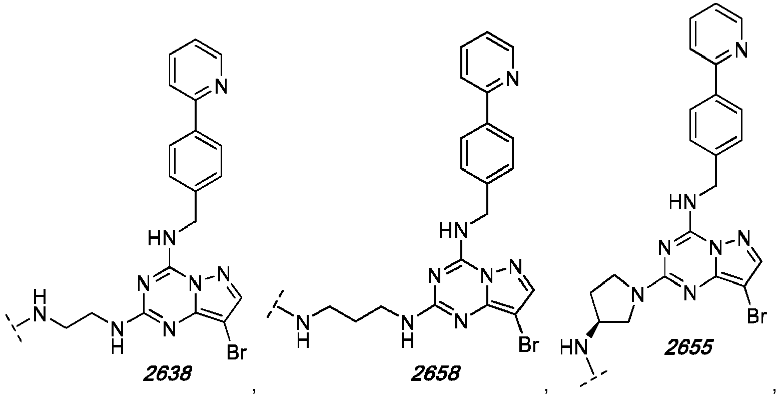

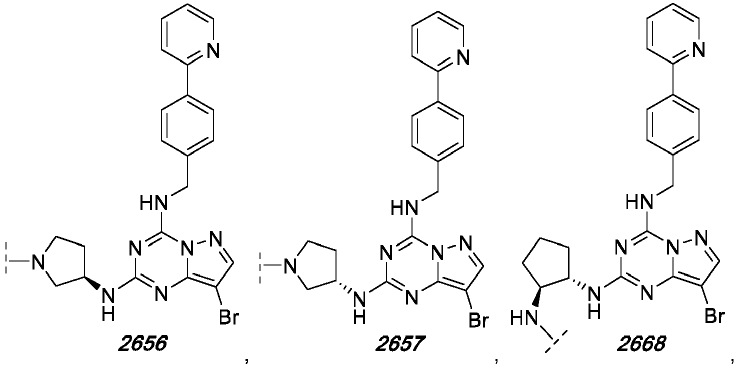

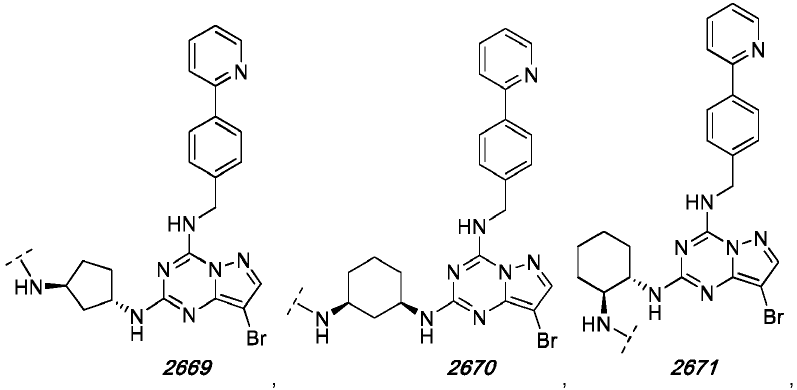

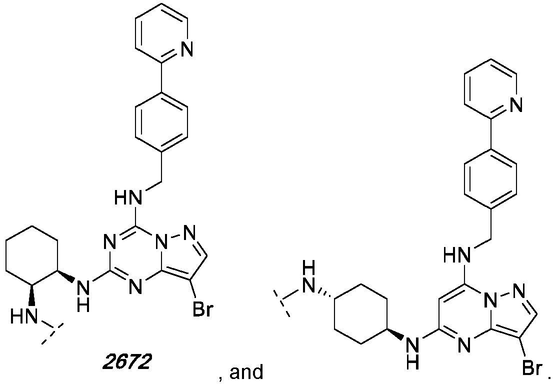

### Linker L

As used herein, a "linker" or "linker moiety" refers to any chemical moiety that is capable of covalently joining a compound, such as a drug moiety to another moiety such as an antibody moiety.

The ADCs of the disclosure contain a cleavable linker moiety L bonded to Ab, on one side, and bonded to the drug D on the other side.

The linker L can be polyvalent, so that it links more than one cytotoxic drug moiety D to the antibody. Examples of polyvalent linkers include the Fleximer^{®} linkers (which use a poly acetal backbone via a sequence of ester bonds) and dendritic linkers (cf. US2006/116422).

As used herein, the wording "cleavable" refers to a linker which can be cleaved under specific environmental conditions (such as redox potential or pH) or specific lysosomal enzymes in response to intracellular environments, for example, after internalizing of the ADC in a cell. For example, the linker can include hydrazone and/or disulfide groups.

When a disulfide linker is used, the cleavage occurs between the two sulfur atoms of the disulfide. A variety of disulfide linkers are known in the art and can be adapted for use in the present disclosure, including, for example, those that can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate), SMPT (N-succinimidyl-oxycarbonyl-alpha-methyl-alpha-(2-pyridyl-dithio)toluene), and SPP (N-succinimidyl 4-(2-pyridyldithio)pentanoate). See, for example U.S. Patent No. 4,880,935.

In some embodiments, the cleavable linker is pH-sensitive and will comprise, for example, an acid-labile linker that is hydrolyzable in the lysosome (e.g., a hydrazone, semicarbazone, thiosemicarbazone, cis-aconitic amide, orthoester, acetal, or ketal group) can be used. (See, e.g., U.S. Patent Nos. 5,122,368; 5,824,805; 5,622,929). Such linkers are relatively stable under neutral pH conditions, such as those in the blood, but are unstable at pH 5.5 or 5.0, the approximate pH of the lysosome.

Other degradable linkages that may be included in linkers include ester linkages formed by the reaction of PEG carboxylic acids or activated PEG carboxylic acids with alcohol groups on a biologically active agent, wherein such ester groups generally hydrolyze under physiological conditions to release the biologically active agent. Hydrolytically degradable linkages include, but are not limited to, carbonate linkages; imine linkages resulting from reaction of an amine and an aldehyde; phosphate ester linkages formed by reacting an alcohol with a phosphate group; acetal linkages that are the reaction product of an aldehyde and an alcohol; orthoester linkages that are the reaction product of a formate and an alcohol; and oligonucleotide linkages formed by a phosphoramidite group, including but not limited to, at the end of a polymer, and a 5' hydroxyl group of an oligonucleotide.

In specific embodiments, L is a cleavable linker moiety of formula -A-W-, wherein A is an optional stretcher unit linked to Ab, and W is the cleavable moiety linked to D.

In specific embodiments, the optional stretcher unit A may be selected from the group consisting of one or more amino acid(s), one or more N-substituted amino acid, optionally substituted polyether, C₁-C₂₀ alkylene, C₁-C₂₀ heteroalkylene arylene having 6 to 10 ring atoms, C₃-C₈ cycloalkylene, heterocycloalkylene having 5 to 10 ring atoms, heteroarylene having 5 to 10 ring atoms, C₂-C₁₀ alkenylene, and any combination thereof,
said alkylene and alkenylene being optionally interrupted by one or more heteroatom or chemical groups selected from -O-, -S-, -C(O)-, -NR"-, -C(O)NR"-, -NR"-C(O)-, -NR"-C(O)-NR‴-, -NR"-C(O)-O-, -O-C(O)NR"- and triazole,
and said alkylene, arylene, cycloalkylene, heterocycloalkylene, heteroarylene, and alkenylene being optionally substituted with one or more substituents, preferably selected from : halogen, oxo, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R'", -NR"-(CO)-R', and -NR"R‴;
R', R" and R‴ being independently selected from H and C₁-C₆ alkyl

Example of cleavable linkers which may be used for the ADC of the disclosure includes acid-sensitive or acid-labile linkers, such as acid-labile hydrazone linker, lysosomal protease-sensitive linkers, b-glucuronide linker or glutathione-sensitive disulfide linker.

In specific embodiments wherein L is a lysosomal protease-sensitive linker, W comprises a cleavable peptide moiety which may be selected from the group consisting of valine-citruline (Val-Cit), alanine-alanine-asparagine (Ala-Ala-Asn), valine-alanine (Val-Ala) and phenylalanine-lysine (Phe-Lys). The cleavable peptide moiety can be a dipeptide, a tripeptide or a tetrapeptide. Preferably, W comprises a Valine-Alanine peptide moiety. The peptide group can be linked to the rest of the ADC via a par-aminobenzyl group. The peptide is linked through the amino group via an amide bond. If the peptide comprises a free end, the latter can be capped with a group like acetyl.

In other specific embodiments, wherein L is a protease sensitive linker, W comprises a sugar cleavable unit, preferably selected from b-glucuronide or b-galactoside moiety.

In other specific embodiments, wherein L is a glutathione sensitive linker, W comprises a disulfide moiety.

In preferred embodiments, L is a cleavable linker moiety of formula -A-W-, wherein W is of the following formula (VI) 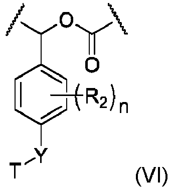 wherein
each R₂ is independently selected from the group consisting electron-withdrawing groups and C1-C4 alkyl;
n is 0, 1 or 2 ;
T is a sugar cleavable unit or a polypeptide cleavable unit;
Y is O when T is a sugar cleavable unit, or NR₃ when T is a polypeptide cleavable unit; and
R₃ is selected from the group consisting of H, C₁-C₂₄ alkyl, C₂-C₆ alkenyl; optionally substituted polyether, aryl having 6 to 10 ring atoms, C₃-C₈ cycloalkyl, heterocycloalkyl having 3 to 10 ring atoms, heteroaryl having 5 to 10 ring atoms, and any combination thereof, said alkyl and alkenyl being optionally interrupted by one or more heteroatom or chemical groups selected from -O-, -S-, -C(O)-, -NR"-, -C(O)NR"-, -NR"-C(O)-, -NR"-C(O)-NR‴-, -NR"-C(O)-O-, - O-C(O)NR"- and triazole,
R" and R‴ being independently selected from H and C1-C6 alkyl,
and pharmaceutically acceptable salts thereof

In specific embodiments, T is a sugar cleavable unit which is a glucuronide or a galactoside.

In other specific embodiments, T is a dipeptide, preferably selected from Val-Cit, Val-Ala and Phe-Lys.

In a more specific embodiment, L corresponds to a linker -A-W- of formula (VII) 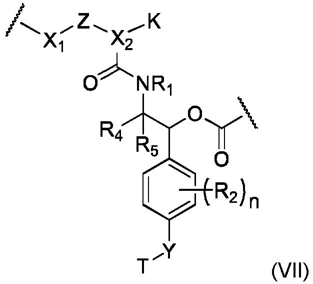 wherein
X₁ is a connector unit;
Z is an optional spacer;
X₂ is a connector unit;
K is an optional hydrophobicity masking entity, preferably selected from polysarcosine and polyethylene glycol;
R₁ is selected from the group consisting of H, C₁-C₂₄ alkyl, C₂-C₆ alkenyl; optionally substituted polyether, aryl having 6 to 10 ring atoms, C₃-C₈ cycloalkyl, heterocycloalkyl having 3 to 10 ring atoms, heteroaryl having 5 to 10 ring atoms, and any combination thereof, said alkyl and alkenyl being optionally interrupted by one or more heteroatom or chemical groups selected from -O-, -S-, -C(O)-, -NR"-, -C(O)NR"-, -NR"-C(O)-, -NR"-C(O)-NR‴-, -NR"-C(O)-O-, - O-C(O)NR"- and triazole;
R₄ is selected from the group consisting H, C₁-C₆ alkyl and C₂-C₆ alkenyl, said alkyl and alkenyl being optionally interrupted by one or more heteroatom or chemical groups selected from -O-, -S-, -C(O)-, and -NR"-;
R₅ is selected from the group consisting H, C₁-C₆ alkyl and C₂-C₆ alkenyl, said alkyl and alkenyl being optionally interrupted by one or more heteroatom or chemical groups selected from -O-, -S-, -C(O)-, and -NR"-.

In a preferred embodiment of the linker of Formula (IV), X1 and X2 are independently selected from the group consisting of one or more amino acid(s), one or more N-substituted amino acid, optionally substituted polyether, C₁-C₁₂ alkylene, arylene having 6 to 10 ring atoms, C₃-C₈ cycloalkylene, heterocycloalkylene having 5 to 10 ring atoms, heteroarylene having 5 to 10 ring atoms, C₂-C₁₀ alkenylene, and any combination thereof,
said alkylene and alkenylene being optionally interrupted by one or more heteroatom or chemical groups selected from -O-, -S-, -C(O)-, -NR"-, -C(O)NR"-, -NR"-C(O)-, -NR"-C(O)-NR‴-, -NR"-C(O)-O-, -O-C(O)NR"- and triazole,
and said alkylene, arylene, cycloalkylene, heterocycloalkylene, heteroarylene, and alkenylene being optionally substituted with one or more of the substituents selected from : halogen, oxo, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R‴, -NR"-(CO)-R', and -NR"R‴;
R', R" and R‴ being independently selected from H and C₁-C₆ alkyl.

In other preferred embodiments of of the linker of Formula (IV), Z is independently selected from the group consisting of one or more amino acid(s), one or more N-substituted amino acid, optionally substituted polyether, C₁-C₁₂ alkylene, arylene having 6 to 10 ring atoms, C₃-C₈ cycloalkylene, heterocycloalkylene having 5 to 10 ring atoms, heteroarylene having 5 to 10 ring atoms, C₂-C₁₀ alkenylene, and any combination thereof,
said alkylene and alkenylene being optionally interrupted by one or more heteroatom or chemical groups selected from -O-, -S-, -C(O)-, -NR"-, -C(O)NR"-, -NR"-C(O)-, -NR"-C(O)-NR‴-, -NR"-C(O)-O-, -O-C(O)NR"- and triazole,
and said alkylene, arylene, cycloalkylene, heterocycloalkylene, heteroarylene, and alkenylene being optionally substituted with one or more of the substituents selected from : halogen, oxo, -OH, -NO₂, -CN, C₁-C₆ alkyl, C₃-C₆ cycloalkyl, heterocyclyl having 5 to 10 ring atoms, aryl having 6 to 10 ring atoms, heteroaryl having 5 to 10 ring atoms, C₁-C₆ alkoxy, C₁-C₆ haloalkyl, C₁-C₆ haloalkoxy, -(CO)-R', -O-(CO)-R', -(CO)-O-R', -(CO)-NR"R‴, -NR"-(CO)-R', and -NR"R‴;
R', R" and R‴ being independently selected from H and C₁-C₆ alkyl.

In other embodiments of the linker L of formula (IV) or (V), K is a polysarcosine, preferably of the following formula (VIII) 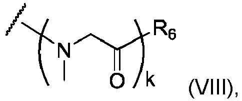 wherein k is an integer between 2 and 50, preferably between 4 and 30, and R₆ corresponds to OH or NH₂.

In other embodiments of the linker L of formula (IV) or (V), K is a polyethylene glycol moiety (PEG), preferably comprising between 2 and 50 ethylene-glycol moieties.

### Antibody Ab

Ab is an antibody or an antigen-binding fragment thereof.

According to an embodiment, the antibody or antigen-binding fragment thereof binds an antigen selected from the group comprising or consisting of HER2/neu, folate receptor alpha, CD30, CD33 and CD74.

According to an embodiment, the antibody or antigen-binding fragment thereof binds to HER2/neu.

Some examples of antibodies suitable as ligand L in the compound of formula (I) described herein include, but are not limited to, trastuzumab, brentuximab, loncastuximab, rosopatamab, rituximab, pinatuzumab, polatuzumab, and naratuximab.

According to an embodiment, the antibody is trastuzumab, farletuzumab, huMy9-6, milatuzumab, or brentuximab.

According to an embodiment, the antibody is trastuzumab.

According to an embodiment, the ADC is of formula (IX) or (X)

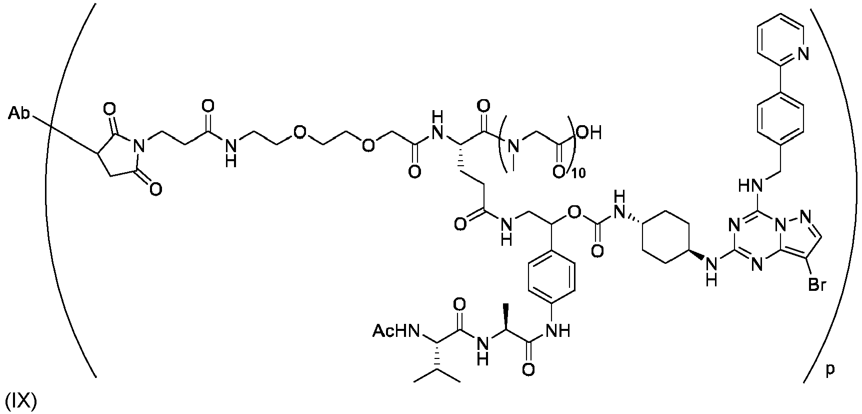

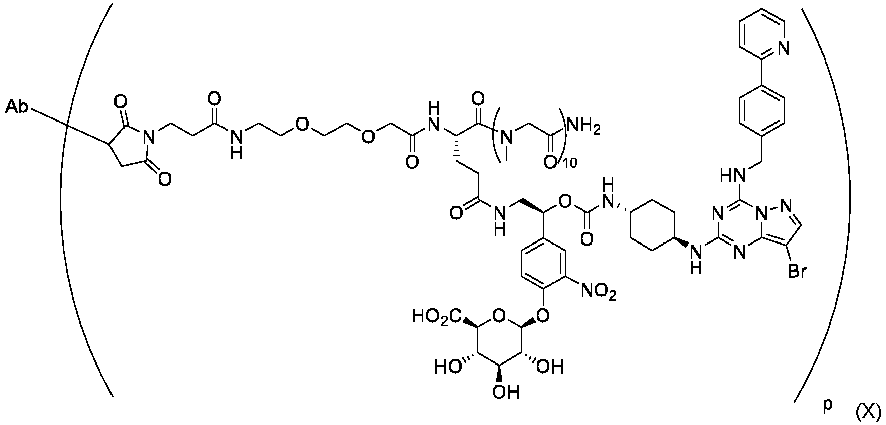

### Pharmaceutical composition

The disclosure also relates to a pharmaceutical composition comprising a compound of the present disclosure and a pharmaceutically acceptable carrier.

In another aspect, the present disclosure provides a composition, e.g. a pharmaceutical composition, containing one or a combination of ADC disclosed herein, (for example, an ADC of formula (I)), formulated together with a pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. The pharmaceutical formulation of the disclosure may further comprise one or more pharmaceutically acceptable excipients selected from stabilizers, surfactants, buffering agents, antimicrobial preservatives, protectants, antioxidants, chelating agents, bulking agents.

As used herein, a "solvent" is any pharmaceutically acceptable (i.e., safe and non-toxic for administration to a human or another mammal) and useful ingredient for the preparation of a liquid formulation, such as an aqueous formulation. Exemplary solvents include water such as sterile water for injection (WFI) or bacteriostatic water for injection (BWFI), pH buffered solution (e.g., phosphate-buffered saline), sterile saline solution, Ringer's solution or dextrose solution, and combinations thereof. Preferably, the solvent is sterile water for disclosure or bacteriostatic water for injection (BWFI).

As used herein, stabilizers are compounds increasing protein stability, especially against unfolding and aggregation. Preferably, the stabilizer is admitted by the authorities as a suitable additive or excipient in pharmaceutical formulations.

The stabilizer may be a saccharide. A "saccharide" herein comprises the general composition (CH₂O)ₙ and derivatives thereof, including monosaccharides, disaccharides, trisaccharides, polysaccharides, sugar alcohols, reducing sugars, nonreducing sugars, etc. Examples of saccharides herein include glucose, sucrose, trehalose, lactose, fructose, maltose, dextran, glycerin, dextran, erythritol, glycerol, arabitol, sylitol, sorbitol, mannitol, mellibiose, melezitose, raffinose, mannotriose, stachyose, maltose, lactulose, maltulose, glucitol, maltitol, lactitol, iso-maltulose, etc.

The concentration of the stabilizer in the pharmaceutical formulation of the disclosure be comprised between 1 and 500 mM.

As used herein, a "surfactant" refers to a surface-active agent. Surfactants are generally added in protein formulations in order to reduce the exposure of hydrophobic regions and so decreasing protein-protein interactions and interface-induced aggregation, also prevented by competition for adsorption sites.

Examples of surfactants herein include polysorbate (for example, polysorbate 20 and, polysorbate 80); poloxamer (e.g. poloxamer 188); Triton; sodium dodecyl sulfate (SDS); sodium laurel sulfate; sodium octyl glycoside; lauryl-, myristyl-, linoleyl-, or stearyl- sulfobetaine; lauryl-, myristyl-, linoleyl- or stearyl-sarcosine; linoleyl-, myristyl-, or cetyl- betaine; lauroamidopropyl-, cocamidopropyl-,linoleamidopropyl-, myristamidopropyl-, palmidopropyl-, or isostearamidopropyl- betaine (e.g. lauroamidopropyl); myristamidopropyl-, palmidopropyl-, or isostearamidopropyl- dimethylamine; sodium methyl cocoyl-, or disodium methyl oleyl-taurate; polyethylglycol, polypropyl glycol, and copolymers of ethylene and propylene glycol (e.g. Pluronics, PF68 etc). Other examples of pharmaceutically acceptable surfactants include polyoxyethylen-sorbitan fatty acid esters (Tween), polyethylene-polypropylene glycols, polyoxyethylene- stearates, polyoxyethylene alkyl ethers, e.g. polyoxyethylene monolauryl ether, alkylphenylpolyoxyethylene ethers (Triton-X), polyoxyethylene-polyoxypropylene copolymer (Poloxamer, Pluronic), and sodium dodecyl sulphate (SDS). Most suitable polyoxyethylenesorbitan-fatty acid esters are polysorbate 20, (sold under the trademark Tween 20^{™}) and polysorbate 80 (sold under the trademark Tween 80^{™}).

Most suitable polyethylene-polypropylene copolymers are those sold under the names Pluronic^{®} F68 or Poloxamer 188^{™}. Most suitable polyoxyethylene alkyl ethers are those sold under the trademark Brij^{™}. Most suitable alkylphenol-polyoxyethylene ethers are sold under the trade name Triton-X.

The concentration of surfactant in the pharmaceutical formulation of the disclosure may be comprised between 0.01 and 0.1 % (w/v),.

As used herein, the term "buffering agent" refers to an agent which provides that the solution comprising it resists changes in pH by the action of its acid/base conjugate components. Examples of buffering agents that will control the pH in this range include acetate, succinate, gluconate, histidine, citrate, glycylglycine and other organic acid buffers.

A "preservative" is a compound which can be added to the formulations herein to reduce contamination by and/or action of bacteria, fungi, or another infectious agent. The addition of a preservative may, for example, facilitate the production of a multi-use (multiple-dose) formulation. Examples of potential preservatives include octadecyldimethylbenzylammonium chloride, hexamethonium chloride, benzalkonium chloride (a mixture of alkylbenzyldimemylammonium chlorides in which the alkyl groups are long- chained), and benzethonium chloride. Other types of preservatives include aromatic alcohols such as phenol, butyl and benzyl alcohol, alkyl parabens such as methyl or propyl paraben, catechol, resorcinol, cyclohexanol, 3-pentanol, and w-cresol.

A "protectant", as generally used herein, is a substance which, when combined with a protein, significantly reduces chemical and/or physical instability of the protein upon lyophilization and/or subsequent refrigerated storage. Exemplary protectants include sugars and their corresponding sugar alcohols, such as sucrose, lactose, trehalose, dextran, erythritol, arabitol, xylitol, sorbitol, and mannitol; amino acids, such as arginine or histidine; lyotropic salts, such as magnesium sulfate; polyols, such as propylene glycol, glycerol, polyethylene glycol), or polypropylene glycol); and combinations thereof. Additional exemplary of protectants include gelatin, dextrins, modified starch, and carboxymethyl cellulose.

The protectant may be added to a pre-lyophilized formulation in a "lyoprotecting amount". This means that, following lyophilization of the protein in the presence of lyoprotecting amount of the protectant, the protein essentially retains its physical and chemical stability and integrity.

An "antioxidant", as generally used herein is a pharmaceutically acceptable excipient generally used to limit oxidation reactions and maintain the stability and safety of proteins. Examples of antioxidants are ascorbic acid, sodium metabisulfite, histamine, methionine, ascorbic acid, glutathione, vitamin E, polyethylenimine.

The antioxidant concentration in the pharmaceutical formulation of the disclosure may be comprised between 5 and 25 mM.

A "chelating agent" is a pharmaceutically acceptable excipient generally used to maintain the stability of proteins. Examples of chelating agent include edetate disodium, diethylenetriamine penta-acetic acid, citric acid, hexaphosphate, thioglycolic acid, zinc.

A "bulking agent," as generally used herein, is a pharmaceutically acceptable excipient generally used to add mass to a lyophilized mixture and contributes to the physical structure of the lyophilized cake (e.g. facilitates the production of an essentially uniform lyophilized cake which maintains an open pore structure). Exemplary bulking agents include mannitol, glycine, lactose, modified starch, polyethylene glycol), and sorbitol.

The form of the pharmaceutical compositions, the route of administration, the dosage and the regimen naturally depend upon the condition to be treated, the severity of the illness, the age, weight, and sex of the patient.

The pharmaceutical compositions of the disclosure can be formulated for a topical, oral, parenteral, intraperitoneal, intranasal, intravenous, intramuscular, subcutaneous or intraocular administration and the like, preferably intraperitoneal or intravenous.

Preferably, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the ADC may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders or lyophilisates for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

An ADC of the disclosure can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The ADC of the disclosure may be formulated within a therapeutic mixture to comprise about 0.0001 to 1.0 milligrams, or about 0.001 to 0.1 milligrams, or about 0.1 to 1.0 or even 1.0 to about 10 milligrams per dose. Multiple doses can also be administered.

The pharmaceutical formulation comprising the ADC of the disclosure may be a "Ready-to-Use" injectable formulation or a lyophilized formulation.

In a specific embodiment, the pharmaceutical formulation comprising the ADC of the disclosure may be supplied in a pre-filled syringe.

### Method of use

The disclosure also relates to a compound of the disclosure for use as a drug.

ADC of the present disclosure have therapeutic utilities. For example, these molecules can be administered in a subject, e.g. in vivo, to treat, or prevent a variety of disorders.

It is the contemplated herein to use the ADC of the present disclosure as a medicament, in particular for use in the treatment of cancer in a subject in need thereof, in particular cancer with tumor cells expressing FRα, more specifically, a cancer with a solid tumor, and more specifically selected from the group consisting of ovarian cancer, breast cancer, lung cancer, or mesothelioma.

The terms "cancer" refers to or describe the physiological condition in mammals that is typically characterized by unregulated cell growth. Examples of cancer include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma and islet cell cancer), mesothelioma, schwannoma (including acoustic neuroma), meningioma, adenocarcinoma, melanoma, and leukemia or lymphoid malignancies. More particular examples of such cancers include squamous cell cancer (e.g. epithelial squamous cell cancer), lung cancer including small-cell lung cancer, non- small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer.

Hence, the disclosure relates to a method of treating cancer, in particular one of the above listed cancers, said method comprising administering a therapeutically efficient amount of an ADC of formula (I) as disclosed herein.

The ADC for use as disclosed above may be administered as the sole active ingredients or in conjunction with, e.g. as an adjuvant to or in combination to, other drugs e.g. anti-viral, anti-inflammatory agents or cytotoxic, anti-proliferative, chemotherapy or anti-tumor agents, e.g. for the treatment or prevention of diseases mentioned above.

For example, the ADC for use as disclosed above may be used in combination with AZT, IFN-alpha, anti-CD20 mAb, anti-CD25 mAb, anti-PD1 mAb, anti-PDL-1 mAb, anti-CTLA4 mAb, chemotherapy agents.

Examples of such anti-PD1 or anti-PDL1 antibody includes without limitation, nivolumab, pembrolizumab, avelumab, durvalumab, cemiplimab, or atezolizumab.

Suitable anti-tumor agents may include without limitation, alkylating agents (such as cyclophosphamide, mechloretamine, chlorambucil, melphalan, nitrosureas, temozolomide), anthracyclines (such as daunorubicin, doxorubicin, epirubicin, idarubicin, mitoxantrone, valrubicin), taxanes (such as paclitaxel, docetaxel), epothilones, inhibitors of Topoisomerase I (such as Irinotecan or Topotecan), inhibitors of Topoisomerase II (such as etoposide, teniposide, or tafluposide), nucleotide analogs and precursor analogs (such as azacitidine, azathioprine, capecitabine, cytarabine, flurouracil, gemcitabine, hydroxyurea, mercaptopurine, methotrexate, or Tioguanine), peptide antibiotics (such as bleomycin, colistin, gramicidin), platinum-based antineoplastic agents (such as carboplatin, cisplatin and oxaliplatin), retinoids (such as tretinoin, alitretinoin, bexarotene), vinca alkaloids and derivatives (such as vinblastine, vincristine, vindesine, vinorelbine), anti-VEGF agents (such as bevacizumab, ranibizumab, sunitinib, sorafenib, pazopanib, targeted therapies such as kinase inhibitors (such as ibrutinib, idelalisib, erlotinib, gefitinib, imatinib, vemurafenib, vismodegib), proteasome inhibitors (such as bortezomib, carfilzomib), histone deacetylase inhibitors (such as vorinostat or romidepsin).

In accordance with the foregoing the present disclosure provides in a yet further aspect a method comprising co-administration, e.g. concomitantly or in sequence, of a therapeutically effective amount of an ADC of the disclosure, and at least one second drug substance, said second drug substance being anti-viral, anti-inflammatory agents or cytotoxic, anti-proliferative, chemotherapy or other anti-tumor agents, e.g. as indicated above.

Also within the scope of the present disclosure are kits consisting of the compositions (e.g. comprising ADC) disclosed herein and instructions for use. The kit can further contain a least one additional reagent, or one or more additional antibodies or proteins (e.g. an antibody having a complementary activity which binds to an epitope on the target antigen distinct from the first antibody). Kits typically include a label indicating the intended use of the contents of the kit. The term label includes any writing, or recorded material supplied on or with the kit, or which otherwise accompanies the kit. The kit may further comprise tools for diagnosing whether a patient belongs to a group that will respond to an ADC treatment, as defined above.

The disclosure also relates to an ADC of the disclosure for use in a method for treating inflammatory disease. The inflammatory disease can be caused, induced, initiated and/or enhanced by bacteria, viruses, prions, parasites, fungi, and/or caused by irritative, traumatic, metabolic, allergic, autoimmune, or idiopathic agents.

The inflammatory disease can be selected from the group comprising or consisting of inflammatory diseases of the central nervous system (CNS), inflammatory rheumatic diseases, inflammatory diseases of blood vessels, inflammatory diseases of the middle ear, inflammatory bowel diseases, inflammatory diseases of the skin, inflammatory disease uveitis, and inflammatory diseases of the larynx.

### Process of making ADC of the disclosure

Antibodies of the disclosure can be conjugated to at least one drug by linkers L by any techniques known in the art. Such techniques are for example described in Greg T. Hermanson, Bioconjugate Techniques, 3rd Edition, 2013, Academic Press (eBook ISBN: 9780123822406). For more information relative to methods for conjugating therapeutic agents to antibodies, see Lyon et al., "Chapter six - Conjugation of Anticancer Drugs Through Endogenous Monoclonal Antibody Cysteine Residues", 2012, Methods in Enzymology, 502, 123-138 (doi:10.1016/B978-0-12-416039-2.00006-9); Chapter 2-7 of "Antibody-Drug Conjugates: Fundamentals, Drug Development, and Clinical Outcomes to Target Cancer", 3 November 2016, eBook ISBN:9781119060727, doi:10.1002/9781119060727 and Panowksi S *et al.* 2014 Jan 1; 6(1): 34-45.

The moiety -L-D can be prepared following procedures that are known in the art of organic synthesis (chemical reactions, extraction, evaporation, precipitation, chromatography, filtration, trituration, crystallization, and the like), and analytical procedures, which are known to persons of ordinary skill in the art of analytical chemistry. The details of such reactions and techniques can be found in a number of treatises, including Richard Larock, Comprehensive Organic Transformations, A Guide to Functional Group Preparations, 2nd Ed (2010), and the multi-volume serie edited by Michael B. Smith and others, Compendium of Organic Synthetic Methods (1974 et seq.). Starting materials and reagents may be obtained from commercial sources or may be prepared using literature methods.

Final ADC of formula (I) can be obtained after conjugation of -L-D component to native or engineered cysteine thiol residues of the antibody, following known procedures. See for example Lyon et al., "Chapter six - Conjugation of Anticancer Drugs Through Endogenous Monoclonal Antibody Cysteine Residues", 2012, Methods in Enzymology, 502, 123-138 (doi:10.1016/B978-0-12-416039-2.00006-9) or SJ Walsh et al., Site-selective modification strategies in antibody-drug conjugates, Chem. Soc. Rev., 2021,50, 1305-1353, doi:10.1039/D0CS00310G). Typically, the antibody component is reduced with a reducing agent such as tris(2-carboxyethyl)phosphine (TCEP) or dithiothreitol (DTT); -L-D component is added and is allowed to covalently react with the cysteine thiol residues of the antibody; the final ADC compound is purified and buffer-exchanged.

For example, the cytotoxic drug D can be synthesized as follows:

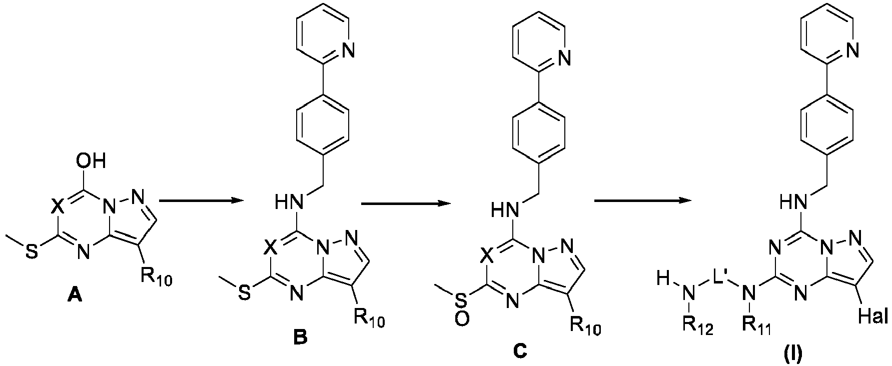

Compound **A** can be prepared as described in WO2018195397. Compound **A** can be converted to compound **B** using first phosphorous oxychloride and then 4-(Pyridin-2-yl)phenyl)methanamine. Compound **B** is then oxidized to the corresponding sulfone **C**, for example using meta-chloroperoxybenzoic acid. Finally, the compound of formula (I) can be prepared by nucleophilic aromatic substitution using the amine HNR₁₂-L'-NHR₁₁. This step can be done by heating the reaction mixture to a temperature comprised between 100 and 180°C, like at 140°C

The invention having been fully described is now further illustrated by the following embodiments and examples, which are illustrative only and are not meant to be further limiting.

### Examples

### Example 1: synthesis of 2-substituted 8-halogeno-N4-(4-(pyridin-2-yl) benzyl) pyrazolo[1,5-a][1,3,5] triazine-2,4-diamine family of compounds

### Materials and general organic synthesis methods

All solvents, reagents and active pharmaceutical ingredients were obtained from reputable commercial sources (Sigma-Aldrich, Fluorochem, TCI Chemicals, Acros Organics, Alfa Aesar, Enamine, Thermo Fisher, Carbosynth, WuXi AppTec, MedChemExpress) and used without further purification unless stated otherwise. Anhydrous solvents were purchased from Sigma-Aldrich. Unless stated otherwise, all chemical reactions were carried out at room temperature under an inert argon atmosphere. Compounds were obtained with HPLC purity >95% at 254nm, unless stated otherwise.

Liquid nuclear magnetic resonance spectra were recorded on a Bruker Fourier 300HD or Bruker AVANCE III HD400 spectrometer, using residual solvent peak for calibration. Exchangeable N*H*s were not described. Mass spectroscopy analysis has been performed by the Centre Commun de Spectrométrie de Masse (CCSM) of the UMR5246 CNRS institute of the University Claude Bernard Lyon 1.

Normal phase flash chromatography was performed either on Teledyne Isco CombiFlash^{®} Rf200 devices or Combiflash^{®} NextGen 300 devices, using Macherey-Nagel Chromabond^{®} flash cartridges (40-63µm). Reverse phase chromatography was performed using Biotage^{®} Sfar C18 Duo 100Å 30µm cartridges or using an Agilent 1100 preparative binary HPLC purification system.

Chemical reactions and compound characterization were respectively monitored and analyzed by thin-layer chromatography using pre-coated 40-63µm silica gel (Macherey-Nagel), HPLC-UV (Agilent 1100 systems) or UHPLC-UV/MS (Thermo UltiMate 3000 UHPLC system equipped with a Bruker Impact II^{™} Q-ToF mass spectrometer or Agilent 1260 HPLC system equipped with a Bruker MicrOTOF-QII mass spectrometer).

HPLC Method 1: Agilent 1100 HPLC system equipped with DAD detection. Mobile phase A was water + 0.1% TFA and mobile phase B was acetonitrile. Column was an Agilent Zorbax SB-Aq 4.6x150mm 5µm (room temperature). Linear gradient was 0%B to 50%B in 30 min, followed by a 5 min hold at 50%B. Flow rate was 1.0 mL/min.

HPLC Method 2: Agilent 1100 HPLC system equipped with DAD detection. Mobile phase A was water + 0.1% TFA and mobile phase B was acetonitrile. Column was an Agilent Poroshell 120 EC-C18 3.0x50mm 2.7µm (room temperature). Linear gradient was 5%B to 80%B in 9 min, followed by a 1 min hold at 80%B. Flow rate was 0.8 mL/min.

HPLC Method 3: Agilent 1100 HPLC system equipped with DAD detection. Mobile phase A was water + 0.1% TFA and mobile phase B was acetonitrile. Column was an Agilent Poroshell 120 EC-C18 3.0x50mm 2.7µm (room temperature). Linear gradient was 5%B to 80%B in 20 min, followed by a 2 min hold at 80%B. Flow rate was 0.8 mL/min.

HPLC Method 4: Thermo UltiMate 3000 UHPLC system + Bruker Impact II^{™} Q-ToF mass spectrometer. Mobile phase A was water + 0.1% formic acid and mobile phase B was acetonitrile + 0.1% formic acid. Column was an Agilent PLRP-S 1000Å 2.1x150mm 8µm (80°C). Linear gradient was 10%B to 50%B in 25 min. Flow rate was 0.4 mL/min. UV detection was monitored at 280 nm. The Q-ToF mass spectrometer was used in the m/z range 500-3500 (ESI⁺). Data were deconvoluted using the MaxEnt algorithm included in the Bruker Compass^{®} software.

HPLC Method 5 (preparative method): Teledyne Isco CombiFlash^{®} Rf200 binary MPLC system equipped with DAD detection. Mobile phase A was water + 0.1% TFA and mobile phase B was acetonitrile. Reusable cartridges were Biotage^{®} Sfär C18 Duo 100Å 30µm (30g). Linear gradient was 10%B to 50%B in 35 min, followed by a 5 min hold at 50%B. Flow rate was 25 mL/min.

HPLC Method 6 (preparative method): Agilent 1100 preparative binary HPLC system equipped with dual-loop auto-injector, DAD detection and fraction collector. Mobile phase A was water + 0.1% TFA and mobile phase B was acetonitrile. Column was a Waters SunFire C18 OBD Prep Column, 100Å, 5 µm, 19mm x 250mm (room temperature). Linear gradient was 10%B to 60%B in 40 min, followed by a 5 min hold at 60%B. Flow rate was 25 mL/min.

### 1.1) Synthesis of compound 2384

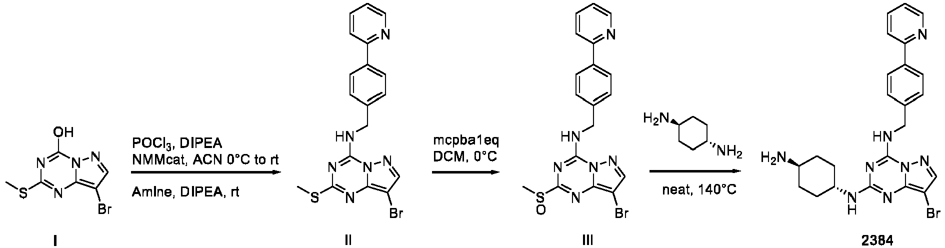

### Synthesis of compound I

Compound 8-Bromo-2-(methylthio)pyrazolo[1,5-*a*]-1,3,5-triazin-4(3*H*)-one [CAS 54346-35-9] (*compound I*) was prepared as described in WO2018195397 with slight modifications. To 2-(methylthio)pyrazolo[1,5-*a*]-1,3,5-triazin-4(3*H*)-one (3.0 g, 16.46 mmol) in DMF (30 mL) was added NBS (2.51 g, 14.10 mmol) within 5 min in small portions, maintaining the temperature between 8-10°C. The mixture was kept for 30 min at 0°C and concentrated in vacuo. The resulting solid was dispersed in EtOH (20 mL), filtered and washed with EtOH (5 mL x2). 3.09 g (84% yield) of bromo derivative as a white solid were obtained after drying. ¹H NMR (300 MHz, DMSO) δ 13.07 (s, 1H), 8.12 (s, 1H), 2.57 (s, 3H). HPLC Method 2 retention time = 4.64 min.

### Synthesis of compound II

Prepared from compound *I* and 4-(Pyridin-2-yl)phenyl)methanamine (the free base was obtained by DCM extraction of pH 9-10 basified aqueous solution of commercial hydrochloride salt CAS1498333-87-1). To a clear solution of 8-Bromo-2-(methylthio)pyrazolo[1,5-*a*]-1,3,5-triazin-4(3H)-one (*compound I*) (0.685 g, 2.62 mmol), DIPEA (0.914 mL, 2 eq) and N-methyl morpholine (0.001 mL, 0.005 eq) in ACN (4.6 mL) was added POCl₃ (0.318 mL, 1.3 eq) at 0°C. The mixture was then warmed to room temperature and stirred for 1h. To the suspension obtained was added at 0°C (4-(Pyridin-2yl)phenyl)methanamine (0.725 g, 1.5 eq) followed by DIPEA (0.457 mL, 1eq). The mixture clarified briefly before re-precipitation. After overnight stirring at room temperature the solid was filtrated and washed with ACN (1 mL x2) giving expected 8-bromo-2-(methylthio)-N-(4-(pyridin-2-yl)benzyl)pyrazolo[1,5-a][1,3,5]triazin-4-amine (0.898 g, 80% yield, HPLC purity 94%, 254 nm) as a beige solid). MS m/z (ESI+): Calc for C₁₈H₁₅BrN₆S [M+H]+ = 427.03 ; Exp [M+H]+ = 427.0. HPLC Method 2, retention time = 5.91 min. ¹H NMR (300 MHz, DMSO) δ 9.66 (t, *J* = 6.2 Hz, 1H), 8.65 (ddd, *J* = 4.8, 1.7, 0.9 Hz, 1H), 8.25 (s, 1H), 8.05 (d, *J* = 8.4 Hz, 2H), 7.95 (d, *J* = 8.0 Hz, 1H), 7.91 - 7.84 (m, 1H), 7.49 (d, *J* = 8.4 Hz, 2H), 7.35 (ddd, *J* = 7.2, 4.8, 1.3 Hz, 1H), 4.74 (d, *J* = 6.2 Hz, 2H), 2.49 (s, 3H). MS m/z (ESI+): Calc for C₁₈H₁₅BrN₆S [M+H]+ = 427.0 ; Exp [M+H]+ = 427.0.

### Synthesis of compound III

To 8-bromo-2-(methylthio)-N-(4-(pyridin-2-yl)benzyl)pyrazolo[1,5-a][1,3,5]triazin-4-amine (*compound II*) (0.896 g, 2.10 mmol) in DCM (42 mL) was added MCPBA 70% (0.517 mg, 1.0 eq) portionwise at 0°C within 5 min. The mixture was stirred at 0°C for 1 h, then diluted with DCM (250 mL) and washed with aqueous saturated NaHCOs (50 mL x2) then brine (30 mL). The organic layer was dried on Na₂SO₄, filtered and concentrated in vacuo to give the expected sulfoxide compound *III* (0.920 g, 99% yield, HPLC purity: 91%, 254 nm) as a near white solid. MS m/z (ESI+): Calc for C₁₈H₁₅BrN₆OS [M+H]+ = 443.03 ; Exp [M+H]+ = 443.0. HPLC Method 2, retention time = 4.10 min. ¹H NMR (300 MHz, DMSO) δ 10.10 (s, 1H), 8.64 (ddd, *J* = 4.8, 1.8, 1.0 Hz, 1H), 8.45 (s, 1H), 8.10 - 7.99 (m, 2H), 7.94 (dt, *J* = 8.1, 1.2 Hz, 1H), 7.86 (td, *J* = 7.7, 1.8 Hz, 1H), 7.56 - 7.46 (m, 2H), 7.34 (ddd, *J* = 7.3, 4.8, 1.3 Hz, 1H), 4.79 (s, 2H), 2.83 (s, 3H).

### Synthesis of compound 2384

A mixture of 8-bromo-2-(methylsulfinyl)-N-(4-(pyridin-2-yl)benzyl)pyrazolo[1,5-*a*][1,3,5]triazin-4-amine (*compound III*) (0.262 g, 0.591 mmol) and trans-1,4-diaminocyclohexane (0.540 g, 8 eq) were heated for 30 min at 140°C. After cooling to room temperature was partitioned between DCM (20 mL) and aqueous saturated NaHCOs (10 mL) then extract with DCM (20 mL x2) and washed with brine (10 mL). The organic layer was dried on Na₂SO₄, filtered and concentrated in vacuo. The crude was purified by chromatography on silicagel (15g) eluting with 1% to 20% MeOH in DCM to give compound 2384 (0.150 g, 51% yield) as a white foam. MS m/z (ESI+): Calc for C₂₃H₂₅BrN₈ [M+H]+ = 493.15 ; Exp = 493.2. HPLC Method 2, retention time = 3.84 min. ¹H NMR (300 MHz, DMSO) δ 8.65 (ddd, *J* = 4.8, 1.8, 1.0 Hz, 1H), 8.04 (d, *J* = 8.3 Hz, 2H), 7.94 - 7.91 (m, 2H), 7.86 (ddd, *J* = 8.0, 7.2, 1.8 Hz, 1H), 7.54 - 7.41 (m, 2H), 7.33 (ddd, *J* = 7.2, 4.8, 1.3 Hz, 1H), 4.71 - 4.61 (m, 2H), 3.79 - 3.54 (m, 1H), 2.63 - 2.41 (m, 1H), 1.93 - 1.66 (m, 4H), 1.36 - 1.04 (m, 4H).

### 1.2) Synthesis of compound 2388

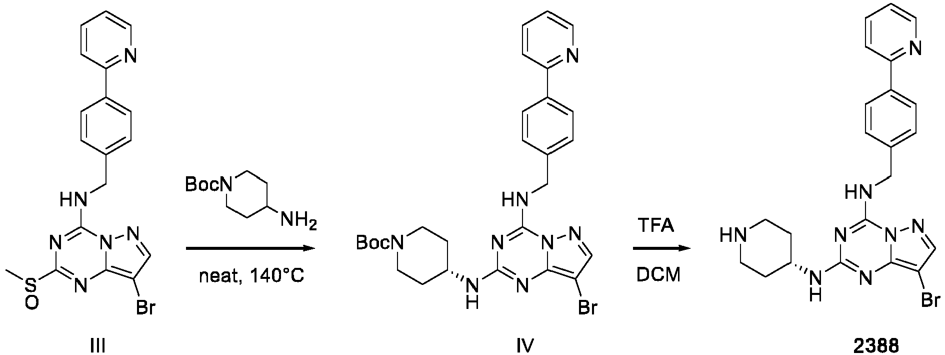

### Synthesis of intermediate compound IV

The described above sulfoxide 8-bromo-2-(methylsulfinyl)-N-(4-(pyridin-2-yl)benzyl)pyrazolo[1,5-a][1,3,5]triazin-4-amine (*compound III*) (0.300 g, 0.68 mmol) and tert-Butyl 4-aminopiperidine-1-carboxylate [CAS 87120-72-7] (3 eq) were finely mixed and warmed for 35 min at 145°C. The crude mixture was partitioned between DCM (20 mL) and aqueous saturated NaHCOs (10 mL) then extract with DCM (20 mL x2) and washed with brine (10 mL). The organic layer was dried on Na₂SO₄, filtered and concentrated in vacuo. The crude was purified by chromatography on silicagel (15g) eluting with DCM/MeOH: gradient from 100/0 to 95/5 to afford *compound IV* (0.288 g, 73% yield, HPLC purity: 99% at 254 nm) as a beige solid (foam). MS m/z (ESI+): Calc for C₂₇H₃₂BrN₈O₂ [M+H]+ = 579.18 ; Exp = 579.2. HPLC Method 2, retention time = 5.86 min

### Synthesis of compound 2388

The Boc-protected *compound IV* tert-butyl 4-((8-bromo-4-((4-(pyridin-2-yl)benzyl)amino)pyrazolo[1,5-a][1,3,5]triazin-2-yl)amino)piperidine-1-carboxylate (257 mg, 0.44 mmol) in DCM (5 mL) was reacted with TFA (1 mL) at room temperature for 30 min. The mixture was concentrated in vacuo then taken up with water and basified with 1M NaOH (pH9-10). The mixture was partitioned between DCM (20 mL) and aqueous saturated NaHCOs (10 mL) then extract with DCM (20 mL x2) and washed with brine (10 mL). The organic layer was dried on Na₂SO₄, filtered and concentrated in vacuo to give 8-bromo-N2-(piperidin-4-yl)-N4-(4-(pyridin-2-yl)benzyl)pyrazolo[1,5-a][1,3,5]triazine-2,4-diamine (*compound* 2388) (0.189 g, 88% yield) as a white foam. MS m/z (ESI+): Calc for C₂₂H₂₄BrN₈ [M+H]+ = 479.1 ; Exp = 479.1. HPLC Method 2, retention time = 3.70 min. ¹H NMR (300 MHz, CDCl3) δ 8.69 (ddd, *J* = 4.9, 1.8, 1.0 Hz, 1H), 7.99 (d, *J* = 8.3 Hz, 2H), 7.79 - 7.69 (m, 2H), 7.46 (d, *J* = 8.2 Hz, 2H), 7.23 (ddd, *J* = 6.8, 4.8, 1.8 Hz, 1H), 6.91 - 6.55 (m, 1H), 5.28 - 4.91 (m, 1H), 4.79 (broad s, 2H), 4.20 - 3.80 (m, 1H), 3.21 - 2.99 (m, 2H), 2.90 - 2.56 (m, 2H), 2.20 - 1.83 (m, 2H), 1.48 - 1.27 (m, 2H). ¹H NMR (300 MHz, DMSO) δ 8.64 (ddd, *J* = 4.8, 1.8, 1.0 Hz, 1H), 8.04 (d, *J* = 7.0 Hz, 2H), 7.96 - 7.89 (m, 2H), 7.86 (td, *J* = 7.6, 1.8 Hz, 1H), 7.53 - 7.42 (m, 2H), 7.33 (ddd, *J* = 7.3, 4.8, 1.3 Hz, 1H), 4.77 - 4.57 (m, 2H), 3.95 - 3.61 (m, 1H), 2.93 (s, 2H), 2.62 - 2.5 (m, 2H), 1.90 - 1.56 (m, 2H), 1.46 - 1.12 (m, 2H).

### 1.3 Synthesis of compound 2568

### Synthesis of intermediate compound tert-butyl (cis-4-((8-bromo-4-((4-(pyridin-2-yl)benzyl)amino)pyrazolo[1,5-a][1,3,5]triazin-2-yl)amino)cyclohexyl)carbamate

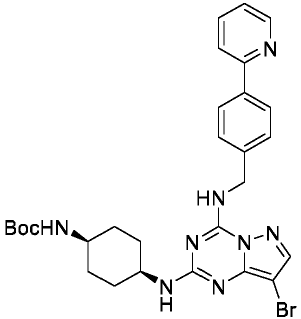

Prepared from N-Boc-cis-1,4-Cyclohexanediamine [CAS n°177906-48-8] and the described above sulfoxide 8-bromo-2-(methylsulfinyl)-N-(4-(pyridin-2-yl)benzyl)pyrazolo[1 ,5-a][1,3,5]triazin-4-amine (*compound III*) (0.100 g, 0.226 mmol) using the same protocol as described above for compound IV. Boc protected compound was obtained as a beige solid (foam) (0.113 g, 84% yield). MS m/z (ESI+): Calc for C₂₈H₃₃BrN₈O₂ [M+H]+ = 593.20 ; Exp = 593.2. HPLC Method 2, retention time = 5.99 min. ¹H NMR (300 MHz, DMSO) δ 8.64 (ddd, *J* = 4.8, 1.8, 1.0 Hz, 1H), 8.04 (d, *J* = 8.0 Hz, 2H), 7.96 - 7.90 (m, 2H), 7.86 (td, *J* = 7.7, 1.8 Hz, 1H), 7.48 (d, *J* = 8.3 Hz, 2H), 7.33 (ddd, *J* = 7.3, 4.8, 1.3 Hz, 1H), 6.61 (s, 1H, NHBoc), 4.78 - 4.60 (m, 2H), 3.89 - 3.68 (m, 1H), 3.39 (broad s, 1H), 1.82 - 1.41 (m, 8H), 1.37 (s, 9H).

### Synthesis of compound 2568

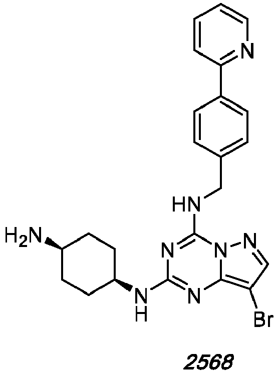

Prepared by TFA deprotection of previous Boc amino compound (0.085 g, 0.143 mmol) as described above in the synthesis procedure of compound *2388.* Compound *2568* was obtained as a white foam (0.069 g, 98% yield). MS m/z (ESI+): Calc for C₂₃H₂₅BrN₈ [M+H]+ = 493.15 ; Exp = 493.1. HPLC Method 2, retention time = 3.85 min. ¹H NMR (300 MHz, DMSO) δ 8.64 (ddd, *J* = 4.8, 1.8, 1.0 Hz, 1H), 8.04 (d, *J* = 7.9 Hz, 2H), 7.96 - 7.89 (m, 2H), 7.86 (td, *J* = 7.6, 1.8 Hz, 1H), 7.48 (d, *J* = 8.0 Hz, 2H), 7.33 (ddd, *J* = 7.2, 4.8, 1.3 Hz, 1H), 8.06 - 8.01 (m, 2H), 2.85 (broad s, 1H), 1.82 - 1.38 (m, 8H)

### 1.4 Synthesis of compound 2584

### Synthesis of Boc protected intermediate compound tert-butyl (4-((8-bromo-4-((4-(pyridin-2-yl)benzyl)amino)pyrazolo[1,5-a][1,3,5]triazin-2-yl)amino)phenyl)carbamate

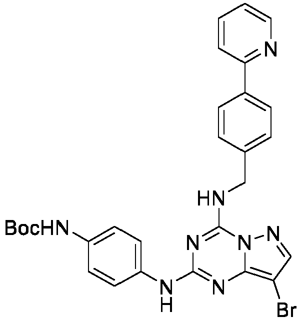

Prepared from *tert*-Butyl (4-aminophenyl)carbamate [CAS n°71026-66-9] and the described above sulfoxide 8-bromo-2-(methylsulfinyl)-N-(4-(pyridin-2-yl)benzyl)pyrazolo[1 ,5-a][1 ,3,5]triazin-4-amine (0.096 g, 0.217 mmol) using the same protocol described for intermediate IV. Boc protected compound was obtained as a beige solid (foam) (0.087 g, 68% yield). MS m/z (ESI+): Calc for C₂₈H₂₈BrN₈O₂ [M+H]+ = 587.15 ; Exp = 587.2. HPLC Method 2, retention time = 6.84 min. ¹H NMR (300 MHz, DMSO) δ 9.52 (broad s, 1H, exch NH), 9.29 (broad s, 1H, exch NH), 9.19 (broad s, 1H, exch NH), 8.65 (ddd, *J* = 4.8, 1.8, 0.9 Hz, 1H), 8.10 - 8.03 (m, 3H), 7.95 (dt, *J* = 8.0, 1.2 Hz, 1H), 7.87 (ddd, *J* = 8.1, 7.3, 1.9 Hz, 1H), 7.51 (d, *J* = 8.2 Hz, 2H), 7.41 - 7.20 (m, 3H), 7.74 - 7.44 (m, 2H), 4.75 (d, *J* = 6.1 Hz, 2H).

### Synthesis of compound 2584

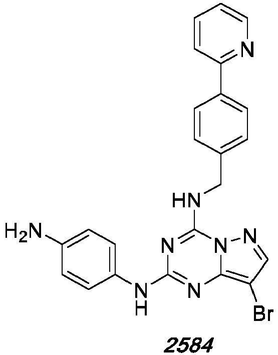

Prepared by TFA deprotection of previous Boc amino compound (0.060 g, 0.102 mmol) as described above in the synthesis procedure of compound 2388. Compound *2584* was obtained as a white foam (0.046 g, 92% yield). MS m/z (ESI+): Calc for C₂₃H₁₉BrN₈ [M+H]+ = 487.10 ; Exp = 487.1. HPLC Method 2, retention time = 4.20 min. ¹H NMR (300 MHz, DMSO) δ 9.19 (broad s, 2H, exch NH), 8.64 (ddd, *J* = 4.8, 1.8, 1.0 Hz, 1H), 8.05 (d, *J* = 8.3 Hz, 2H), 8.01 (s, 1H), 7.96 - 7.90 (m, 1H), 7.86 (td, *J* = 7.6, 1.8 Hz, 1H), 7.48 (d, *J* = 7.9 Hz, 2H), 7.33 (ddd, *J* = 7.3, 4.8, 1.3 Hz, 1H), 7.03 - 7.64 (m, 1H), 6.49 (d, *J* = 8.4 Hz, 2H), 4.79 (s, 2H, exch NH), 4.71 (broad s, 2H).

### 1.5 Synthesis of compound 2559

### Synthesis of Boc protected intermediate compound tert-butyl (1-(8-bromo-4-((4-(pyridin-2-yl)benzyl)amino)pyrazolo[1,5-a][1,3,5]triazin-2-yl)piperidin-4-yl)carbamate

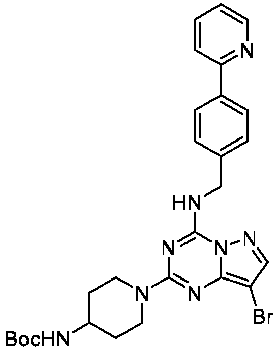

Prepared from 4-(*tert*-Butoxycarbonylamino)piperidine [CAS n°73874-95-0] and the described above sulfoxide 8-bromo-2-(methylsulfinyl)-N-(4-(pyridin-2-yl)benzyl)pyrazolo[1,5-*a*][1,3,5]triazin-4-amine (0.092 g, 0.208 mmol) using the same protocol as described for intermediate *IV.* Boc protected compound was obtained as a foam (0.105 g, 87% yield). MS m/z (ESI+): Calc for C₂₇H₃₁BrN₈O₂ [M+H]+ = 579.18 ; Exp = 579.2. HPLC Method 2, retention time = 6.69 min. ¹H NMR (300 MHz, DMSO) δ 9.18 (t, *J* = 6.2 Hz, 1H), 8.64 (ddd, *J* = 4.8, 1.8, 1.0 Hz, 1H), 8.04 (d, *J* = 8.3 Hz, 2H), 7.97 (s, 1H), 7.92 (dt, *J* = 8.1, 1.2 Hz, 1H), 7.86 (ddd, *J* = 8.1, 7.2, 1.8 Hz, 1H), 7.50 (d, *J* = 8.3 Hz, 2H), 7.33 (ddd, *J* = 7.2, 4.8, 1.3 Hz, 1H), 6.82 (d, *J* = 8.0 Hz, 1H), 4.66 (d, *J* = 6.1 Hz, 2H), 4.59 - 4.49 (m, 2H), 3.60 - 3.38 (m, 1H), 3.06 - 2.92 (m, 2H), 1.81 - 1.70 (m, 2H), 1.37 (s, 9H), 1.32 - 1.15 (m, 2H).

### Synthesis of compound 2559

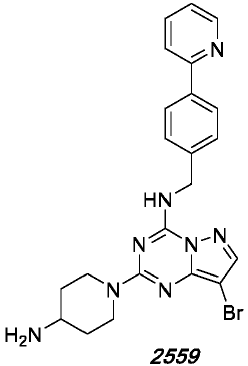

Prepared by TFA deprotection of previous Boc amino compound (0.100 g, 0.173 mmol) as described above in the synthesis procedure of compound *2388.* Compound *2559* was obtained as a white foam (0.072 g, 87% yield). MS m/z (ESI+): Calc for C₂₂H₂₃BrN₈ [M+H]+ = 479.13; Exp = 479.1. HPLC Method 2, retention time = 3.91 min. ¹H NMR (300 MHz, DMSO) δ 8.64 (ddd, *J* = 4.8, 1.8, 0.9 Hz, 1H), 8.03 (d, *J* = 8.3 Hz, 2H), 7.96 (s, 1H), 7.92 (dt, *J* = 8.1, 1.2 Hz, 1H), 7.86 (ddd, *J* = 8.0, 7.2, 1.8 Hz, 1H), 7.50 (d, *J=* 8.4 Hz, 2H), 7.33 (ddd, *J* = 7.3, 4.8, 1.3 Hz, 1H), 4.66 (s, 2H), 4.55 - 4.41 (m, 2H), 3.08 - 2.92 (m, 2H), 2.85 - 2.68 (m, 1H), 1.80 - 1.62 (m, 2H), 1.19 - 1.01 (m, 2H). Exchangeable NH, NH2 were not visible.

### 1.6 Synthesis of compound 2622

### Synthesis of Boc protected intermediate compound tert-butyl 4-(1-(8-bromo-4-((4-(pyridin-2-yl)benzyl)amino)pyrazolo[1,5-a] [1,3,5]triazin-2-yl)piperidin-4-yl)piperazine-1 - carboxylate

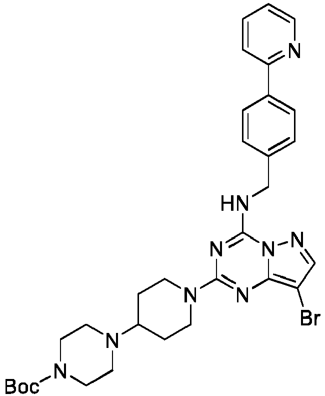

Prepared from 1,1-Dimethylethyl 4-(4-piperidinyl)-1-piperazinecarboxylate [CAS n°205059-24-1] and the described above sulfoxide 8-bromo-2-(methylsulfinyl)-N-(4-(pyridin-2-yl)benzyl)pyrazolo[1,5-*a*][1,3,5]triazin-4-amine (0.090 g, 0.203 mmol) using the same protocol as described for intermediate *IV.* Boc protected compound was obtained as a foam (0.094 g, 71% yield). MS m/z (ESI+): Calc for C₃₁H₃₈BrN₉O₂ [M+H]+ = 648.24 ; Exp = 648.2. HPLC Method 2, retention time = 5.29 min. ¹H NMR (300 MHz, DMSO) δ 9.19 (t, *J* = 6.1 Hz, 1H), 8.64 (ddd, *J* = 4.8, 1.8, 1.0 Hz, 1H), 8.04 (d, *J* = 8.3 Hz, 2H), 7.98 (s, 1H), 7.92 (dt, *J* = 8.1, 1.2 Hz, 1H), 7.85 (ddd, *J* = 8.0, 7.3, 1.8 Hz, 1H), 7.48 (d, *J* = 8.3 Hz, 2H), 7.32 (ddd, *J* = 7.3, 4.8, 1.3 Hz, 1H), 4.71 - 4.56 (m, 4H), 3.29 - 3.14 (m, 4H), 2.88 - 2.76 (m, 1H), 2.45 - 2.28 (m, 4H), 1.81 - 1.63 (m, 2H), 1.38 (s, 9H), 1.26 - 1.09 (m, 2H).

### Synthesis of compound 2622

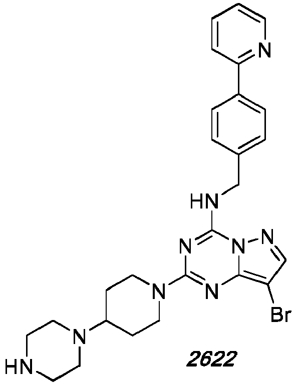

Prepared by TFA deprotection of previous Boc amino compound (0.090 g, 0.139 mmol) as described above in the synthesis procedure of compound 2388, and purified by chromatography on silica gel (gradient 1% to 40% MeOH in DCM). Compound 2622 was obtained as a white foam (0.029 g, 38% yield). MS m/z (ESI+): Calc for C₂₆H₃₀BrN₉ [M+H]+ = 548.19 ; Exp = 548.1. HPLC Method 2, retention time = 3.95 min. ¹H NMR (300 MHz, DMSO) δ 9.18 (bs, 1H), 8.64 (ddd, *J* = 4.8, 1.8, 1.0 Hz, 1H), 8.04 (d, *J* = 8.4 Hz, 2H), 7.97 (s, 1H), 7.92 (dt, J = 8.1, 1.2 Hz, 1H), 7.86 (ddd, *J* = 8.0, 7.2, 1.8 Hz, 1H), 7.49 (d, *J* = 8.4 Hz, 2H), 7.33 (ddd, *J* = 7.2, 4.8, 1.3 Hz, 1H), 4.74 - 4.56 (m, 4H), 2.90 - 2.75 (m, 2H), 2.67 - 2.55 (m, 4H), 2.45 - 1.08 (m, 9H).

### 1.7 Synthesis of compound 2569

### Synthesis of Boc protected intermediate compound tert-butyl 4-(((8-bromo-4-((4-(pyridin-2-yl)benzyl)amino)pyrazolo[1,5-a][1,3,5]triazin-2-yl)amino)methyl)piperidine-1-carboxylate

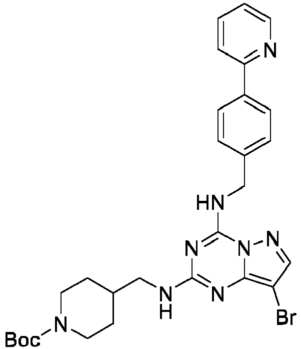

Prepared from [(Piperidin-4-yl)methyl]carbamic acid tert-butyl ester [CAS n°135632-53-0] and the described above sulfoxide 8-bromo-2-(methylsulfinyl)-N-(4-(pyridin-2-yl)benzyl)pyrazolo[1,5-a][1,3,5]triazin-4-amine (0.088 g, 0.199 mmol) using the same protocol as described for intermediate IV. Boc protected compound was obtained as a colorless resin (0.108 g, 92% yield). MS m/z (ESI+): Calc for C₂₈H₃₃BrN₈O₂ [M+H]+ = 593.20 ; Exp = 593.2. HPLC Method 2, retention time = 5.82 min. ¹H NMR (300 MHz, DMSO) δ 8.64 (d, *J* = 4.8 Hz, 1H), 8.04 (d, *J* = 8.3 Hz, 2H), 7.97 - 7.81 (m, 3H), 7.46 (d, *J* = 8.1 Hz, 2H), 7.37 - 7.30 (m, 1H), 4.68 (s, 2H), 4.00 - 3.71 (m, 2H), 3.13 (d, *J* = 9.2 Hz, 2H), 2.73 - 2.60 (m, 1H), 1.81 - 1.23 (m, 13H), 1.11 - 0.83 (m, 2H).

### Synthesis of compound 2569

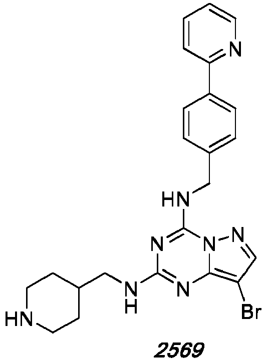

Prepared by TFA deprotection of previous Boc amino compound (0.110 g, 0.185 mmol) as described above in the synthesis procedure of compound 2388. Compound *2569* was obtained as a colorless foam (0.075 g, 82% yield). MS m/z (ESI+): Calc for C₂₃H₂₅BrN₈ [M+H]+ = 493.15 ; Exp = 493.1. HPLC Method 2, retention time = 3.62 min. ¹H NMR (300 MHz, DMSO) δ 8.65 (dt, *J* = 4.9, 1.3 Hz, 1H), 8.04 (d, *J* = 8.4 Hz, 2H), 7.96 (s, 1H), 7.93 (dt, *J* = 8.0, 1.2 Hz, 1H), 7.90 - 7.83 (m, 1H), 7.52 - 7.43 (m, 2H), , 7.34 (ddd, *J* = 7.2, 4.8, 1.3 Hz, 1H), 4.69 (s, 2H), 3.24 - 3.09 (m, 5H), 2.83 - 2.68 - 2.56 (m, 2H), 1.89 - 1.55 (m, 2H), 1.38 - 1.11 (m, 2H).

### 1.8 Synthesis of compound 2560

### Synthesis of Boc protected intermediate compound tert-butyl ((1-(8-bromo-4-((4-(pyridin-2-yl)benzyl)amino)pyrazolo[1,5-a][1,3,5]triazin-2-yl)piperidin-4-yl)methyl)carbamate

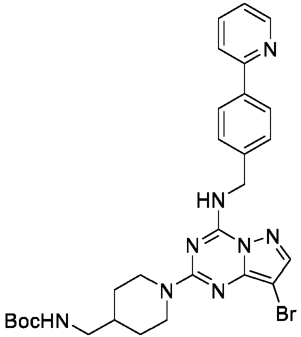

Prepared from [(Piperidin-4-yl)methyl]carbamic acid tert-butyl ester [CAS n°135632-53-0] and the described above sulfoxide 8-bromo-2-(methylsulfinyl)-N-(4-(pyridin-2-yl)benzyl)pyrazolo[1,5-a][1,3,5]triazin-4-amine (0.077 g, 0.174 mmol) using the same protocol as described for intermediate IV. Boc protected compound was obtained as a colorless foam, (0.087 g, 84% yield). MS m/z (ESI+): Calc for C₂₈H₃₃BrN₈O₂ [M+H]+ = 593.20 ; Exp = 593.2. HPLC Method 2, retention time = 6.89 min. ¹H NMR (300 MHz, DMSO) δ 9.14 (t, *J* = 6.2 Hz, 1H), 8.64 (ddd, *J* = 4.8, 1.9, 1.0 Hz, 1H), 8.03 (d, *J* = 8.3 Hz, 2H), 7.96 (s, 1H), 7.93 (dt, *J* = 8.1, 1.2 Hz, 1H), 7.86 (ddd, *J* = 8.0, 7.3, 1.8 Hz, 2H), 7.50 (d, *J* = 8.3 Hz, 2H), 7.33 (ddd, *J* = 7.3, 4.8, 1.3 Hz, 1H), 6.87 (t, *J* = 5.9 Hz, 1H), 4.77 - 4.52 (m, 4H), 2.91 - 2.76 (m, 4H), 1.71 - 1.53 (m, 3H), 1.36 (s, 9H), 1.11 - 0.88 (m, 2H).

### Synthesis of compound 2560

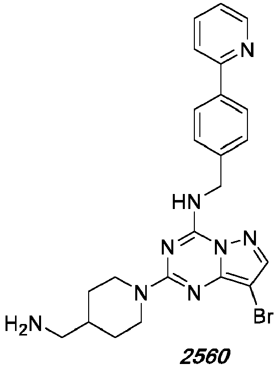

Prepared by TFA deprotection of previous Boc amino compound (0.083 g, 0.140 mmol) as described above in the synthesis procedure of compound 2388. Compound *2560* was obtained as a white foam (0.057 g, 83% yield). MS m/z (ESI+): Calc for C₂₃H₂₅BrN₈ [M+H]+ = 493.15 ; Exp = 493.1. HPLC Method 2, retention time = 4.17 min. ¹H NMR (300 MHz, DMSO) δ 8.64 (ddd, *J* = 4.8, 1.8, 1.0 Hz, 1H), 8.04 (d, *J* = 8.3 Hz, 1H), 7.97 (s, 1H), 7.93 (dt, *J* = 8.1, 1.3 Hz, 1H), 7.86 (ddd, *J* = 8.0, 7.2, 1.8 Hz, 1H), 7.49 (d, *J* = 8.3 Hz, 2H), 7.33 (ddd, *J* = 7.2, 4.8, 1.4 Hz, 1H), 4.73 - 4.57 (m, 4H), 2.92 - 2.76 (m, 2H), 2.66 - 2.54 (m, 2H), 1.79 - 1.62 (m, 2H), 1.62 - 1.47 (m, 1H), 1.09 - 0.88 (m, 2H).

### 1.9 Synthesis of compound 2606

### Synthesis of Boc protected intermediate compound tert-butyl 3-((8-bromo-4-((4-(pyridin-2-yl)benzyl)amino)pyrazolo[1,5-a][1,3,5]triazin-2-yl)amino)piperidine-1 -carboxylate

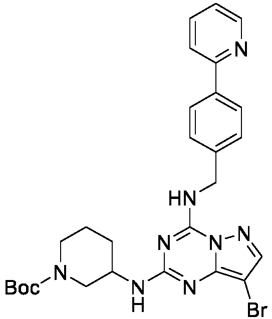

Prepared from 1-Piperidinecarboxylic acid, 3-amino-, 1,1-dimethylethyl ester [CAS n°184637-48-7] and the described above sulfoxide 8-bromo-2-(methylsulfinyl)-N-(4-(pyridin-2-yl)benzyl)pyrazolo[1,5-*a*][1,3,5]triazin-4-amine (0.085 g, 0.192 mmol) using the same protocol as described for intermediate IV. Boc protected compound was obtained as a white solid (foam) (0.075 g, 67% yield). MS m/z (ESI+): Calc for C₂₇H₃₁BrN₈O₂ [M+H]+ = 579.18 ; Exp = 579.2. HPLC Method 2, retention time = 6.15 min.

### Synthesis of compound 2606 (racemic)

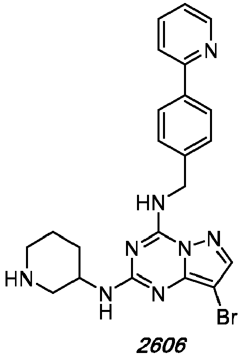

The Boc protected compound was treated with TFA as described above in the synthesis procedure of compound 2388. Compound 2606 (racemic) obtained as a white solid (0.045 g, 74% yield). MS m/z (ESI+): Calc for C₂₂H₂₃BrN₈ [M+H]+ = 479.13; Exp = 479.1. HPLC Method 2, retention time = 3.75 min. ¹H NMR (300 MHz, DMSO) δ 8.64 (ddd, *J* = 4.8, 1.8, 1.0 Hz, 1H), 8.04 (d, *J* = 8.3 Hz, 2H), 7.97 - 7.88 (m, 2H), 7.86 (ddd, *J* = 8.1, 7.2, 1.8 Hz, 1H), 7.56 - 7.42 (m, 2H), 7.33 (ddd, *J* = 7.2, 4.8, 1.3 Hz, 1H), 4.78 - 4.56 (m, 2H), 3.91 - 3.67 (m, 1H), 3.06 - 2.89 (m, 1H), 2.83 - 2.70 (m, 1H), 2.44 - 2.25 (m, 2H), 1.91 - 1.71 (m, 1H), 1.65 - 1.51 (m, 1H), 1.49 - 1.28 (m, 2H).

### Synthesis of compound 2659 (enantiomer S) and 2660 (enantiomer R)

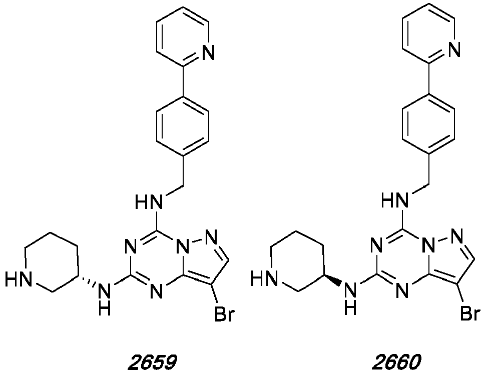

8-bromo-N2-[(3R)-piperidin-3-yl]-N4-{[4-(pyridin-2-yl)phenyl]methyl}pyrazolo[1,5-a][1 ,3,5]triazine-2,4-diamine: *2660 (enantiomer R) and* 8-bromo-N2-[(3R)-piperidin-3-yl]-N4-{[4-(pyridin-2-yl)phenyl]methyl}pyrazolo[1 ,5-a][1 ,3,5]triazine-2,4-diamine 2659 *(enantiomer S)* were prepared from optically active Boc amine in the same way with 92% and 86% respectively from corresponding (3R)-3-aminopiperidine-1-carboxylate and (3S)-3-aminopiperidine-1-carboxylate.

### 1.10 Synthesis of compound 2538

### Synthesis of Boc protected (2S,4R)-tert-butyl 2-(((8-bromo-4-((4-(pyridin-2-yl)benzyl)amino)pyrazolo[1,5 -a] [1,3,5]triazin-2-yl)amino)methyl)-4-hydroxypyrrolidine-1 - carboxylate

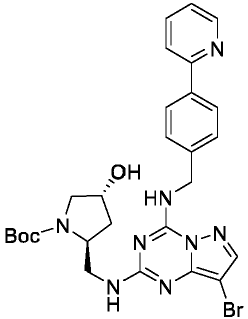

Prepared from (2R,4S)-1-Pyrrolidinecarboxylic acid, 2-(aminomethyl)-4-hydroxy-, 1,1-dimethylethyl ester [CAS n°1983918-82-6] and the described above sulfoxide 8-bromo-2-(methylsulfinyl)-N-(4-(pyridin-2-yl)benzyl)pyrazolo[1,5-a][1,3,5]triazin-4-amine (0.080 g, 0.180 mmol) using the same protocol as described intermediate IV. Boc protected compound was obtained as a white solid (0.086 g, 80% yield). MS m/z (ESI+): Calc for C₂₇H₃₁BrN₈O₃ [M+H]+ = 595.18 ; Exp = 595.2. HPLC Method 2, retention time = 5.16 min. ¹H NMR (300 MHz, DMSO) δ 8.64 (ddd, *J* = 4.9, 1.8, 1.0 Hz, 1H), 8.03 (d, *J* = 8.0 Hz, 2H), 7.95 (s, 1H), 7.92 (d, *J* = 7.9 Hz, 1H), 7.86 (td, *J* = 7.6, 1.8 Hz, 0H), 7.60 - 7.52 (m, 0.5H, exch NH), 7.47 (d, *J* = 7.9 Hz, 2H), 7.33 (ddd, *J* = 7.2, 4.8, 1.4 Hz, 1H), 4.93 - 4.62 (m, 3H, exch OH, CH2), 4.29 - 3.90 (m, 2H), 3.83 - 3.44 (m, 1H), 3.32 - 3.10 (m, 3H), 1.98 - 1.67 (m, 2H), 1.47 - 1.21 (m, 9H).

### Synthesis of compound 2538

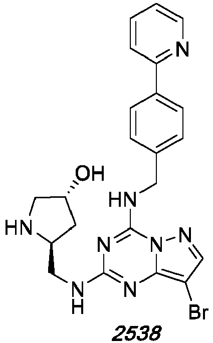

Prepared by TFA deprotection of previous Boc amino compound (0.080 g, 0.134 mmol) as described above in the synthesis procedure of compound 2388. Compound 2538 was obtained as a grey solid (0.066 g, 98% yield). MS m/z (ESI+): Calc for C₂₂H₂₃BrN₈O [M+H]+ = 495.13 ; Exp = 495.1. HPLC Method 2, retention time = 3.75 min. ¹H NMR (300 MHz, DMSO) δ 8.65 (ddd, *J* = 4.8, 1.8, 0.9 Hz, 1H), 8.04 (d, *J* = 8.3 Hz, 2H), 7.95 - 7.91 (m, 2H), 7.86 (ddd, *J* = 8.0, 7.2, 1.8 Hz, 1H), 7.54 - 7.42 (m, 2H), 7.33 (ddd, *J* = 7.3, 4.8, 1.3 Hz, 1H), 4.68 (s, 2H), 4.58 - 4.47 (m, 1H), 4.21 - 4.02 (m, 1H), 3.51 - 3.36 (m, 1H), 3.28 - 3.10 (m, 2H), 2.96 - 2.78 (m, 1H), 1.56 (d, *J* = 56.1 Hz, 4H).

### 1.11 Synthesis of compound 2571

### Synthesis of Boc protected (S)-tert-butyl 2-(((8-bromo-4-((4-(pyridin-2-yl)benzyl)amino)pyrazolo[1,5-a][1,3,5]triazin-2-yl)amino)methyl)pyrrolidine-1-carboxylate

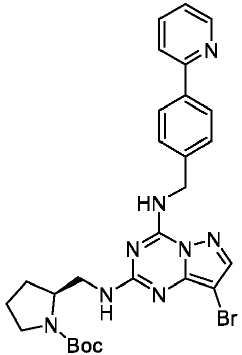

Prepared from (*S*)-*tert*-Butyl 2-(aminomethyl)pyrrolidine-1-carboxylate [CAS n°119020-01-8] and the described above sulfoxide 8-bromo-2-(methylsulfinyl)-N-(4-(pyridin-2-yl)benzyl)pyrazolo[1 ,5-a][1,3,5]triazin-4-amine (0.087 g, 0.196 mmol) using the same protocol as described for intermediate IV. Boc protected compound was obtained as a colorless solid (foam) (0.089 g, 78% yield). HRMS m/z (ESI+): Calc for C₂₇H₃₁BrN₈O₂ [M+H]+ = 579.1826 ; Exp = 579.1827. HPLC Method 2, retention time = 6.02 min. ¹H NMR (300 MHz, DMSO) δ 8.64 (ddd, *J* = 4.8, 1.8, 1.0 Hz, 1H), 8.03 (d, *J* = 7.9 Hz, 2H), 7.95 (s, 1H), 7.92 (broad d, *J* = 8.0 Hz, 1H), 7.86 (td, *J* = 7.6, 1.8 Hz, 1H), 7.46 (d, *J* = 8.1 Hz, 2H), 7.33 (ddd, *J* = 7.2, 4.8, 1.3 Hz, 1H), 4.94 - 4.61 (m, 2H), 4.02 - 3.78 (m, 1H), 3.32 (s, 4H), 1.94 - 1.56 (m, 4H), 1.45 - 1.22 (m, 9H).

### Synthesis of compound 2571

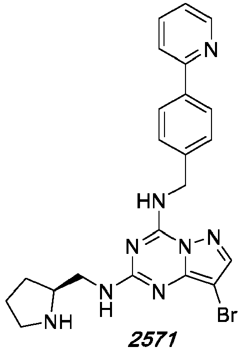

Prepared by TFA deprotection of previous Boc amino compound (0.087 g, 0.150 mmol) as described above in the synthesis procedure of compound 2388. Compound 2571 was obtained as a white foam (0.070 g, 97% yield). MS m/z (ESI+): Calc for C₂₂H₂₃BrN₈ [M+H]+ = 479.1302 ; Exp = 479.1301. HPLC Method 2, retention time = 3.88 min. ¹H NMR (300 MHz, DMSO) δ 9.43 - 8.80 (m, 1H), 8.64 (ddd, *J* = 4.8, 1.8, 1.0 Hz, 1H), 8.04 (d, *J* = 8.4 Hz, 2H), 7.95 (s, 1H), 7.93 (dt, *J* = 8.0, 1.3 Hz, 1H), 7.86 (ddd, *J* = 8.0, 7.2, 1.8 Hz, 1H), 7.47 (broad d, *J* = 7.9 Hz, 2H), 7.33 (ddd, *J* = 7.3, 4.8, 1.3 Hz, 1H), 4.68 (s, 2H), 3.47 - 2.98 (m, 4H), 2.91 - 2.58 (m, 2H), 1.83 - 1.20 (m, 5H).

### 1.12 Synthesis of compound 2585

### Synthesis of Boc protected tert-butyl (4-((8-bromo-4-((4-(pyridin-2-yl)benzyl)amino)pyrazolo[1,5-a][1,3,5]triazin-2-yl)amino)butyl)carbamate

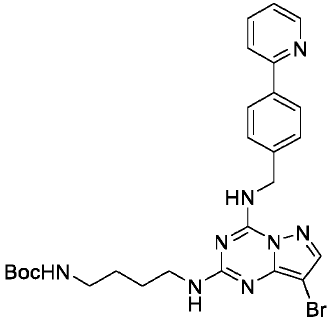

Prepared from *N*-(*tert*-Butoxycarbonyl)-1,4-butanediamine [CAS n°68076-36-8] and the described above sulfoxide 8-bromo-2-(methylsulfinyl)-N-(4-(pyridin-2-yl)benzyl)pyrazolo[1 ,5-*a*][1,3,5]triazin-4-amine (0.100 g, 0.226 mmol) using the same protocol as described for intermediate IV. Boc protected compound was obtained as a white foam (0.113 g, 88% yield). MS m/z (ESI+): Calc for C₂₆H₃₁BrN₈O₂ [M+H]+ = 567.18 ; Exp = 567.2. HPLC Method 2, retention time = 5.49min. ¹H NMR (300 MHz, DMSO) δ 8.65 (ddd, *J* = 4.8, 1.8, 1.0 Hz, 1H), 8.05 (d, *J* = 8.1 Hz, 2H), 7.97 - 7.91 (m, 2H), 7.90 - 7.83 (m, 1H), 7.55 - 7.41 (m, 2H), 7.34 (ddd, *J* = 7.3, 4.8, 1.3 Hz, 1H), 4.77 - 4.63 (m, 2H), 3.31 - 3.18 (m, 2H), 3.01 - 2.83 (m, 2H), 1.53 - 1.29 (m, 13H).

### Synthesis of compound 2585

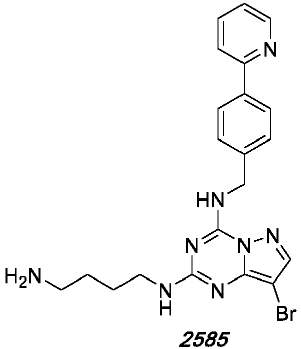

Prepared by TFA deprotection of previous Boc amino compound (0.085 g, 0.143 mmol) as described above in the synthesis procedure of compound 2388. Compound 2585 was obtained as a white foam (0.069 g, 98% yield). MS m/z (ESI+): Calc for C₂₁H₂₃BrN₈ [M+H]+ = 467.12 ; Exp = 467.1. HPLC Method 2, retention time = 3.56 min. ¹H NMR (300 MHz, DMSO) δ 8.65 (ddd, *J* = 4.9, 1.8, 1.0 Hz, 1H), 8.05 (d, *J* = 8.3 Hz, 2H), 7.96 (s, 1H), 7.93 (dt, *J* = 8.1, 1.2 Hz, 1H), 7.87 (ddd, *J* = 8.0, 7.3, 1.8 Hz, 1H), 7.54 - 7.42 (m, 2H), 7.34 (ddd, *J* = 7.2, 4.8, 1.4 Hz, 1H), 4.69 (s, 2H), 3.31 - 3.21 (m, 2H), 2.87 - 2.74 (m, 2H), 1.63 - 1.42 (m, 4H).

### 1.13 Synthesis of compound 2638

### Synthesis of Boc protected tert-butyl (2-((8-bromo-4-((4-(pyridin-2-yl)benzyl)amino)pyrazolo[1,5-a][1,3,5]triazin-2-yl)amino)ethyl)carbamate

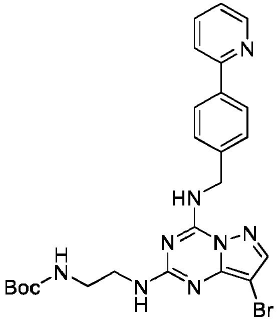

Prepared from 1,1-Dimethylethyl N-(2-aminoethyl)carbamate [CAS n° 57260-73-8] and the described above sulfoxide 8-bromo-2-(methylsulfinyl)-N-(4-(pyridin-2-yl)benzyl)pyrazolo[1,5-a][1,3,5]triazin-4-amine (0.074 g, 0.167 mmol) using the same protocol as described for intermediate IV. Boc protected compound was obtained as a white solid (foam) (0.075 g, 83% yield). MS m/z (ESI+): Calc for C₂₄H₂₇BrN₈O₂ [M+H]+ = 539.15 ; Exp = 539.1. HPLC Method 2, retention time = 5.15 min. ¹H NMR (300 MHz, DMSO) δ 8.66 (ddd, *J* = 4.8, 1.8, 1.0 Hz, 1H), 8.05 (d, *J* = 8.1 Hz, 2H), 7.99 - 7.91 (m, 2H), 7.87 (td, *J* = 7.6, 1.8 Hz, 1H), 7.60 - 7.43 (m, 2H), 7.34 (ddd, *J* = 7.2, 4.8, 1.3 Hz, 1H), 6.92 - 6.79 (m, 1H), 4.78 - 4.63 (m, 2H), 3.33 - 3.21 (m, 2H), 3.21 - 3.01 (m, 2H), 1.37 (s, 9H).

### Synthesis of compound 2638

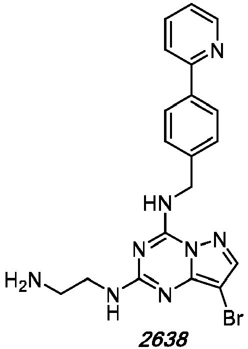

Prepared by TFA deprotection of previous Boc amino compound (0.071 g, 0.132 mmol) as described above in the synthesis procedure of compound 2388 and purified by chromatography on silicagel (gradient 1% to 60% MeOH/DCM). Compound 2638 was obtained as a white solid (0.041 g, 71% yield). MS m/z (ESI+): Calc for C₁₉H₁₉BrN₈ [M+H]+ = 439.10 ; Exp = 439.1. HPLC Method 2, retention time = 3.52 min. ¹H NMR (300 MHz, DMSO) δ 8.64 (ddd, *J* = 4.8, 1.8, 1.0 Hz, 1H), 8.04 (d, *J* = 8.3 Hz, 2H), 7.97 - 7.90 (m, 2H), 7.86 (ddd, *J* = 8.1, 7.3, 1.8 Hz, 1H), 7.47 (d, *J* = 8.6 Hz, 2H), 7.33 (ddd, *J* = 7.2, 4.8, 1.3 Hz, 1H), 4.68 (s, 2H), 3.32 - 3.20 (m, 2H), 2.75 - 2.56 (m, 2H).

### 1.14 Synthesis of compound 2586

### Synthesis of Boc protected tert-butyl (2-((8-bromo-4-((4-(pyridin-2-yl)benzyl)amino)pyrazolo[1,5-a][1,3,5]triazin-2-yl)amino)ethyl)(methyl)carbamate

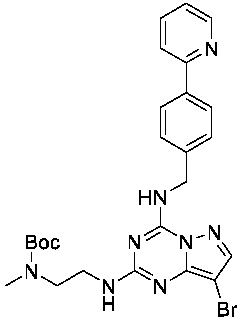

Prepared from 1,1-Dimethylethyl *N*-(2-aminoethyl)-N-methylcarbamate [CAS n°121492-06-6] and the described above sulfoxide 8-bromo-2-(methylsulfinyl)-N-(4-(pyridin-2-yl)benzyl)pyrazolo[1,5-a][1,3,5]triazin-4-amine (0.100 g, 0.226 mmol) using the same protocol as described for intermediate IV. Boc protected compound was obtained as a white solid (foam) (0.113 g, 90% yield). MS m/z (ESI+): Calc for C₂₅H₂₉BrN₈O₂ [M+H]+ = 553.17 ; Exp = 553.2. HPLC Method 2, retention time = 5.58 min. ¹H NMR (300 MHz, DMSO) δ 8.68 - 8.63 (m, 1H), 8.04 (d, *J* = 7.9 Hz, 2H), 7.97 (s, 1H), 7.96 - 7.91 (m, 1H), 7.87 (td, *J* = 7.7, 1.8 Hz, 1H), 7.53 - 7.43 (m, 3H), 7.34 (ddd, *J* = 7.3, 4.8, 1.3 Hz, 1H), 4.70 (broad s, 2H), 3.50 - 3.25 (m, 3H), 2.92 - 2.66 (m, 2H), 1.45 - 1.06 (m, 11H).

### Synthesis of compound 2586

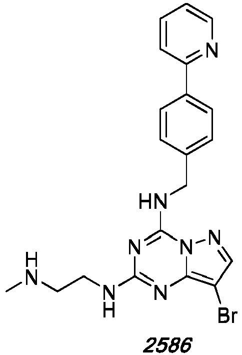

Prepared by TFA deprotection of previous Boc amino compound (0.104 g, 0.188 mmol) as described above in the synthesis procedure of compound 2388. Compound 2586 was obtained as a white solid (0.076 g, 89% yield). MS m/z (ESI+): Calc for C₂₀H₂₁BrN₈ [M+H]+ = 453.11 ; Exp = 453.1. HPLC Method 2, retention time = 3.52 min. ¹H NMR (300 MHz, DMSO) δ 9.30 - 8.81 (m, 1H), 8.65 (ddd, *J* = 4.8, 1.8, 1.0 Hz, 1H), 8.05 (d, *J* = 8.3 Hz, 2H), 7.97 - 7.90 (m, 2H), 7.86 (td, *J* = 7.6, 1.8 Hz, 1H), 7.54 - 7.43 (m, 2H), 7.34 (ddd, *J* = 7.3, 4.8, 1.3 Hz, 1H), 4.69 (s, 2H), 3.39 - 3.33 (m, 2H), 2.71 - 2.55 (m, 2H), 2.38 - 2.15 (m, 3H).

### 1.15 Synthesis of compound 2600

### Synthesis of Boc protected tert-butyl (2-((8-bromo-4-((4-(pyridin-2-yl)benzyl)amino)pyrazolo[1,5-a][1,3,5]triazin-2-yl)(methyl)amino)ethyl)carbamate

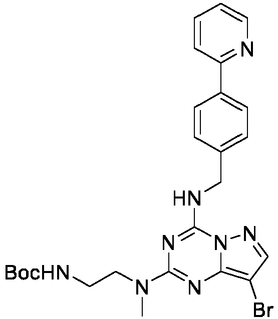

Prepared from 1,1-Dimethylethyl *N*-[2-(methylamino)ethyl]carbamate [CAS n°122734-32-1] and the described above sulfoxide 8-bromo-2-(methylsulfinyl)-N-(4-(pyridin-2-yl)benzyl)pyrazolo[1,5-*a*][1,3,5]triazin-4-amine (0.092 g, 0.208 mmol) using the same protocol as described for intermediate IV. Boc protected compound was obtained as a white solid (foam) (0.098 g, 85% yield). MS m/z (ESI+): Calc for C₂₅H₂₉BrN₈O₂ [M+H]+ = 553.17 ; Exp = 553.2. HPLC Method 2, retention time = 6.23 min. ¹H NMR (300 MHz, DMSO) δ 9.14 (t, *J* = 6.1 Hz, 1H), 8.64 (ddd, *J* = 4.8, 1.8, 1.0 Hz, 1H), 8.03 (d, *J* = 8.3 Hz, 2H), 7.96 (s, 1H), 7.92 (dt, *J* = 8.1, 1.3 Hz, 1H), 7.86 (ddd, *J* = 8.1, 7.2, 1.8 Hz, 1H), 7.52 (d, *J* = 7.9 Hz, 2H), 7.33 (ddd, *J* = 7.2, 4.8, 1.4 Hz, 1H), 6.93 - 6.69 (m, 1H), 4.69 (d, *J* = 6.1 Hz, 2H), 3.60 (t, *J* = 6.4 Hz, 2H), 3.18 - 3.02 (m, 5H), 1.33 (s, 9H).

### Synthesis of compound 2600

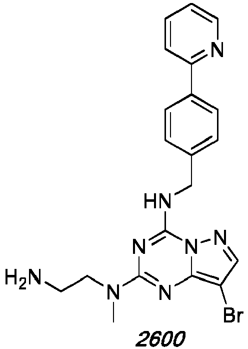

Prepared by TFA deprotection of previous Boc amino compound (0.092 g, 0.166 mmol) as described above in the synthesis procedure of compound 2388. Compound 2600 was obtained as a white foam (0.072 g, 95% yield). MS m/z (ESI+): Calc for C₂₀H₂₁BrN₈ [M+H]+ = 453.11 ; Exp = 453.1. HPLC Method 2, retention time = 3.99 min. ¹H NMR (300 MHz, DMSO) δ 8.64 (ddd, *J* = 4.8, 1.8, 1.0 Hz, 1H), 8.04 (d, *J* = 8.3 Hz, 2H), 7.95 (s, 1H), 7.92 (dt, *J* = 8.1, 1.2 Hz, 1H), 7.86 (ddd, *J* = 8.0, 7.2, 1.8 Hz, 1H), 7.51 (d, *J* = 8.4 Hz, 2H), 7.33 (ddd, *J* = 7.3, 4.8, 1.3 Hz, 1H), 4.67 (s, 2H), 3.54 (t, *J* = 6.7 Hz, 2H), 3.09 (broad s, 3H), 2.82 - 2.58 (m, 2H).

### 1.16 Synthesis of compound 2605

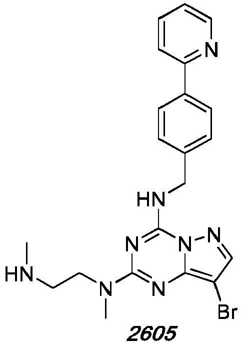

Prepared from sulfoxide compound III (0.082 g, 0.185 mmol) and N,N'-dimethylamino ethylenediamine as described above for compound 2384. Compound 2605 was obtained as a white foam (0.050 g, 58% yield). MS m/z (ESI+): Calc for C₂₁H₂₃BrN₈ [M+H]+ = 467.13 ; Exp = 467.1. HPLC Method 2, retention time = 4.09 min. ¹H NMR (300 MHz, DMSO) δ z8.64 (ddd, *J* = 4.8, 1.9, 1.0 Hz, 1H), 8.04 (d, *J* = 8.3 Hz, 2H), 7.96 (s, 1H), 7.92 (dt, *J* = 8.1, 1.2 Hz, 1H), 7.86 (ddd, *J* = 8.1, 7.2, 1.8 Hz, 1H), 7.49 (broad d, *J* = 8.0 Hz, 2H), 7.33 (ddd, *J* = 7.2, 4.8, 1.4 Hz, 1H), 4.67 (broad s, 2H), 3.63 (t, *J* = 6.5 Hz, 2H), 3.08 (broad s, 3H), 2.70 - 2.54 (m, 2H), 2.34 - 2.09 (m, 3H).

### 1.17 Synthesis of compound 2601

### Synthesis of Boc protected tert-butyl 4-(8-bromo-4-((4-(pyridin-2-yl)benzyl)amino)pyrazolo[1,5-a][1,3,5]triazin-2-yl)piperazine-1-carboxylate

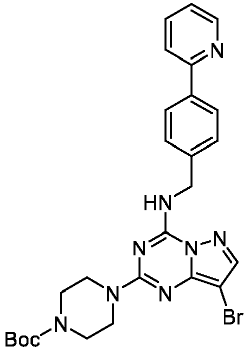

Prepared from Boc piperazine [CAS n°57260-71-6] and the described above sulfoxide 8-bromo-2-(methylsulfinyl)-N-(4-(pyridin-2-yl)benzyl)pyrazolo[1,5-a][1,3,5]triazin-4-amine (0.091 g, 0.205 mmol) using the same protocol as described for intermediate IV. Boc protected compound was obtained as a white solid (foam) (0.098 g, 84% yield). MS m/z (ESI+): Calc for C₂₆H₂₉BrN₈O₂ [M+H]+ = 565.17 ; Exp = 565.2. HPLC Method 2, retention time = 7.08 min. ¹H NMR (300 MHz, DMSO) δ 9.24 (t, *J* = 6.1 Hz, 1H), 8.64 (ddd, *J* = 4.8, 1.8, 1.0 Hz, 1H), 8.04 (d, *J* = 8.4 Hz, 2H), 8.00 (s, 1H), 7.93 (dt, *J* = 8.1, 1.2 Hz, 1H), 7.86 (ddd, *J* = 8.0, 7.2, 1.8 Hz, 1H), 7.51 (d, *J* = 8.3 Hz, 2H), 7.33 (ddd, *J* = 7.2, 4.8, 1.3 Hz, 1H), 4.68 (d, *J* = 5.9 Hz, 2H), 3.76 - 3.69 (m, 4H), 3.42 - 3.32 (m, 4H), 1.40 (s, 9H).

### Synthesis of compound 2601

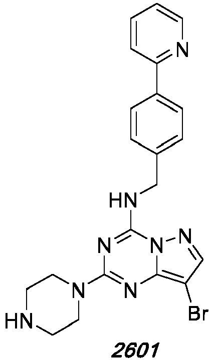

Prepared by TFA deprotection of previous Boc amino compound (0.092 g, 0.163 mmol) as described above in the synthesis procedure of compound 2388. Compound 2601 was obtained as a white solid (0.073 g, 96% yield). MS m/z (ESI+): Calc for C₂₁H₂₁BrN₈ [M+H]+ = 465.12 ; Exp = 465.1. HPLC Method 2, retention time = 3.99 min. ¹H NMR (300 MHz, DMSO) δ 9.18 - 9.12 (m, 1H), 8.64 (ddd, *J* = 4.8, 1.8, 1.0 Hz, 1H), 8.04 (d, *J* = 8.3 Hz, 2H), 7.97 (s, 1H), 7.93 (dt, *J* = 8.0, 1.2 Hz, 1H), 7.86 (ddd, *J* = 8.0, 7.2, 1.8 Hz, 1H), 7.49 (d, *J* = 8.4 Hz, 2H), 7.33 (ddd, *J* = 7.2, 4.8, 1.3 Hz, 1H), 4.67 (d, *J* = 4.9 Hz, 2H), 3.69 - 3.63 (m, 4H), 2.70 - 2.64 (m, 4H).

### 1.18 Synthesis of compound 2655

### Synthesis of Boc protected tert-butyl N-[(3S)-1-[8-bromo-4-({[4-(pyridin-2-yl)phenyl]methyl}amino)pyrazolo[1,5-a][1,3,5]triazin-2-yl]pyrrolidin-3-yl]carbamate

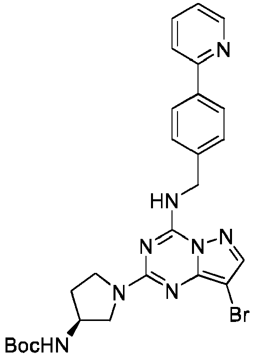

Prepared from tert-butyl N-[(3S)-pyrrolidin-3-yl]carbamate [CAS n° 122536-76-9] and the described above sulfoxide 8-bromo-2-(methylsulfinyl)-N-(4-(pyridin-2-yl)benzyl)pyrazolo[1,5-a][1,3,5]triazin-4-amine (0.069 g, 0.156 mmol) using the same protocol as described for intermediate IV. Boc protected compound was obtained as a white solid (foam) (0.088 g, quant. yield). MS m/z (ESI+): Calc for C₂₆H₂₉BrN₈O₂ [M+H]+ = 565.17 ; Exp = 565.3. HPLC Method 2, retention time = 6.00 min.

### Synthesis of compound 2655

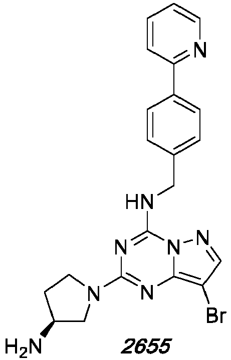

The Boc protected compound (0.086 g, 0.152 mmol) was treated with TFA as described above in the synthesis procedure of compound 2388. Compound 2655 was obtained as a white solid (0.070 g, 99% yield). MS m/z (ESI+): Calc for C₂₁H₂₁BrN₈ [M+H]+ = 465.11; Exp = 465.2. HPLC Method 2, retention time = 3.73 min. ¹H NMR (300 MHz, DMSO) δ 8.64 (ddd, *J* = 4.8, 1.8, 1.0 Hz, 1H), 8.04 (d, *J* = 8.2 Hz, 2H), 7.93 (m, 2H), 7.86 (td, *J* = 7.6, 1.8 Hz, 1H), 7.51 (d, *J* = 8.1 Hz, 2H), 7.33 (ddd, *J* = 7.3, 4.8, 1.3 Hz, 1H), 4.67 (s, 2H), 3.74 - 3.37 (m, 4H), 3.26 - 3.11 (m, 1H), 2.08 - 1.55 (m, 2H).

### 1.19 Synthesis of compounds 2656 (R enantiomer) and 2657 (S enantiomer)

### Synthesis of Boc protected tert-butyl (3R)-3-{[8-bromo-4-({[4-(pyridin-2-yl)phenyl]methyl}amino)pyrazolo[1,5 -a] [1,3,5]triazin-yl]amino}pyrrolidine-1 -carboxylate

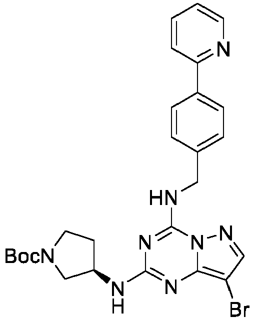

Prepared from tert-butyl (3R)-3-aminopyrrolidine-1-carboxylate [CAS n°147081-49-0] and the described above sulfoxide 8-bromo-2-(methylsulfinyl)-N-(4-(pyridin-2-yl)benzyl)pyrazolo[1,5-*a*][1,3,5]triazin-4-amine (0.065 g, 0.147 mmol) using the same protocol as described for intermediate IV. Boc protected compound was obtained as a white solid (foam) (0.083 g, quant. yield). MS m/z (ESI+): Calc for C₂₆H₂₉BrN₈O₂ [M+H]+ = 565.17 ; Exp = 565.2. HPLC Method 2, retention time = 6.00 min.

### Synthesis of compound 2656

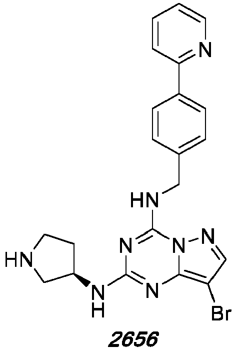

The Boc protected compound (0.080 g, 0.141 mmol) was treated with TFA as described above in the synthesis procedure of compound 2388. Compound 2656 (R enantiomer) was obtained as a white solid (0.051 g, 77% yield). MS m/z (ESI+): Calc for C₂₁H₂₁BrN₈ [M+H]+ = 465.11; Exp = 465.2. HPLC Method 2, retention time = 3.67 min. ¹H NMR (300 MHz, DMSO) δ 8.64 (ddd, *J* = 4.8, 1.8, 1.0 Hz, 1H), 8.04 (d, *J* = 8.3 Hz, 2H), 7.97 - 7.90 (m, 2H), 7.86 (ddd, *J* = 8.0, 7.2, 1.8 Hz, 1H), 7.52 - 7.43 (m, 2H), 7.33 (ddd, *J* = 7.2, 4.8, 1.3 Hz, 1H), 4.69 (s, 2H), 4.37 - 4.19 (m, 1H), 3.00 - 2.57 (m, 4H), 2.02 - 1.82 (m, 1H), 1.68 - 1.47 (m, 1H).

### Synthesis of compound 2657

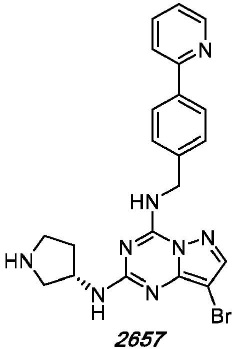

Compound 2657 (S enantiomer) was prepared as described above for compound 2656 with 79% overall yield from tert-butyl (3S)-3-aminopyrrolidine-1-carboxylate [CAS n°147081-44-5].

### 1.20 Synthesis of compound 2658

### Synthesis of Boc protected tert-butyl N-(3-{[8-bromo-4-({[4-(pyridin-2-yl)phenyl]methyl}amino)pyrazolo[1,5-a][1,3,5]triazin-2-yl]amino}propyl)carbamate

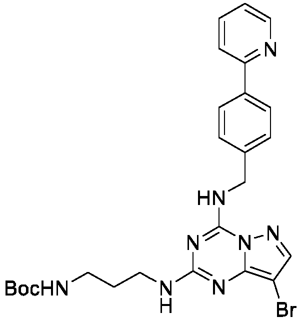

Prepared from tert-butyl N-(3-aminopropyl)carbamate [CAS n° 75178-96-0] and the described above sulfoxide 8-bromo-2-(methylsulfinyl)-N-(4-(pyridin-2-yl)benzyl)pyrazolo[1,5-a][1,3,5]triazin-4-amine (0.061 g, 0.138 mmol) using the same protocol as described for intermediate IV. Boc protected compound was obtained as a white solid (foam) (0.067 g, 88% yield). MS m/z (ESI+): Calc for CzsHzsBrNaOz [M+H]+ = 553.17 ; Exp = 553.2. HPLC Method 2, retention time = 6.5.37 min.

### Synthesis of compound 2658

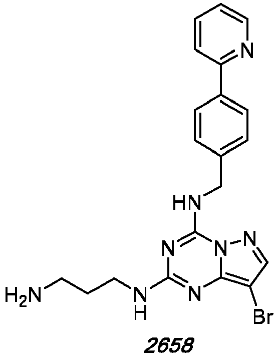

The Boc protected compound (0.065, 0.149 mmol) was treated with TFA as described above in the synthesis procedure of compound 2388. Compound 2658 was obtained as a white solid (0.053 g, 79% yield). MS m/z (ESI+): Calc for C₂₀H₂₁BrN₈ [M+H]+ = 453.11; Exp = 453.1. HPLC Method 2, retention time = 3.42 min. ¹H NMR (300 MHz, DMSO) δ 8.64 (ddd, *J* = 4.8, 1.8, 0.9 Hz, 1H), 8.04 (d, *J* = 8.3 Hz, 2H), 7.97 - 7.89 (m, 2H), 7.86 (td, *J* = 7.6, 1.8 Hz, 1H), 7.52 - 7.42 (m, 2H), 7.33 (ddd, *J* = 7.3, 4.8, 1.3 Hz, 1H), 4.68 (s, 2H), 3.45 - 3.16 (m, 4H), 1.58 (m, 2H).

### 1.21 Synthesis of compound 2668

### Synthesis of Boc protected tert-butyl N-[(1S,2S)-2-{[8-bromo-4-({[4-(pyridin-2-yl)phenyl]methyl}amino)pyrazolo[1,5-a][1,3,5]triazin-2-yl]amino}cyclopentyl]carbamate

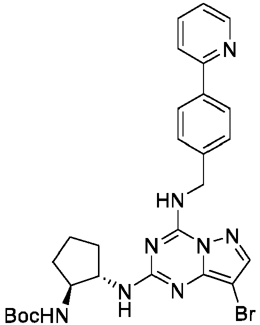

Prepared from tert-butyl N-[(1S,2S)-2-aminocyclopentyl]carbamate [CAS n° 586961-34-4] and the described above sulfoxide 8-bromo-2-(methylsulfinyl)-N-(4-(pyridin-2-yl)benzyl)pyrazolo[1,5-a][1,3,5]triazin-4-amine (0.070 g, 0.158 mmol) using the same protocol as described for intermediate IV. Boc protected compound was obtained as a white solid (foam) (0.079 g, 86% yield). MS m/z (ESI+): Calc for C₂₇H₃₁BrN₈O₂ [M+H]+ = 579.18 ; Exp = 579.3. HPLC Method 2, retention time = 6.02 min.

### Synthesis of compound 2668

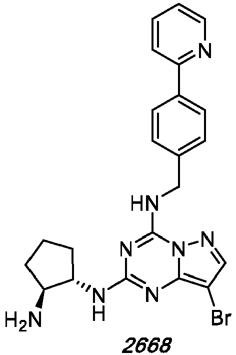

The Boc protected compound (0.076 g, 0.131 mmol) was treated with TFA as described above in the synthesis procedure of compound 2388. Compound 2668 was obtained as a white solid (0.048 g, 76% yield). MS m/z (ESI+): Calc for C₂₂H₂₃BrN₈ [M+H]+ = 479.13; Exp = 479.1. HPLC Method 2, retention time = 3.75 min ¹H NMR (300 MHz, DMSO) δ 8.64 (ddd, *J* = 4.8, 1.8, 0.9 Hz, 1H), 8.04 (d, *J* = 8.3 Hz, 2H), 7.96 - 7.89 (m, 2H), 7.86 (ddd, *J* = 8.0, 7.3, 1.8 Hz, 1H), 7.55 - 7.38 (m, 2H), 7.33 (ddd, *J* = 7.2, 4.8, 1.3 Hz, 1H), 4.69 (s, 2H), 4.39 (s, 1H), 3.42), 2.15 - 1.12 (m, 6H).

### 1.22 Synthesis of compound 2669

### Synthesis of Boc protected tert-butyl N-[(1S,3S)-3-{[8-bromo-4-({[4-(pyridin-2-yl)phenyl]methyl}amino)pyrazolo[1,5-a][1,3,5]triazin-2-yl]amino}cyclopentyl]carbamate

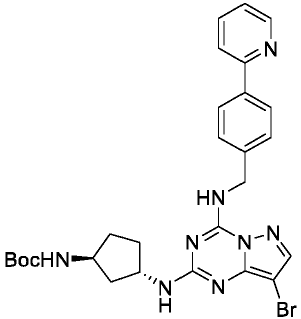

Prepared from tert-butyl N-[(1S,3S)-3-aminocyclopentyl]carbamate [CAS n° 645400-44-8] and the described above sulfoxide 8-bromo-2-(methylsulfinyl)-N-(4-(pyridin-2-yl)benzyl)pyrazolo[1,5-a][1,3,5]triazin-4-amine (0.071 g, 0.160 mmol) using the same protocol as described for intermediate IV. Boc protected compound was obtained as a white solid (foam) (0.075 g, 81% yield). MS m/z (ESI+): Calc for C₂₇H₃₁BrN₈O₂ [M+H]+ = 579.18 ; Exp = 579.3. HPLC Method 2, retention time = 5.70 min.

### Synthesis of compound 2669

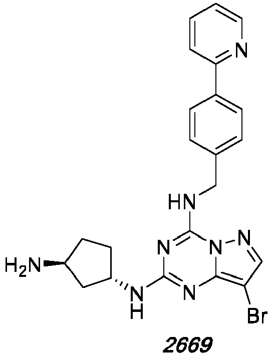

The Boc protected compound (0.0715 g, 0.123 mmol) was treated with TFA as described above in the synthesis procedure of compound 2388. Compound 2669 was obtained as a white solid (0.045 g, 79% yield). MS m/z (ESI+): Calc for C₂₂H₂₃BrN₈ [M+H]+ = 479.13; Exp = 479.2. HPLC Method 2, retention time = 3.61 min. ¹H NMR (300 MHz, DMSO) δ 8.64 (ddd, *J* = 4.8, 1.8, 0.9 Hz, 1H), 8.04 (d, *J* = 8.3 Hz, 2H), 7.96 - 7.89 (m, 2H), 7.86 (ddd, *J* = 8.0, 7.3, 1.8 Hz, 1H), 7.55 - 7.38 (m, 2H), 7.33 (ddd, *J* = 7.2, 4.8, 1.3 Hz, 1H), 4.69 (s, 2H), 4.39 (s, 1H), 3.42 - 3.31 (m, 1H), 2.15 - 1.12 (m, 6H).

### 1.23 Synthesis of compound 2670

### Synthesis of Boc protected tert-butyl N-[(1S,3R)-3-{[8-bromo-4-({[4-(pyridin-2-yl)phenyl]methyl}amino)pyrazolo[1,5-a][1,3,5]triazin-2-yl]amino}cyclohexyl]carbamate

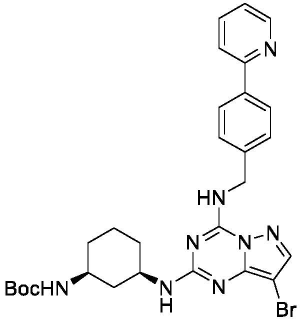

Prepared from tert-butyl N-[(1S,3R)-3-aminocyclohexyl]carbamate [CAS n° 1298101-47-9] and the described above sulfoxide 8-bromo-2-(methylsulfinyl)-N-(4-(pyridin-2-yl)benzyl)pyrazolo[1,5-a][1,3,5]triazin-4-amine (0.068 g, 0.153 mmol) using the same protocol as described for intermediate IV. Boc protected compound was obtained as a white solid (foam) (0.080 g, 88% yield). MS m/z (ESI+): Calc for C₂₈H₃₃BrN₈O₂ [M+H]+ = 593.20 ; Exp = 593.3. HPLC Method 2, retention time = 5.92 min.

### Synthesis of compound 2670

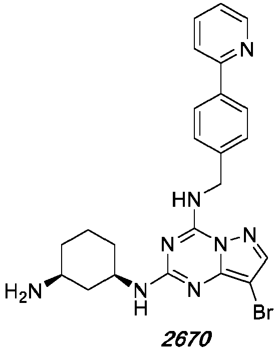

The Boc protected compound (0.0765 g, 0.129 mmol) was treated with TFA as described above in the synthesis procedure of compound 2388. Compound 2670 was obtained as a white solid (0.038 g, 60% yield). MS m/z (ESI+): Calc for C₂₃H₂₅BrN₈ [M+H]+ = 493.15; Exp = 493.2. HPLC Method 2, retention time = 3.81 min. ¹H NMR (300 MHz, DMSO) δ 8.64 (dt, *J* = 4.5, 1.5 Hz, 1H), 8.04 (d, *J* = 8.3 Hz, 2H), 7.96 - 7.89 (m, 2H), 7.88 - 7.80 (m, 1H), 7.53 - 7.43 (m, 2H), 7.33 (ddd, *J* = 7.3, 4.8, 1.3 Hz, 1H), 4.76 - 4.57 (m, 2H), 3.87 - 3.60 (m, 1H), 2.69 - 2.55 (m, 1H), 2.02 - 1.56 (m, 4H), 1.35 - 0.78 (m, 4H).

### 1.24 Synthesis of compound 2671

### Synthesis of Boc protected intermediate compound tert-butyl N-[(1S,2S)-2-{[8-bromo-4-({[4-(pyridin-2-yl)phenyl]methyl}amino)pyrazolo[1,5-a][1,3,5]triazin-2-yl]amino}cyclohexyl]carbamate

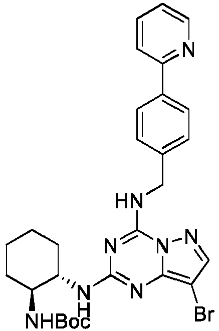

Prepared from tert-butyl N-[(1S,2S)-2-aminocyclohexyl]carbamate [CAS n° 180683-64-1] and the described above sulfoxide 8-bromo-2-(methylsulfinyl)-N-(4-(pyridin-2-yl)benzyl)pyrazolo[1,5-a][1,3,5]triazin-4-amine (0.071 g, 0.160 mmol) using the same protocol as described for intermediate IV. Boc protected compound was obtained as a white solid (foam) (0.091 g, 96% yield). MS m/z (ESI+): Calc for C₂₈H₃₃BrN₈O₂ [M+H]+ = 593.20 ; Exp = 593.3. HPLC Method 2, retention time = 6.22 min.

### Synthesis of compound 2671

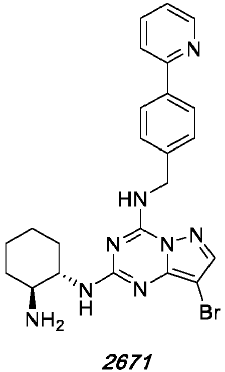

The Boc protected compound (0.089 g, 0.150 mmol) was treated with TFA as described above in the synthesis procedure of compound 2388. Compound 2671 was obtained as a white solid (0.064 g, 86% yield). MS m/z (ESI+): Calc for C₂₃H₂₅BrN₈ [M+H]+ = 493.15; Exp = 493.2. HPLC Method 2, retention time = 4.17 min ¹H NMR (300 MHz, DMSO) δ 8.64 (ddd, *J* = 4.8, 1.8, 0.9 Hz, 1H), 8.04 (d, *J* = 8.0 Hz, 2H), 7.98 - 7.90 (m, 2H), 7.89 - 7.82 (m, 1H), 7.52 - 7.46 (m, 2H), 7.33 (ddd, *J* = 7.2, 4.8, 1.3 Hz, 1H), 4.82 - 4.51 (m, 2H), 3.68 - 3.34 (m, 2H), 2.69 - 2.31 (m, 1H), 2.02 - 1.50 (m, 2H), 1.34 - 0.93 (m, 5H).

### 1.25 Synthesis of compound 2672

### Synthesis of Boc protected tert-butyl N-[(1S,2R)-2-{[8-bromo-4-({[4-(pyridin-2-yl)phenyl]methyl}amino)pyrazolo[1,5-a][1,3,5]triazin-2-yl]amino}cyclohexyl]carbamate

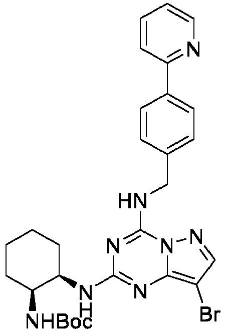

Prepared from tert-butyl N-[(1S,2R)-2-aminocyclohexyl]carbamate [CAS n° 365996-30-1] and the described above sulfoxide 8-bromo-2-(methylsulfinyl)-N-(4-(pyridin-2-yl)benzyl)pyrazolo[1,5-a][1,3,5]triazin-4-amine (0.070 g, 0.158 mmol) using the same protocol as described for intermediate IV. Boc protected compound was obtained as a white solid (foam) (0.091 g, 97% yield). MS m/z (ESI+): Calc for C₂₈H₃₃BrN₈O₂ [M+H]+ = 593.20 ; Exp = 593.3. HPLC Method 2, retention time = 6.08 min.

### Synthesis of compound 2672

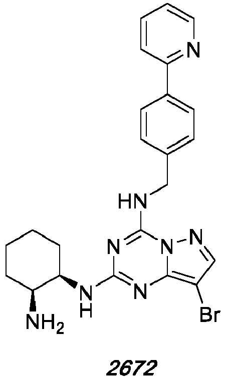

The Boc protected compound (0.088 g, 0.148 mmol) was treated with TFA as described above in the synthesis procedure of compound 2388. Compound 2672 was obtained as a white solid (0.064 g, 73% yield). MS m/z (ESI+): Calc for C₂₈H₃₃BrN₈ [M+H]+ = 493.15; Exp = 493.2. HPLC Method 2, retention time = 4.21 min. ¹H NMR (300 MHz, DMSO) δ 8.65 (ddd, *J* = 4.8, 1.8, 1.0 Hz, 1H), 8.05 (d, *J* = 8.3 Hz, 2H), 7.99 (s, 1H), 7.93 (dt, *J* = 8.1, 1.2 Hz, 1H), 7.86 (ddd, *J* = 8.0, 7.2, 1.8 Hz, 1H), 7.48 (d, *J* = 8.0 Hz, 2H), 7.34 (ddd, *J* = 7.2, 4.8, 1.3 Hz, 1H), 4.72 (m, 2H), 4.17 - 3.93 (m, 1H), 1.83 - 1.22 (m, 9H).

### 1.26 Synthesis of compound 2636

### Synthesis of Boc protected tert-butyl N-{[trans4-{[8-bromo-4-({[4-(pyridin-2-yl)phenyl]methyl}amino)pyrazolo[1,5-a][1,3,5]triazin-2-yl]amino}cyclohexyl]methyl}carbamate

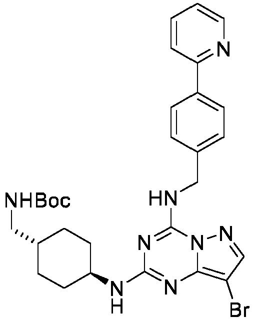

Prepared from tert-butyl *N*-[(trans-4-aminocyclohexyl)methyl]carbamate [CAS n° 192323-07-2] and the described above sulfoxide 8-bromo-2-(methylsulfinyl)-N-(4-(pyridin-2-yl)benzyl)pyrazolo[1,5-*a*][1,3,5]triazin-4-amine (0.080 g, 0.18 mmol) using the same protocol as described for intermediate IV. Boc protected compound was obtained as a white solid (foam) (0.083 g, 76% yield). MS m/z (ESI+): Calc for C₂₉H₃₅BrN₈O₂ [M+H]+ = 607.21 ; Exp = 607.2. HPLC Method 2, retention time = 5.96 min.

### Synthesis of compound 2636

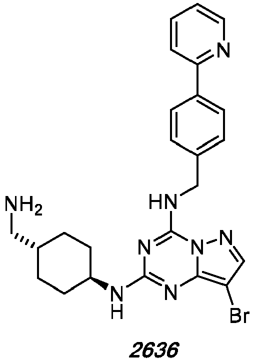

The Boc protected compound (0.081 g, 0.133 mmol) was treated with TFA as described above in the synthesis procedure of compound 2388. Compound 2636 was obtained as a white solid (0.041 g, 61% yield). MS m/z (ESI+): Calc for C₂₄H₂₇BrN₈ [M+H]+ = 507.16; Exp = 507.2. HPLC Method 2, retention time = 3.81 min. ¹H NMR (300 MHz, DMSO) δ 8.62 (ddd, *J* = 4.8, 1.8, 1.0 Hz, 1H), 8.01 (d, *J* = 7.9 Hz, 2H), 7.94 - 7.89 (m, 2H), 7.85 (ddd, *J* = 8.0, 7.1, 1.8 Hz, 1H), 7.52 - 7.41 (m, 2H), 7.33 (ddd, *J* = 7.2, 4.8, 1.4 Hz, 1H), 4.74 - 4.56 (m, 2H), 3.77 - 3.56 (m, 1H), 2.36 (d, *J* = 6.3 Hz, 2H), 1.97 - 1.64 (m, 4H), 1.32 - 0.80 (m, 5H).

### 1.27 Synthesis of compound 2635

### Synthesis of Boc protected tert-butyl N-[trans-4-({[8-bromo-4-({[4-(pyridin-2-yl)phenyl]methyl}amino)pyrazolo[1,5-a][1,3,5]triazin-2-yl]amino}methyl)cyclohexyl]carbamate

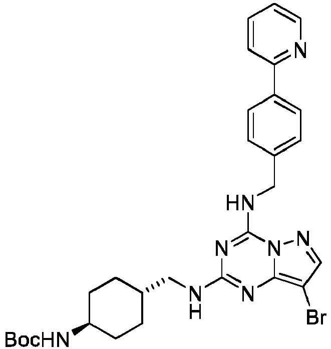

Prepared from tert-butyl N-[trans-4-(aminomethyl)cyclohexyl]carbamate [CAS n° 177583-27-6] and the described above sulfoxide 8-bromo-2-(methylsulfinyl)-N-(4-(pyridin-2-yl)benzyl)pyrazolo[1,5-*a*][1,3,5]triazin-4-amine (0.081 g, 0.183 mmol) using the same protocol as described for intermediate IV. Boc protected compound was obtained as a white solid (foam) (0.085 g, 77% yield). MS m/z (ESI+): Calc for C₂₉H₃₅BrN₈O₂ [M+H]+ = 607.21 ; Exp = 607.2. HPLC Method 2, retention time = 5.97 min.

### Synthesis of compound 2635

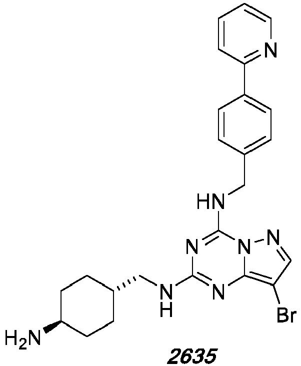

The Boc protected compound (0.0825 g, 0.136 mmol) was treated with TFA as described above in the synthesis procedure of compound 2388. Compound D27 was obtained as a white solid (0.020 g, 29% yield). MS m/z (ESI+): Calc for C₂₈H₃₃BrN₈ [M+H]+ = 507.16; Exp = 507.2. HPLC Method 2, retention time = 3.75 min. ¹H NMR (300 MHz, DMSO) δ 8.66 (ddd, *J* = 4.8, 1.8, 1.0 Hz, 1H), 8.05 (d, *J* = 8.4 Hz, 2H), 7.97 - 7.91 (m, 2H), 7.87 (ddd, *J* = 8.0, 7.2, 1.8 Hz, 1H), 7.52 - 7.42 (m, 2H), 7.37 - 7.31 (m, 1H), 4.69 (d, *J* = 7.2 Hz, 2H), 3.16 - 3.01 (m, 2H), 1.83 - 0.67 (m, 10H).

### 1.28 Synthesis of compound 2590

The iododerivative 2590 was prepared as described above in the synthesis of compounds 2384 and 2388)

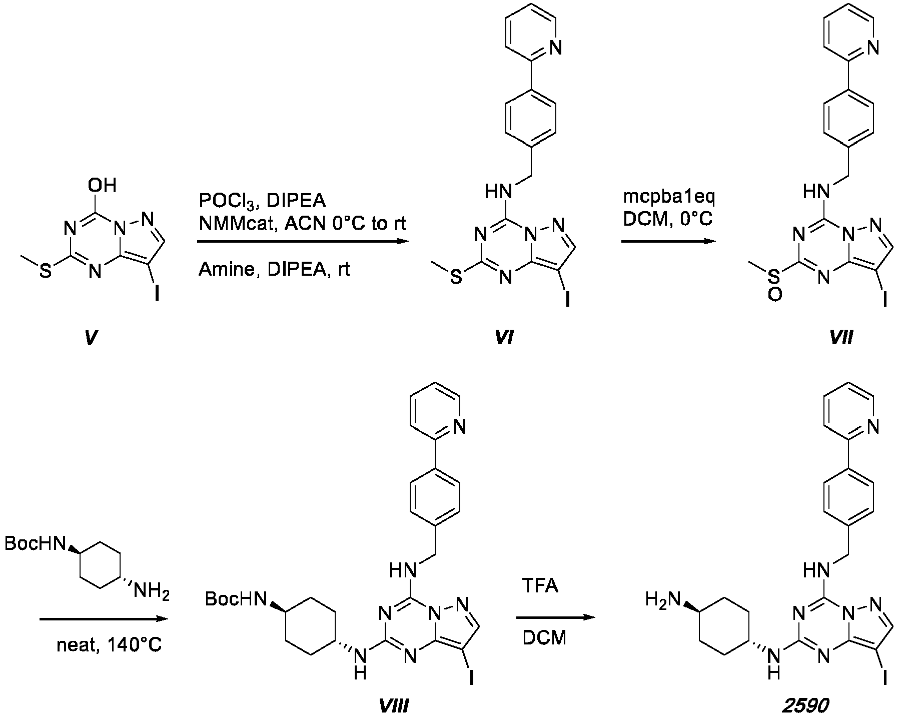

### Synthesis of compound V

Compound V (8-Iodo-2-(methylthio)pyrazolo[1,5-*a*]-1,3,5-triazin-4(3*H*)-one [CAS n°147916-85-6]) was prepared by standard iodination protocol of 2-(Methylthio)pyrazolo[1,5-*a*]-1,3,5-triazin-4(3*H*)-one [CAS n°54346-18-8], as described above for bromoderivative compound I (N-iodosuccinimide was used instead of N-bromosuccinimide).

### Synthesis compound VI

Prepared from 8-Iodo-2-(methylthio)pyrazolo[1,5-*a*]-1,3,5-triazin-4(3*H*)-one [CAS n°147916-85-6] (compound V) (0.200 g, 0.649 mmol) as described above for compound II. Compound *VI* (0.196 g, 64% yield) was obtained as a white powder. MS m/z (ESI+): Calc for C₁₈H₁₅IN₆S [M+H]+ = 475.02 ; Exp = 475.0. HPLC Method 2, retention time = 6.08 min. ¹H NMR (300 MHz, DMSO) δ 9.61 (t, *J* = 6.3 Hz, 1H), 8.65 (ddd, *J* = 4.8, 1.8, 1.0 Hz, 1H), 8.18 (s, 1H), 8.05 (d, *J* = 8.4 Hz, 2H), 7.93 (dt, *J* = 8.0, 1.2 Hz, 1H), 7.86 (td, *J* = 7.7, 1.8 Hz, 1H), 7.48 (d, *J* = 8.4 Hz, 2H), 7.34 (ddd, *J* = 7.3, 4.8, 1.3 Hz, 1H), 4.74 (d, *J* = 6.2 Hz, 2H), 2.5 (s, 3H, under the solvent peak).

### Synthesis of compound VII

Prepared as described above for compound III by mcpba oxidation of previous 8-lodo-2-(methylthio)-N-(4-(pyridin-2-yl)benzyl)pyrazolo[1,5-*a*][1,3,5]triazin-4-amine (compound VI) (0.193 g, 0.407 mmol). Compound *VII* (0.199 g, quant. yield) was obtained as a white powder). MS m/z (ESI+): Calc for C₁₈H₁₅IN₆OS [M+H]+ = 491.01 ; Exp = 491.0. HPLC Method 2, retention time = 4.00 min. ¹H NMR (300 MHz, DMSO) δ 10.04 (s, 1H), 8.64 (ddd, *J* = 4.8, 1.8, 1.0 Hz, 1H), 8.37 (s, 1H), 8.04 (d, *J* = 8.4 Hz, 2H), 7.93 (dt, *J* = 8.1, 1.2 Hz, 1H), 7.86 (ddd, *J* = 8.0, 7.3, 1.8 Hz, 1H), 7.51 (d, *J* = 8.4 Hz, 2H), 7.33 (ddd, *J* = 7.3, 4.8, 1.3 Hz, 1H), 4.79 (s, 2H), 2.83 (s, 3H).

### Compound VIII

Prepared from N-Boc-trans-1,4-Cyclohexanediamine [CAS n° 195314-59-1] and the described above sulfoxide 8-Iodo-2-(methylsulfinyl)-N-(4-(pyridin-2-yl)benzyl)pyrazolo[1,5-*a*][1,3,5]triazin-4-amine (compound VII) (0.065 g, 0.133 mmol) using the same protocol as previously described for intermediate IV. Compound VIII was obtained as a white solid (0.054 g, 64% yield). MS m/z (ESI+): Calc for C₂₈H₃₃IN₈O₂ [M+H]+ = 641.18 ; Exp = 641.2. HPLC Method 2, retention time = 5.93 min. ¹H NMR (300 MHz, DMSO) δ 9.16, 8.87 (2 broad s, 1H, exch NH), 8.64 (ddd, *J* = 4.8, 1.8, 1.0 Hz, 1H), 8.04 (d, *J* = 7.9 Hz, 2H), 7.95 - 7.81 (m, 3H), 7.52 - 7.41 (m, 2H), 7.33 (ddd, *J* = 7.2, 4.8, 1.3 Hz, 1H), 7.25 (broad d, *J* = 7.8 Hz, 0.5 H, exch NH), 7.08 (broad d, *J* = 8.1 Hz, 0.5 H, exch NH), 6.73 (s, 1H), 4.74 - 4.55 (m, 2H), 3.76 - 3.52 (m, 1H), 3.25 - 3.06 (m, 1H), 1.76 (s, 4H), 1.38 (s, 9H), 1.35 - 1.10 (m, 4H).

### Synthesis of compound 2590

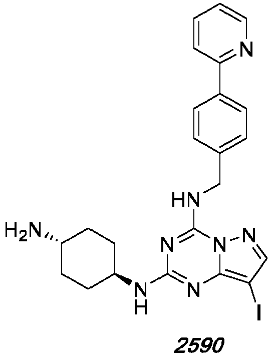

Prepared by TFA deprotection of previous Boc amino compound VIII (0.051 g, 0.080 mmol) as described above for compound 2388. Compound 2590 was obtained as a grey solid (0.028 g, 65% yield). MS m/z (ESI+): Calc for C₂₃H₂₅IN₈ [M+H]+ = 541.13 ; Exp = 541.1. HPLC Method 2, retention time = 3.77 min¹H NMR (300 MHz, DMSO) δ 8.64 (ddd, *J* = 4.8, 1.8, 1.0 Hz, 1H), 8.03 (d, *J* = 8.2 Hz, 2H), 7.95 - 7.89 (m, 1H), 7.89 - 7.82 (m, 2H), 7.54 - 7.42 (m, 2H), 7.33 (ddd, *J* = 7.2, 4.8, 1.4 Hz, 1H), 7.23 (d, *J* = 7.9 Hz, 0.5H exch NH), 7.04 (d, *J* = 8.2 Hz, 0.5H, exch NH), 4.74 - 4.58 (m, 2H), 3.78 - 3.55 (m, 1H), 2.50 - 2.40 (m, 1H), 1.92 - 1.03 (m, 8H).

### 1.29 Synthesis of compound 2629

The chloroderivative 2629 was prepared as described above in the synthesis of compounds 2384 and 2388)

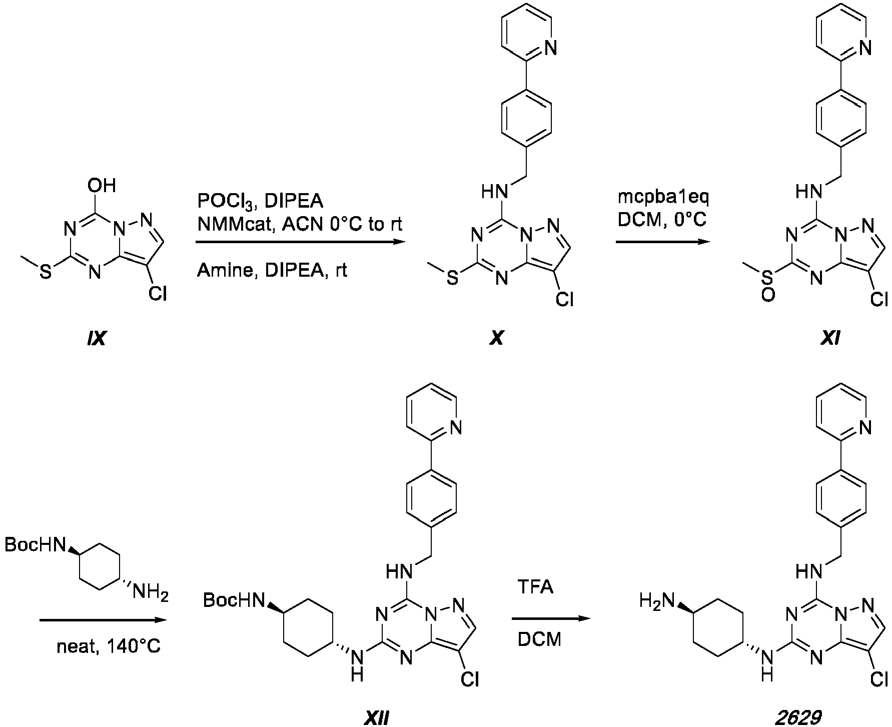

### Synthesis compound IX

Compound *IX (*8-Chloro-2-(methylthio)pyrazolo[1,5-*a*]-1,3,5-triazin-4(3*H*)-one [CAS n°1843260-73-0]) was prepared by standard chlorination protocol of 2-(Methylthio)pyrazolo[1,5-*a*]-1,3,5-triazin-4(3*H*)-one [CAS n°54346-18-8] using N-chlorosuccinimide instead of N-bromosuccinimide.

### Synthesis of compound X

Prepared from 8-chloro-2-(methylthio)pyrazolo[1,5-a]-1,3,5-triazin-4(3*H*)-one [CAS n°1843260-73-0] (compound IX) (0.250 g, 1.154 mmol) as described above for compound II. Compound X (0.407 g, 92% yield) was obtained as a beige powder. MS m/z (ESI+): Calc for C₁₈H₁₅ClN₆S [M+H]+ = 383.08 ; Exp = 383.1. HPLC Method 2, retention time = 5.68 min. ¹H NMR (300 MHz, DMSO) δ 9.68 (t, *J* = 6.3 Hz, 1H), 8.65 (ddd, *J* = 4.8, 1.8, 0.9 Hz, 1H), 8.26 (s, 1H), 8.05 (d, *J* = 8.3 Hz, 2H), 7.94 (dt, *J* = 8.0, 1.2 Hz, 1H), 7.87 (ddd, *J* = 8.0, 7.3, 1.8 Hz, 1H), 7.48 (d, *J* = 8.4 Hz, 2H), 7.34 (ddd, *J* = 7.3, 4.8, 1.3 Hz, 1H), 4.74 (d, *J* = 6.3 Hz, 2H), 2.49 (s, 3H).

### Synthesis of compound XI

Prepared as described above for compound II by mcpba oxidation of previous 8-chloro-2-(methylthio)-N-(4-(pyridin-2-yl)benzyl)pyrazolo[1,5-*a*][1,3,5]triazin-4-amine (compound X) (0.353 g, 0.922 mmol). Compound XI (quant. yield) was obtained as a white powder). MS m/z (ESI+): Calc for C₁₈H₁₅CIN₆OS [M+H]+ = 399.08 ; Exp = 399.1. HPLC Method 2, retention time = 3.79 min. ¹H NMR (300 MHz, DMSO) δ 10.11 (broad s, 1H), 8.64 (ddd, *J* = 4.8, 1.8, 0.9 Hz, 1H), 8.35 (s, 1H), 8.04 (d, *J* = 8.3 Hz, 2H), 7.96 - 7.91 (m, 1H), 7.86 (ddd, *J* = 8.0, 7.3, 1.8 Hz, 1H), 7.51 (d, *J* = 8.4 Hz, 2H), 7.33 (ddd, *J* = 7.3, 4.8, 1.3 Hz, 1H), 4.77 (s, 2H), 2.82 (s, 3H).

### Synthesis of compound XII

Prepared from N-Boc-trans-1,4-Cyclohexanediamine [CAS n° 195314-59-1] and the described above sulfoxide 8-chloro-2-(methylsulfinyl)-N-(4-(pyridin-2-yl)benzyl)pyrazolo[1,5-*a*][1,3,5]triazin-4-amine (compound XI) (0.100 g, 0.251 mmol) using the same protocol as described above for intermediate IV. Compound XII was obtained as a white solid (0.107 g, 78% yield). MS m/z (ESI+): Calc for C₂₈H₃₃CIN₈O₂ [M+H]+ = 549.25 ; Exp = 549.2. HPLC Method 2, retention time = 5.76 min. ¹H NMR (300 MHz, DMSO) δ 8.66 (ddd, *J* = 4.8, 1.8, 1.0 Hz, 1H), 8.09 - 8.02 (m, 2H), 7.98 - 7.91 (m, 2H), 7.87 (td, *J* = 7.6, 1.8 Hz, 1H), 7.54 - 7.42 (m, 2H), 7.34 (ddd, *J* = 7.2, 4.8, 1.4 Hz, 1H), 6.81 - 6.70 (m, 1H), 4.77 - 4.58 (m, 2H), 3.77 - 3.52 (m, 1H), 3.25 - 3.07 (m, 1H), 1.96 - 1.64 (m, 4H), 1.39 (s, 9H), 1.35 - 1.07 (m, 4H).

### Synthesis of compound 2629

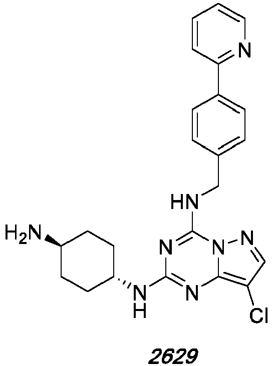

Prepared by TFA deprotection of previous Boc amino compound XII (0.103 g, 0.188 mmol) as described for compound IV. Compound 2629 was obtained as a grey solid (0.082 g, 97% yield). MS m/z (ESI+): Calc for C₂₀H₂₁BrN₈ [M+H]+ = 449.20 ; Exp = 449.2. HPLC Method 2, retention time = 3.59 min. ¹H NMR (300 MHz, DMSO) δ 8.65 (ddd, *J* = 4.8, 1.8, 1.0 Hz, 1H), 8.04 (d, *J* = 8.2 Hz, 2H), 7.96 - 7.90 (m, 2H), 7.86 (ddd, *J* = 8.0, 7.2, 1.8 Hz, 1H), 7.54 - 7.43 (m, 2H), 7.33 (ddd, *J* = 7.2, 4.8, 1.3 Hz, 1H), 4.71 - 4.61 (m, 2H), 3.78 - 3.54 (m, 1H), 2.50 - 2.38 (m, 1H), 1.93 - 0.99 (m, 8H).

### 1.30 Synthesis of compound 2420

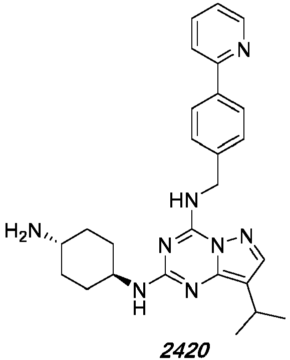

Compound 2420 [CAS n° 2254007-13-9], N2-((1r,4r)-4-aminocyclohexyl)-8-isopropyl-N4-(4-(pyridin-2-yl)benzyl)pyrazolo[1,5-a][1,3,5]triazine-2,4-diamine was obtained as described by G. Compain et al, Eur. J. Med. Chem, 2018, 208, 158, 1-6 (doi.org/10.1016/j.ejmech.2018.08.100).

### 1.31 Synthesis of compound 2428

### Synthesis of compound XIII

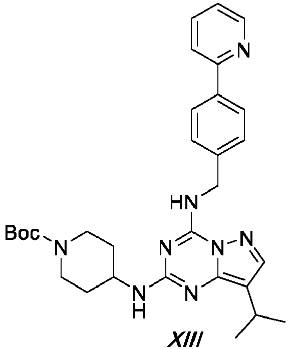

Prepared from tert-Butyl 4-aminopiperidine-1-carboxylate [CAS 87120-72-7] and the known sulfoxide 8-(1-Methylethyl)-2-(methylsulfinyl)-*N*-[[4-(2-pyridinyl)phenyl]methyl]pyrazolo[1,5-*a*]-1,3,5-triazin-4-amine [CAS n°1244955-43-8]; see G. Compain et al, Eur. J. Med. Chem, 2018, 208, 158, 1-6 (0.074 g, 0.136 mmol) using the same protocol as described above for intermediate IV. Compound XIII was obtained as a white solid. HPLC Method 2, retention time = 5.96 min. ¹H NMR (300 MHz, DMSO) δ 8.35 - 8.28 (m, 1H), 7.78 (d, *J* = 7.9 Hz, 2H), 7.73 (s, 1H), 7.64 - 7.54 (m, 1H), 7.49 - 7.42 (m, 2H), 7.41 - 7.36 (m, 2H), 4.67 (s, 2H), 4.00 - 3.71 (m, 3H), 3.04 - 2.69 (m, 3H), 1.96 - 1.55 (m, 2H), 1.50 - 1.11 (m, 17H).

### Synthesis of compound 2428

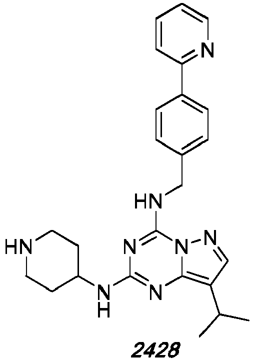

Prepared by TFA deprotection of previous Boc amino compound XIII, white solid. MS m/z (ESI+): Calc for C₂₅H₃₀N₈ [M+H]+ = 443.27 ; Exp = 443.3. HPLC Method 2, retention time = 4.08 min. ¹H NMR (300 MHz, DMSO) δ 8.65 (ddd, *J* = 4.8, 1.8, 1.0 Hz, 1H), 8.04 (d, *J* = 8.0 Hz, 2H), 7.93 (dt, *J* = 8.1, 1.2 Hz, 1H), 7.86 (td, *J* = 7.7, 1.8 Hz, 1H), 7.71 (s, 1H), 7.47 (d, *J* = 7.9 Hz, 2H), 7.34 (ddd, *J* = 7.2, 4.8, 1.4 Hz, 1H), 4.66 (broad s, 2H), 3.78 - 3.60 (m, 1H), 2.99 - 2.81 (m, 3H), 2.50 - 2.41 (m, 2H), 1.92 - 1.59 (m, 2H), 1.41 - 1.10 (m, 8H).

### 1.32 Synthesis of compound 2478

Prepared from known 2-(Methylthio)-4-oxo-3,4-dihydropyrazolo[1,5-a][1,3,5]triazine-8-carbonitrile (see G. Compain et al, Eur. J. Med. Chem, 2018, 208, 158, 1-6 [CAS n°1273577-70-0]).

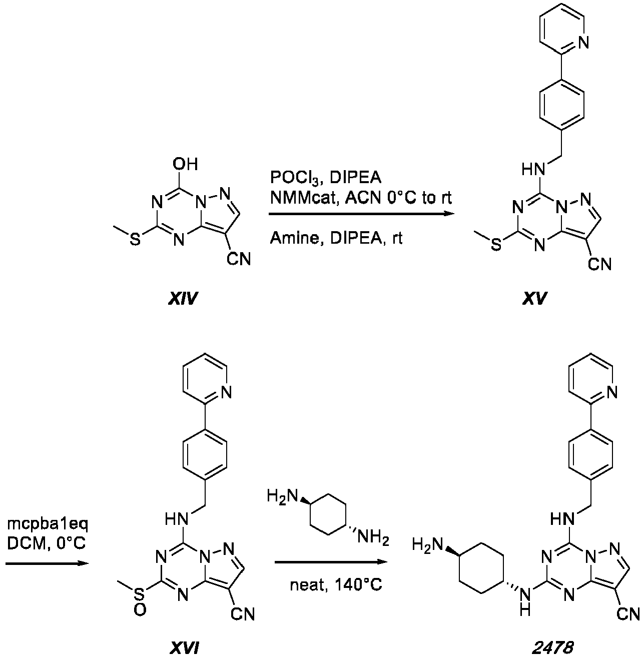

### Synthesis of compound XIV

Prepared from 2-(Methylthio)-4-oxo-3,4-dihydropyrazolo[1,5-*a*][1,3,5]triazine-8-carbonitrile [CAS n°1273577-70-0] (compound XIV) (0.195 g, 0.941 mmol) as described above for compound II. Compound XV (0.308 g, 88% yield) was obtained as a foam. MS m/z (ESI+): Calc for C₁₉H₁₅N₇S [M+H]+ = 374.12 ; Exp = 374.1. HPLC Method 2, retention time = 5.00 min. ¹H NMR (300 MHz, DMSO) δ 9.93 (t, *J* = 6.0 Hz, 1H), 8.65 (d, *J* = 4.0 Hz, 1H), 8.60 (s, 1H), 8.05 (d, *J* = 7.9 Hz, 2H), 7.94 (d, *J* = 7.6 Hz, 1H), 7.87 (t, *J* = 7.7 Hz, 1H), 7.49 (s, 2H), 7.39 - 7.30 (m, 1H), 4.76 (d, *J* = 6.1 Hz, 2H), 2.50 (CH₃S under the solvent signal, , 3H).

### Synthesis of compound XVI

Prepared as described above for compound III by mcpba oxidation of previous methylsulfinyl compound XV (0.300 g, 0.803 mmol). Compound XI (quant. yield) was obtained as a white foam). MS m/z (ESI+): Calc for C₁₉H₁₅N₇OS [M+H]+ = 390.11 ; Exp = 390.1. HPLC Method 2, retention time = 3.39 min.

### Synthesis of compound 2478

Prepared from previous sulfoxide compound XVI (0.101 g, 0.259 mmol) as described above for compound 2384. Compound 2478 was obtained as foam (0.059 g, 52% yield). MS m/z (ESI+): Calc for C₂₄H₂₅N₉ [M+H]+ = 440.23 ; Exp = 440.2. HPLC Method 2, retention time = 3.63 min. ¹H NMR (300 MHz, DMSO) δ 8.65 (broad d, *J* = 4.2 Hz, 1H), 8.29 (d, *J* = 6.4 Hz, 1H), 8.04 (d, *J* = 7.7 Hz, 2H), 7.96 - 7.83 (m, 2H), 7.53 - 7.43 (m, 2H), 7.36 - 7.30 (m, 1H), 4.71 - 4.61 (m, 2H), 3.81 - 3.53 (m, 1H), 2.50 under solvent peak (m, 1H), 1.88 - 1.64 (m, 4H), 1.36 - 0.99 (m, 4H).

### 1.33 Synthesis of compound 2533

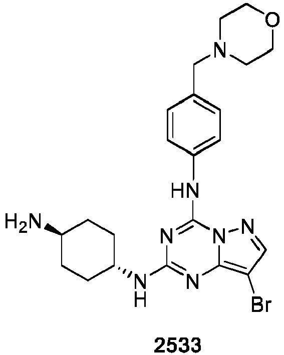

Prepared following the procedures described above for compound 2384, using 4-Morpholin-4-ylmethyl-phenylamine (CAS# 51013-67-3) instead of 4-(Pyridin-2-yl)phenyl)methanamine. Compound 2533 was obtained as a white foam (0.056 g, 93% yield). MS m/z (ESI+): Calc for C₂₂H₂₉BrN₈O [M+H]+ = 501.17 ; Exp = 501.2. HPLC Method 2, retention time = 3.75 min. ¹H NMR (300 MHz, DMSO) δ 8.01 (s, 1H), 7.87 - 7.76 (m, 2H), 7.32 - 7.23 (m, 2H), 3.57 (t, *J* = 4.6 Hz, 5H), 3.45 (m, 2H), 3.37 - 3.29 (m, 1H), 2.40 - 2.30 (m, 4H), 1.95 - 1.71 (m, 4H), 1.40 - 1.01 (m, 4H).

### 1.34 Synthesis of compound 2554

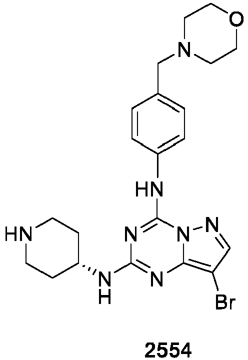

Prepared following the procedures described above for compound 2388, using 4-Morpholin-4-ylmethyl-phenylamine (CAS# 51013-67-3) instead of 4-(Pyridin-2-yl)phenyl)methanamine. Compound 2554 was obtained as a beige solid. MS m/z (ESI+): Calc for C₂₁H₂₇BrN₈O [M+H]+ = 487.16 ; Exp = 487.1. HPLC Method 2, retention time = 3.77 min. ¹H NMR (300 MHz, DMSO) δ 8.02 (s, 1H), 7.87 - 7.78 (m, 2H), 7.32 - 7.24 (m, 2H), 3.91 - 3.60 (m, 1H), 3.59 - 3.54 (m, 4H), 3.48 - 3.42 (m, 2H), 3.01 - 2.91 (m, 2H), 2.39 - 2.30 (m, 4H), 1.86 - 1.76 (m, 2H), 1.45 - 1.11 (m, 4H).

### 1.35 Synthesis of compound 2749

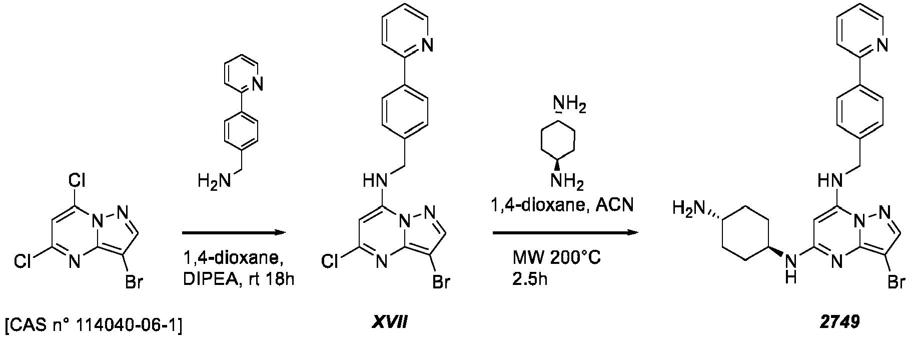

### Synthesis of compound XVII

To the commercially available 3-Bromo-5,7-dichloropyrazolo[1,5-a]pyrimidine [CAS n° 114040-06-1] (0.300 g, 1.124 mmol) and (4-(Pyridin-2yl)phenyl)methanamine (0.217 g, 1.05 eq) in 1,4-Dioxane (2.3 mL) was added DIPEA (0.392 mL, 2.0 eq) at room temperature. The mixture was stirred at 0°C for 18 h, then sonicated and filtrated. The cake was washed with dioxane then acetonitrile. The resulting solid was dried in vacuo (0.390 g, 84% yield, HPLC purity: 96%, 254 nm). MS m/z (ESI+): Calc for C₁₈H₁₃BrClN₅ [M+H]+ = 414.01 ; Exp [M+H]+ = 414.0. HPLC Method 2, retention time = 5.38 min. ¹H NMR (300 MHz, DMSO) δ 8.65 (ddd, *J* = 4.8, 1.8, 0.9 Hz, 1H), 8.33 (s, 1H), 8.06 (d, *J* = 8.3 Hz, 2H), 7.94 (dt, *J* = 8.1, 1.2 Hz, 1H), 7.86 (td, *J* = 7.6, 1.8 Hz, 1H), 7.51 (d, *J* = 8.4 Hz, 2H), 7.34 (ddd, *J* = 7.3, 4.8, 1.3 Hz, 1H), 6.32 (s, 1H), 4.72 (d, *J* = 6.5 Hz, 2H).

### Synthesis of compound 2749

A solution of compound ***XVII*** (0.057 g, 0.137 mmol) and *trans*-1,4-diaminocyclohexane (157 mg, 10.0 eq) in a mixture 3/1 of 1,4-dioxane / acetonitrile (4.5 mL) was heated in a sealed tube at 200°C by microwave irradiation for 2.5 h. The reactional mixture was concentrated in vacuo and the crude The crude residue was taken up in 0.1% TFA (v/v) in water / acetonitrile 1/1 (1 mL) and purified using HPLC preparative method 5 to give compound **2749** as trifluoroacetate salt. Dilution in saturated aqueous NaHCO3 and extraction with DCM gave free base **2749** (0.046 g, 68% yield, HPLC purity: 97%, 254 nm). MS m/z (ESI+): Calc for C₂₄H₂₆BrN₇ [M+H]+ = 492.2 ; Exp [M+H]+ = 492.2. HPLC Method 2, retention time = 3.61 min. ¹H NMR (300 MHz, DMSO) δ 8.64 - 8.56 (m, 1H), 7.99 (d, *J* = 8.3 Hz, 2H), 7.94 - 7.79 (m, 3H), 7.45 (d, *J* = 8.1 Hz, 2H), 7.33 (ddd, *J* = 6.7, 4.8, 1.7 Hz, 1H), 5.14 (s, 1H), 4.50 (s, 2H), 3.71 - 3.50 (m, 1H), 2.68 - 2.54 (m, 1H), 1.95 - 1.68 (m, 4H), 1.28 - 1.02 (m, 4H).

### Example 2: synthesis of drug-linkers incorporating the payload compounds of the disclosure

### 2.1 Synthesis of drug-linker DL-VA-2384

### Synthesis of intermediate B1

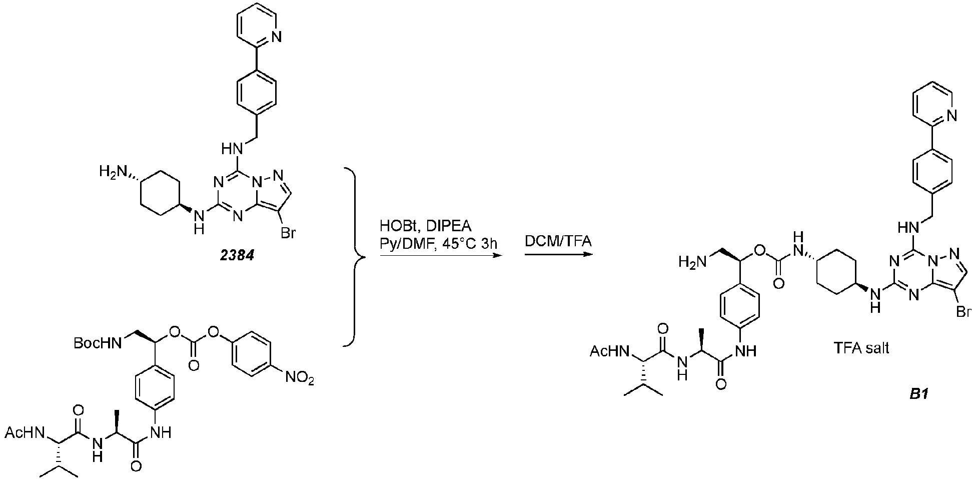

To the compound 2384 (9.0 mg, 18 mmol) in pyridine/DMF (20:80 v/v, 0.300 mL) was added successively a solution of tert-butyl (2-(4-((S)-2-((S)-2-acetamido-3-methylbutanamido)propanamido)phenyl)-2-(((4-nitrophenoxy)carbonyl)oxy)ethyl)carbamate [previously described in WO2022207699] (13.8 mg, 1.2 eq), HOBt (2.5 mg, 1 eq) in pyridine/DMF (20/80) (0.550 mL) then DIPEA (0.0035 mL, 1.1 eq). The mixture was stirred 3h at 45°C in the dark and concentrated in vacuo. The crude was purified by chromatography on silicagel eluting with DCM/MeOH: gradient from 99/1 to 90/10 to afford Boc protected intermediate (17.6 mg, 98%) as a beige solid (foam). MS m/z (ESI+): Calc for C₄₇H₆₀BrN₁₂O₇ [M+H]+ = 983.39 ; Exp [M+H]+ = 983.4. HPLC Method 2, retention time = 5.40 min. The solid was dissolved in DCM (0.8 mL) and TFA (0.2 mL) was added at room temperature. The stirring was continued for 30 min and the mixture was concentrated in vacuo. The crude residue was taken up in 0.1% TFA (v/v) in water (1 mL) and purified using HPLC preparative method 5 to give compound B1 (16 mg, 80% yield from 2384). MS m/z (ESI+): Calc for C₄₂H₅₂BrN₁₂O₅ [M+H]+ = 883.34 ; Exp [M+H]+ = 883.3. HPLC Method 2, retention time = 4.46 min.

### Synthesis of intermediate B2 and DL-VA-2384

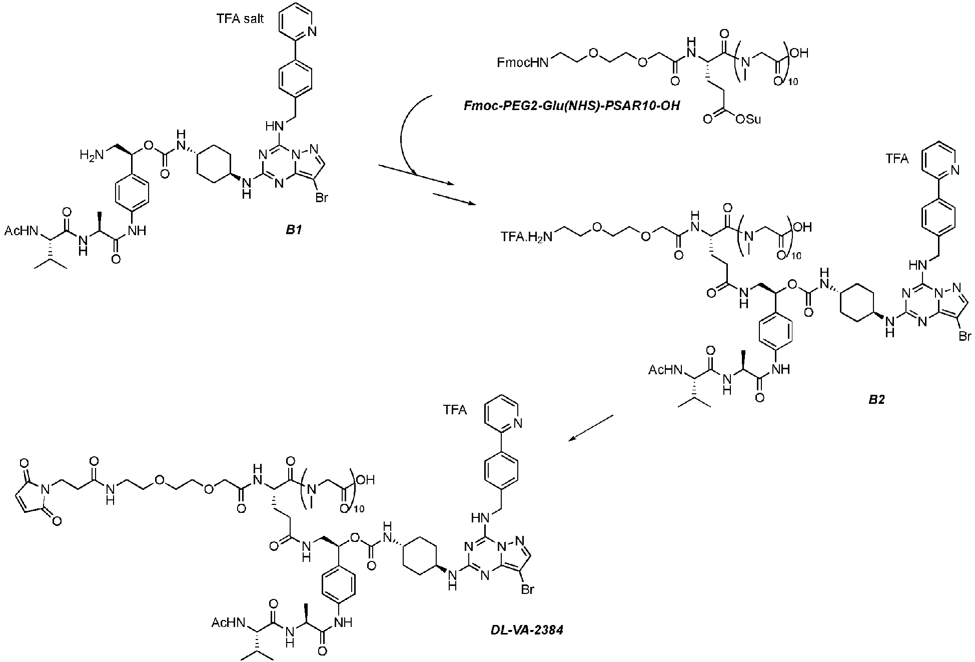

*First step*: To a clear solution of compound B1 (15 mg, 0.014 mmol) and PSAR derivative compound **Fmoc-PEG2-Glu(NHS)-PSAR10-OH** (24 mg, 1.3 eq) in DMF (0.140 mL) was added at room temperature Et3N (0.0078 mL, 4 eq). The mixture was stirred until total conversion of compound ***B1*** (1h 45 min, checked by HPLC). Piperidine (0.014 mL) was added and after 15 min at room temperature the mixture was diluted in a 10% TFA water sol./ACN: 1/1 (v/v) and purified by HPLC preparative method 6 to afford ***B2*** (17 mg, 59% yield from compound ***B1***). MS m/z (ESI+): Calc for C₈₃H₁₂₁BrN₂₄O₂₁Na [M+2H+Na]3+ = 630.6; Exp = 630.7. HPLC Method 2, retention time = 4.48 min. Method 3, retention time = 7.30 min. *Second step:* To a clear solution of intermediate compound ***B2*** (17 mg, 0.008 mmol) and 3-(Maleimido)propionic acid N-hydroxysuccinimide ester [CAS 55750-62-4] (2.8 mg, 1.3 eq) was added Et₃N and the mixture was stirred for 30 min. The reaction was quenched by adding a 1% TFA water sol./ACN: 1/1 (v/v) and purified by HPLC preparative method 6 to afford final drug linker DL-VA-2384 (10.7 mg (62%). HRMS m/z (ESI+): Calc for C₉₀H₁₂₇BrN₂₅O₂₄ [M+3H]3+ = 673.6218; Exp [M+3H]3+ = 673.6205. HPLC Method 3, retention time = 7.56 min.

### 2.2 Synthesis of drug-linker DL-GLUCU-2384

### Synthesis of intermediate B4

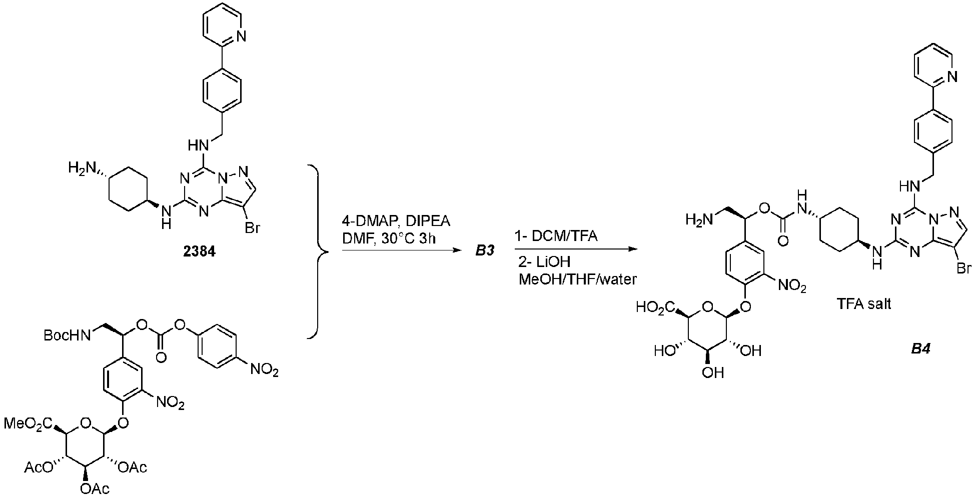

To the compound 2384 (25 mg, 0.042 mmol) in DMF (0.5 mL) was added DIPEA (0.018 mL, 2.5 eq.), 4-DMAP (4 mg, 0.8 eq.) and previously described (see patent WO2022207699) methyl (2S,3S,4S,5R,6S)-3,4,5-tris(acetyloxy)-6-{4-[(1S)-2-{[(tert-butoxy)carbonyl]amino}-1-{[(4-nitrophenoxy)carbonyl]oxy}ethyl]-2-nitrophenoxy}oxane-2-carboxylate (41 mg, 1.25 eq.). The mixture was stirred at 30°C for 3h and concentrated in vacuo. The residue was purified by chromatography on silicagel eluting from 0% to 20% MeOH in DCM to give compound B3 (42 mg, 88%) as a white foam. MS m/z (ESI+): Calc for C₅₀H₅₇BrN₁₀O₁₆ [M+H]+ = 1133.32 ; Exp = 1133.3. HPLC Method 2, retention time = 7.08 min. To a solution of this compound B3 (42 mg, 0.037 mmol) in DCM (0.280 mL) was added TFA (0.120 mL). The mixture was stirred at room temperature for 30 min and the mixture was concentrated under argon flow. Isopropylether (1 mL) was added, and the mixture sonicated, the supernatant was discarded and residues of solvents eliminated in vacuo. The amine deprotected intermediate product was diluted in MeOH/THF/water (5/2/6, 1.3 mL) and treated with LiOH.H2O (15.5 mg, 10 eq.) at 0°C for 40 min. AcOH (0.3 mL) was added and the residue purified by reverse phase chromatography method 5 to afford compound B4 (22 mg, 53% from compound B3) as a white solid. MS m/z (ESI+): Calc for C₃₈H₄₁BrN₁₀O₁₁ [M+2H]2+ = 447.1 ; Exp = 447.1. HPLC Method 2, retention time = 3.9 min.

### Synthesis of intermediate B5 and DL-GLUCU-2384

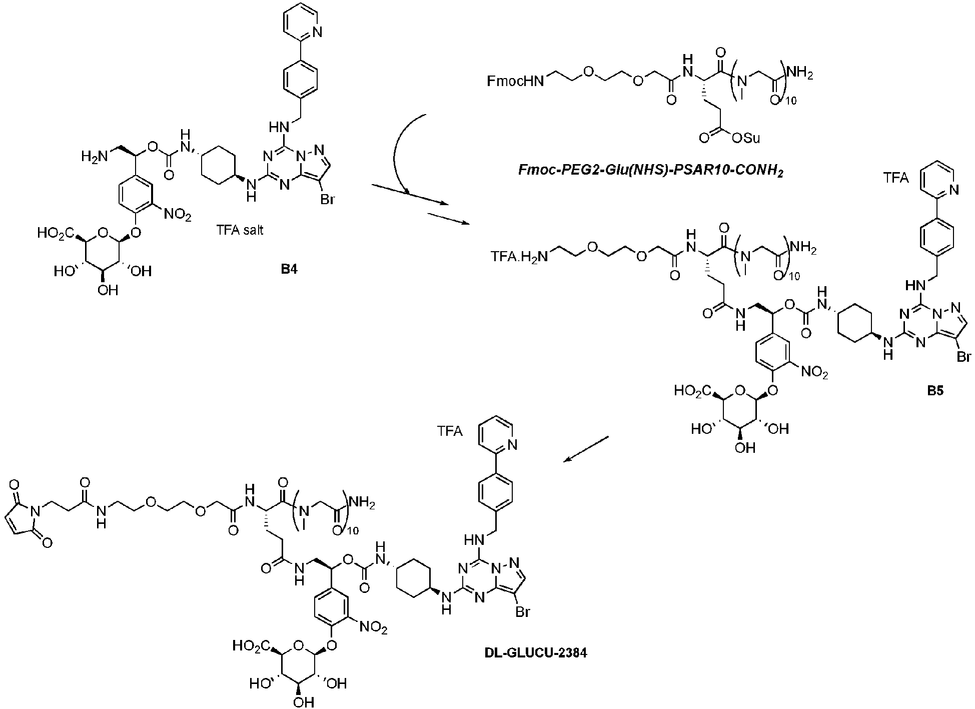

Intermediate compound B5 was prepared from intermediate B4 (22 mg, 0.0196 mmol) following procedure as described above for compound B2 synthesis. Compound B5 (38 mg, 92%) was obtained. MS m/z (ESI+): Calc for C₇₉H₁₁₀BrN₂₃O₂₆ [M+3H]3+ = 938.9 ; Exp = 938.9. HPLC method 2 retention time: 4.02 min, method 3 retention time: 6.29 min

Final compound DL-GLUCU-2384 was prepared from intermediate B5 (34.5 mg, 0.016 mmol) following procedure as described above for compound DL-VA-2384 synthesis. Final compound DL-GLUCU-2384 (27.8 mg, 79%) was obtained. HRMS m/z (ESI+): Calc for C₈₆H₁₁₇BrN₂₄O₂₉ [M+2H]2+ = 1014.3795; Exp = 1014.3796. HPLC method 3 retention time: 6.73 min.

### 2.3 Synthesis of drug-linker DL-VA-2388

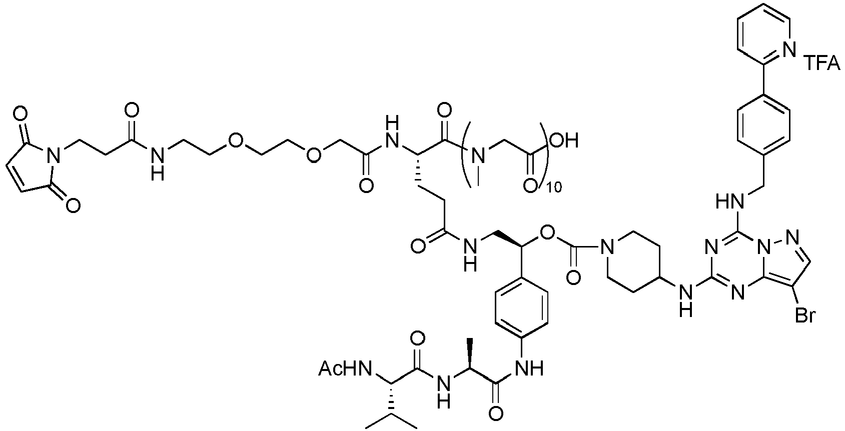

This compound was prepared following procedure described in above synthesis of DL-VA-2384, using compound 2388 instead of compound 2384. Final drug linker DL-VA-2388 (4 mg, 7% yield starting from compound 2388) was obtained as a white solid. HRMS m/z (ESI+): Calc for C₈₉H₁₂₄BrN₂₅O₂₄ [M+2H]2+ = 1002.9212; Exp = 1002.9229. HPLC method 3 retention time: 7.21 min.

### 2.4 Synthesis of drug-linker DL-VA-2568

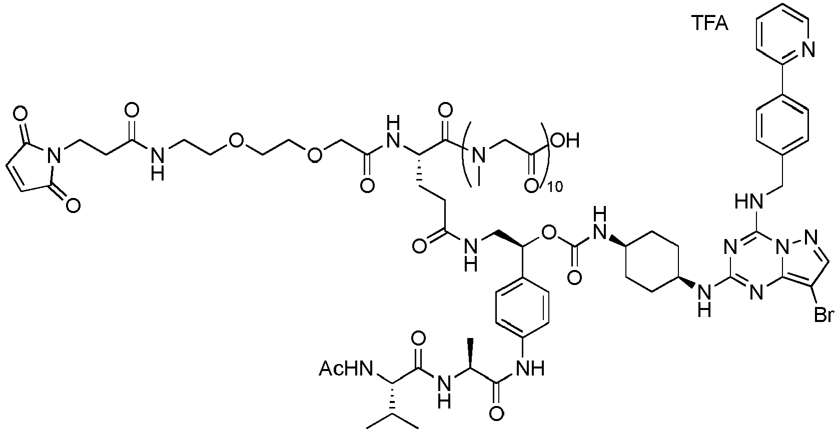

This compound was prepared following procedure described in above synthesis of DL-VA-2384, using compound 2568 instead of compound 2384. Final drug linker DL-VA-2568 (80 mg, 53% yield starting from compound 2568) was obtained as a white solid. HRMS m/z (ESI+): Calc for C₉₀H₁₂₆BrN₂₅O₂₄ [M+2H]2+ = 1009.9290; Exp = 1009.9293. HPLC method 3 retention time: 8.14 min.

### 2.5 Synthesis of drug-linker DL-VA-2600

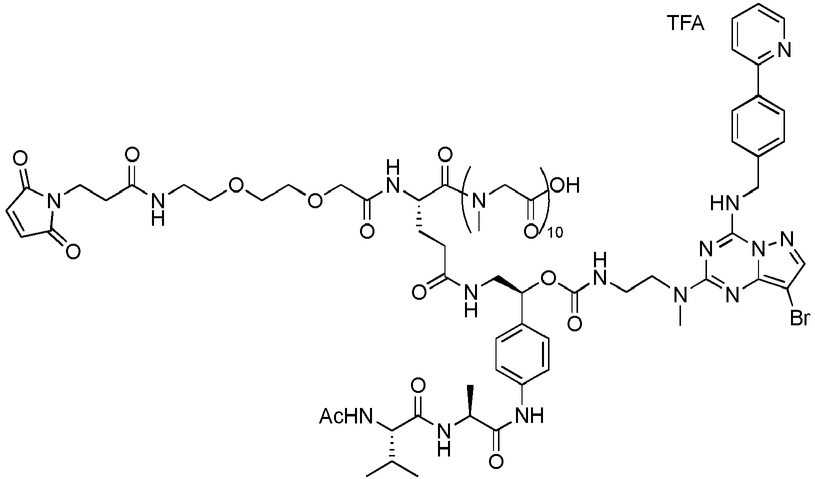

This compound was prepared following procedure described in above synthesis of DL-VA-2384, using compound 2600 instead of compound 2384. Final drug linker DL-VA-2600 (105 mg, 51% yield starting from compound 2600) was obtained as a white solid. HRMS m/z (ESI+): Calc for C₈₇H₁₂₁BrN₂₅O₂₄ [M+H+Na]2+ = 1000.9043; Exp = 1000.9034. HPLC method 3 retention time: 7.98 min.

### 2.6 Synthesis of drug-linker DL-VA-2605

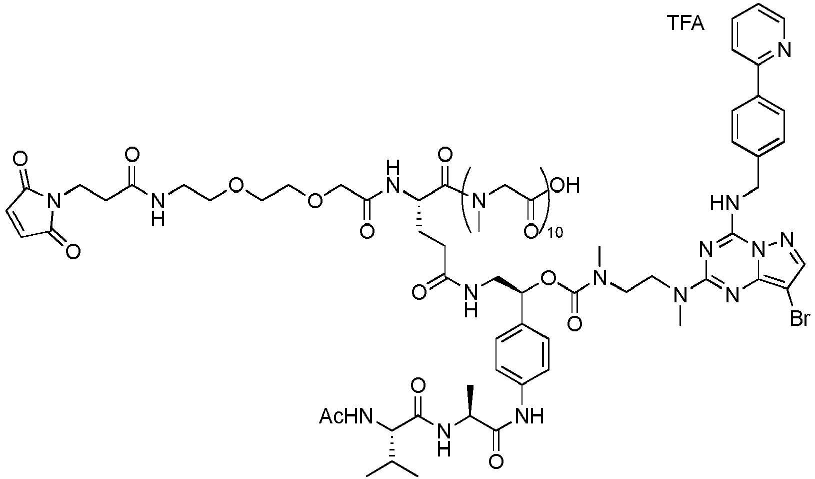

This compound was prepared following procedure described in above synthesis of DL-VA-2384, using compound 2605 instead of compound 2384. Final drug linker DL-VA-2605 (94 mg, 67% yield starting from compound 2605) was obtained as a white solid. HRMS m/z (ESI+): Calc for C₈₈H₁₂₃BrN₂₅O₂₄ [M+H+Na]2+ = 1007.9121; Exp = 1007.9126. HPLC method 3 retention time: 8.50 min.

### 2.7 Synthesis of drug-linker DL-VA-2636

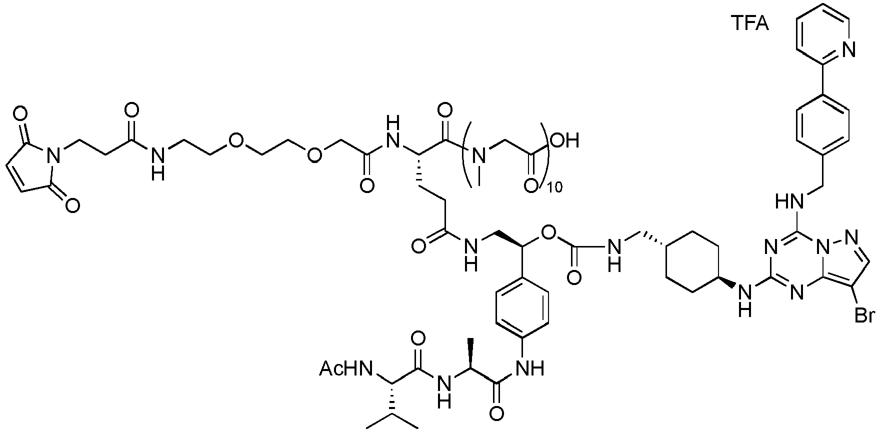

This compound was prepared following procedure described in above synthesis of DL-VA-2384, using compound 2636 instead of compound 2384. Final drug linker DL-VA-2636 (73 mg, 80% yield starting from compound 2636) was obtained as a white solid. HRMS m/z (ESI+): Calc for C₉₁H₁₂₈BrN₂₅O₂₄ [M+2H]2+ = 1016.9368; Exp = 1016.9372. HPLC method 3 retention time: 8.06 min.

### 2.8 Synthesis of drug-linker DL-VA-2655

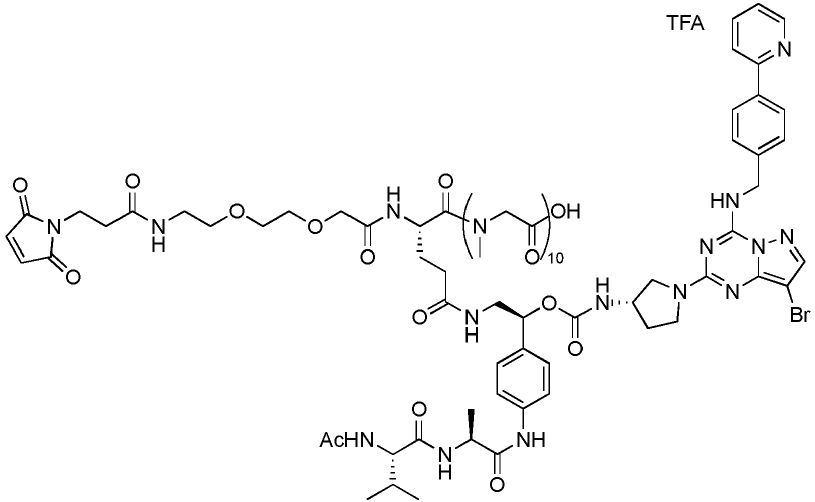

This compound was prepared following procedure described in above synthesis of DL-VA-2384, using compound 2655 instead of compound 2384. Final drug linker DL-VA-2655 (75 mg, 79% yield starting from compound 2655) was obtained as a white solid. HRMS m/z (ESI+): Calc for C₈₈H₁₂₂BrN₂₅O₂₄ [M+2H]2+ = 995.9133; Exp = 995.9132. HPLC method 3 retention time: 8.06 min.

### 2.9 Synthesis of drug-linker DL-VA-2668

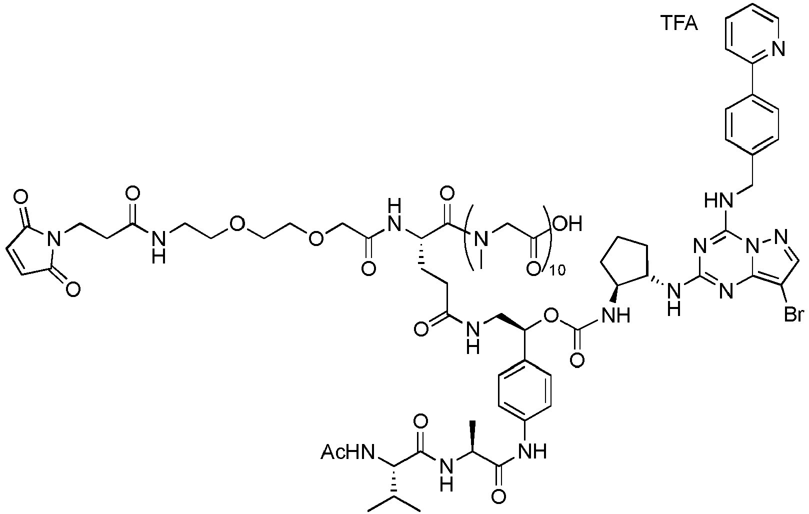

This compound was prepared following procedure described in above synthesis of DL-VA-2384, using compound 2668 instead of compound 2384. Final drug linker DL-VA-2668 (108 mg, 71% yield starting from compound 2668) was obtained as a white solid. HRMS m/z (ESI+): Calc for C₈₉H₁₂₄BrN₂₅O₂₄ [M+2H]2+ = 1002.9212; Exp = 1002.9215. HPLC method 3 retention time: 8.25 min.

### 2.10 Synthesis of drug-linker DL-VA-2671

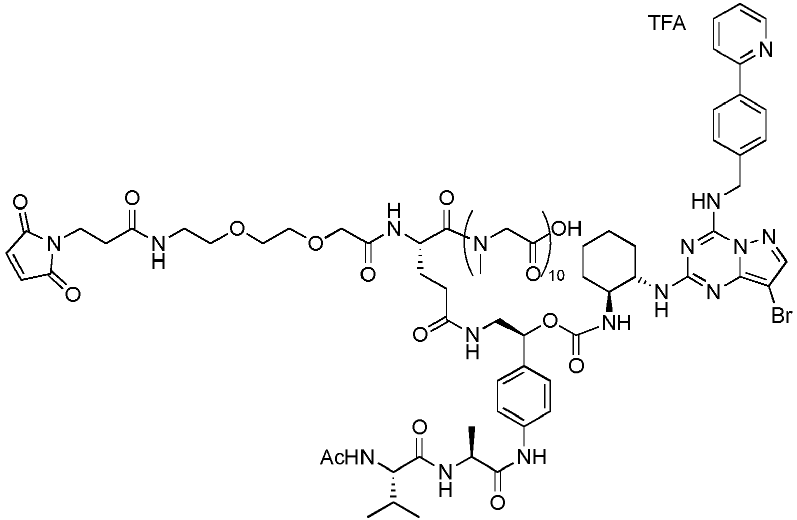

This compound was prepared following procedure described in above synthesis of DL-VA-2384, using compound 2671 instead of compound 2384. Final drug linker DL-VA-2671 (59 mg, 38% yield starting from compound 2671) was obtained as a white solid. HRMS m/z (ESI+): Calc for C₉₀H₁₂₆BrN₂₅O₂₄ [M+2H]2+ = 1009.9290; Exp = 1009.9280. HPLC method 3 retention time: 8.46 min.

### 2.11 Synthesis of drug-linker DL-VA-2420

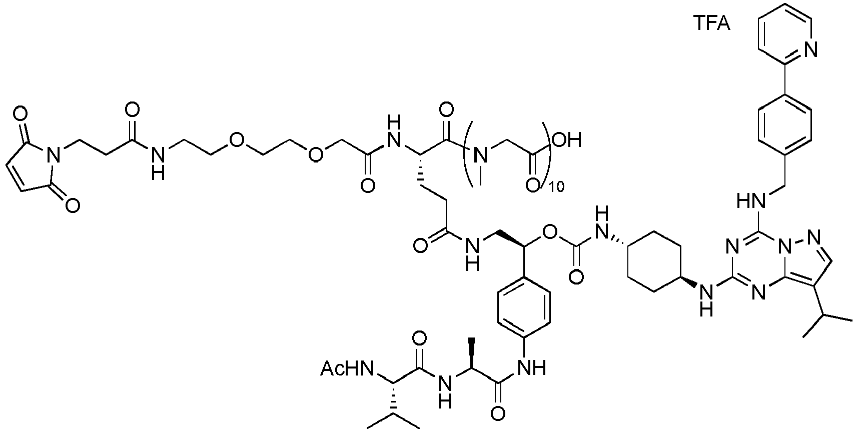

This compound was prepared following procedure described in above synthesis of DL-VA-2384, using compound 2420 instead of compound 2384. Final drug linker DL-VA-2420 (42.8 mg, 58% yield starting from compound 2420) was obtained as a white solid. HRMS m/z (ESI+): Calc for C₉₃H₁₃₁N₂₅O₂₄ [M+2H]2+ = 991.9972; Exp = 991.9968. HPLC method 3 retention time: 7.96 min.

### 2.12 Synthesis of drug-linker DL-VA-2428

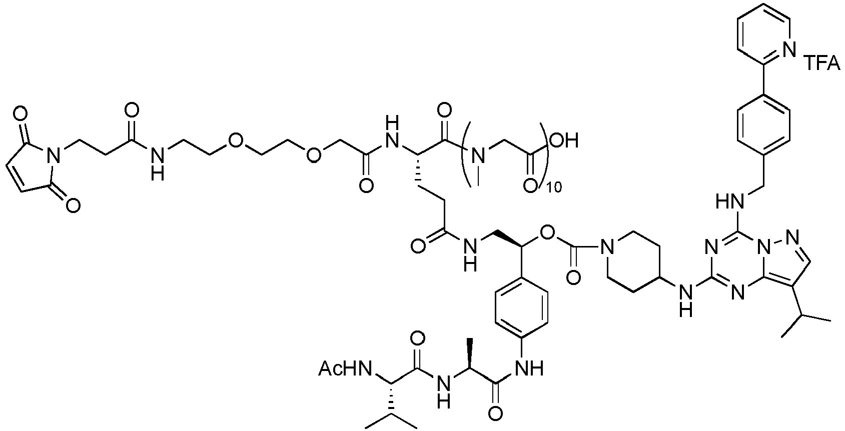

This compound was prepared following procedure described in above synthesis of DL-VA-2384, using compound 2428 instead of compound 2384. Final drug linker DL-VA-2428 (14 mg, 25% yield starting from compound 2428) was obtained as a white solid. HRMS m/z (ESI+): Calc for C₉₂H₁₂₉N₂₅O₂₄ [M+2H]+ = 984.9894; Exp = 984.9908. HPLC method 3 retention time: 7.79 min.

### Example 3: Preparation and characterization of antibody-drug conjugates (ADCs)

### 3.1) Antibody production and aminoacid sequences

Monoclonal antibodies were produced by transient transfection of CHO cells using art-recognized techniques (outsourced either to Evitria AG, Switzerland or WuXi Biologics, China). cDNAs were cloned into an appropriate vector system using conventional (non-PCR based) cloning techniques. pDNA was prepared under low-endotoxin conditions based on anion exchange chromatography. Correctness of the sequences was verified with Sanger sequencing. Suspension-adapted CHO were used for antibody production. Cell supernatant was harvested by centrifugation and subsequent filtration (0.2µm filter). The antibody was purified using protein A purification resin and SEC preparative chromatography if required. Purity of antibody materials was confirmed by SDS-PAGE and size exclusion chromatography and was over 95%. Endotoxin content was measured using the Charles River Endosafe PTS system. Human IgG1k non-binding isotype control was cat#HG1K from Sino Biologicals (proprietary amino acid sequences). Trastuzumab (Herceptin^{®} 150mg), trastuzumab emtansine (Kadcyla^{®} 160mg) were purchased from Roche. Trastuzumab deruxtecan (Enhertu^{®}) was purchased from AstraZeneca.

Table 1 below presents the aminoacid sequences of heavy and light chain monoclonal antibodies used in the present application. Those aminoacid sequences were obtained from the available literature (scientific article, patent applications, International Non proprietary Names for Pharmaceutical Substances (INN) lists published in the World Health Organization).

**Table 1: aminoacid sequences of heavy and light chain monoclonal antibodies used for practicing the invention**

| **SEQ ID** | **Name of the monoclonal antibody** | **Aminoacid sequence** |
|---|---|---|
| 1 | Farletuzumab_LALA (heavy chain) | EVQLVESGGGVVQPGRSLRLSCSASGFTFSGYGLSWVRQAPGKGLEWVAMISSGGSYTYYADSVKGRF AISRDNAKNTLFLQMDSLRPEDTGVYFCARHGDDPAWFAYWGQGTPVTVSSASTKGPSVFPLAPSSKSTS GGTAALGCLVKDYFPEPVTVSWNSGALTSGVHTFPAVLQSSGLYSLSSVVTVPSSSLGTQTYICNVNHKPS NTKVDKKVEPKSCDKTHTCPPCPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 2 | Farletuzumab_LALA (light chain) | DIQLTQSPSSLSASVGDRVTITCSVSSSISSNNLHWYQQKPGKAPKPWIYGTSNLASGVPSRFSG SGSGTDYTFTISSLQPEDIATYYCQQWSSYPYMYTFGQGTKVEIKRTVAAPSVFIFPPSDEQLKS GTASVVCLLNNFYPREAKVQWKVDNALQSGNSQESVTEQDSKDSTYSLSSTLTLSKADYEKHK VYACEVTHQGLSSPVTKSFNRGEC |
| 3 | Milatuzumab (heavy chain) | QVQLQQSGSELKKPGASVKVSCKASGYTFTNYGVNWIKQAPGQGLQWMGWINPNTGEPTFDDDFKGRF AFSLDTSVSTAYLQISSLKADDTAVYFCSRSRGKNEAWFAYWGQGTLVTVSSASTKGPSVFPLAPSSKSTS GGTAALGCLVKDYFPEPVTVSWNSGALTSGVHTFPAVLQSSGLYSLSSVVTVPSSSLGTQTYICNVNHKPS NTKVDKRVEPKSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAKGQPREPQ VYTLPPSREEMTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 4 | Milatuzumab (light chain) | DIQLTQSPLSLPVTLGQPASISCRSSQSLVHRNGNTYLHWFQQRPGQSPRLLIYTVSNRFSGVPDRFSGSG SGTDFTLKISRVEAEDVGVYFCSQSSHVPPTFGAGTRLEIKRTVAAPSVFIFPPSDEQLKSGTASVVCLLNN FYPREAKVQWKVDNALQSGNSQESVTEQDSKDSTYSLSSTLTLSKADYEKHKVYACEVTHQGLSSPVTKS FNRGEC |
| 5 | huMy9-6 (heavy chain) | QVQLQQPGAEVVKPGASVKMSCKASGYTFTSYYIHWIKQTPGQGLEWVGVIYPGNDDISYNQKFQGKATL TADKSSTTAYMQLSSLTSEDSAVYYCAREVRLRYFDVWGQGTTVTVSSASTKGPSVFPLAPSSKSTSGGT AALGCLVKDYFPEPVTVSWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVVDVSHEDPEVKFNWYVD |
| | | GVEVHNAKTKPREEQYNSTYRVVSVLTVLHQDWLNGKEYKCKVSNKALPAPIEKTISKAKGQPREPQVYTL PPSRDELTKNQVSLTCLVKGFYPSDIAVEWESNGQPENNYKTTPPVLDSDGSFFLYSKLTVDKSRWQQGN VFSCSVMHEALHNHYTQKSLSLSPGK |
| 6 | huMy9-6 (light chain) | EIVLTQSPGSLAVSPGERVTMSCKSSQSVFFSSSQKNYLAWYQQIPGQSPRLLIYWASTRESGVPDRFTG SGSGTDFTLTISSVQPEDLAIYYCHQYLSSRTFGQGTKLEIKRTVAAPSVFIFPPSDEQLKSGTASVVCLLNN FYPREAKVQWKVDNALQSGNSQESVTEQDSKDSTYSLSSTLTLSKADYEKHKVYACEVTHQGLSSPVTKS FNRGEC |
| 7 | Brentuximab_LALA (heavy chain) | QIQLQQSGPEVVKPGASVKISCKASGYTFTDYYITWVKQKPGQGLEWIGWIYPGSGNTKYNEKFKGKATLT VDTSSSTAFMQLSSLTSEDTAVYFCANYGNYWFAYWGQGTQVTVSAASTKGPSVFPLAPSSKSTSGGTA ALGCLVKDYFPEPVTVSWNSGALTSGVHTFPAVLQSSGLYSLSSVVTVPSSSLGTQTYICNVNHKPSNTKV DKKVEPKSCDKTHTCPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTVDKSRWQQGNV FSCSVMHEALHNHYTQKSLSLSPG |
| 8 | Brentuximab_LALA (light chain) | DIVLTQSPASLAVSLGQRATISCKASQSVDFDGDSYMNWYQQKPGQPPKVLIYAASNLESGIPARFSGSGS GTDFTLNIHPVEEEDAATYYCQQSNEDPWTFGGGTKLEIKRTVAAPSVFIFPPSDEQLKSGTASVVCLLNNF YPREAKVQWKVDNALQSGNSQESVTEQDSKDSTYSLSSTLTLSKADYEKHKVYACEVTHQGLSSPVTKSF NRGEC |

### 3.2) Preparation of antibody-drug conjugates (ADCs)

A solution of antibody (10 mg/mL in PBS 7.4 + 1 mM EDTA) was treated with a required amount (2.2 molar equivalent for final ~DAR4 ADC or 14 molar equivalent for final DAR8 ADC) of tris(2-carboxyethyl)phosphine (TCEP) for 1-2 hours at 37°C. The reduced antibody was buffer-exchanged with potassium phosphate 100 mM pH 7.4 + 1 mM EDTA by three rounds of dilution/centrifugation using Amicon 30K centrifugal filters device (Millipore). For a final ~DAR4 ADC, 6 molar equivalents of drug-linker (from a 12 mM DMSO stock solution) was added to the antibody. For a final DAR8 ADC, 10-12 molar equivalents of drug-linker was added. The solution was incubated 30 min at room temperature. The final conjugate was buffer-exchanged/purified with 3 rounds of PBS pH 7.4 buffer and 2 runs of histidine sucrose formulation buffer (20 mM histidine buffer pH 6.0, 4% (w/v) sucrose + 75 mM NaCl) using Amicon 30K centrifugal filters device and was sterile-filtered (0.20µm PES filter).

Final protein concentration was assessed spectrophotometrically at 280 nm using a Nanodrop One device (Thermo Fisher). Final endotoxin content was assessed using Pierce^{™} LAL chromogenic endotoxin testing kit (Thermo Fisher).

### 3.3) Characterization of antibody-drug conjugates

The resulting conjugates were characterized as follows:
Drug-Antibody-Ratio (DAR) assessment by reverse phase liquid chromatography-mass spectrometry (RPLC-MS):
Denaturing RPLC-QToF analysis was performed using the HPLC method 4 described above in Example 1. Briefly, conjugates were eluted on an Agilent PLRP-S 1000Å 2.1x150mm 8µm (80°C) using a mobile phase gradient of water/acetonitrile + 0.1% formic acid (0.4 mL/min) and detected using a Bruker Impact IITM Q-ToF mass spectrometer scanning the 500-3500 m/z range (ESI+). Data were deconvoluted using the MaxEnt algorithm included in the Bruker Compass^{®} software. Optionally, ADC samples were deglycosylated using Genovis IgGZERO enzyme prior to MS analysis, following manufacturer's protocol.

### Reverse phase liquid chromatography (RPLC-UV):

Denaturing RPLC-UV analysis was also conducted on an Agilent 1100 HPLC-DAD system, using a slightly modified version of HPLC method 4 described above. Mobile phase modifier 0.1% formic acid was replaced with 0.1% TFA, and detection was made using a DAD UV absorbance only (no mass spectrometry detector).

### Size exclusion chromatography (SEC):

SEC was performed on an Agilent 1100 HPLC system having an extra-column volume below 15µL (equipped with short sections of 0.12mm internal diameter peek tubing and a micro-volume UV flow cell). Column was an Agilent AdvanceBioSEC 300Å 4.6x150mm 2.7µm (maintained at 30°C). Mobile phase was 100 mM sodium phosphate and 200 mM sodium chloride (pH 6.8). 10% acetonitrile (v/v) was added to the mobile phase to minimize secondary hydrophobic interactions with the stationary phase and prevent bacterial growth. Flow rate was 0.35 mL/min. UV detection was monitored at 280 nm or any other relevant wavelength.

### 3.4) Characterization overview of synthesized antibody-drug conjugates

**Table 2: characterization overview of the synthesized antibody-drug conjugates**

| **Name of the conjugate** | **Drug-linker component** | **Antibody component** | **DAR** | **Deconvoluted mass spectrometry data** | **Monomeric purity (%)** |
|---|---|---|---|---|---|
| **TRA-VA-2384** | DL-VA-2384 | Trastuzumab | 8 | Obs LC-1d: 25459 Da (Mass error 1 Da) | >95% |
| | | | | Obs HC-3d: 56653 Da (Mass error 2 Da) | |
| **TRA-VA-2388** | DL-VA-2388 | Trastuzumab | 8 | Obs LC-1d: 25445 Da (Mass error 0 Da) | >95% |
| | | | | Obs HC-3d: 56612 Da (Mass error 3 Da) | |
| **TRA-VA-2568** | DL-VA-2568 | Trastuzumab | 8 | Obs LC-1d: 25459 Da (Mass error 0 Da) | >95% |
| | | | | Obs deglycosylated HC-3d: 55558 Da (Mass error 3 Da) | |
| **TRA-VA-2600** | DL-VA-2600 | Trastuzumab | 8 | Obs LC-1d: 25419 Da (Mass error 0 Da) | >95% |
| | | | | Obs deglycosylated HC-3d: 55436 Da (Mass error 1 Da) | |
| **TRA-VA-2605** | DL-VA-2605 | Trastuzumab | 8 | Obs LC-1d: 25433 Da (Mass error 0 Da) | >95% |
| | | | | Obs deglycosylated HC-3d: 55480 Da (Mass error 1 Da) | |
| **TRA-VA-2636** | DL-VA-2636 | Trastuzumab | 8 | Obs LC-1d: 25472 Da (Mass error 0 Da) | >95% |
| | | | | Obs deglycosylated HC-3d: 55598 Da (Mass error 2 Da) | |
| **TRA-VA-2655** | DL-VA-2655 | Trastuzumab | 8 | Obs LC-1d: 25430 Da (Mass error 0 Da) | >95% |
| | | | | Obs deglycosylated HC-3d: 55473 Da (Mass error 3 Da) | |
| **TRA-VA-2668** | DL-VA-2668 | Trastuzumab | 8 | Obs LC-1d: 25444 Da (Mass error 0 Da) | >95% |
| | | | | Obs deglycosylated HC-3d: 55516 Da (Mass error 1 Da) | |
| **TRA-VA-2671** | DL-VA-2671 | Trastuzumab | 8 | Obs LC-1d: 25458 Da (Mass error 0 Da) | >95% |
| | | | | Obs deglycosylated HC-3d: 55557 Da (Mass error 3 Da) | |
| **FARLE_LALA-VA-2384** | DL-VA-2384 | Farletuzumab_LALA | 8 | Obs LC-1d: 25770 Da (Mass error 0 Da) | >95% |
| | | | | Obs HC-3d: 56230 Da (Mass error 3 Da) | |
| **FARLE_LALA-VA-2388** | DL-VA-2388 | Farletuzumab_LALA | 8 | Obs LC-1d: 25757 Da (Mass error 0 Da) | >95% |
| | | | | Obs deglycosylated HC-3d: 55092 Da (Mass error 0 Da) | |
| **FARLE_LALA-VA-2568** | DL-VA-2568 | Farletuzumab_LALA | 8 | Obs LC-1d: 25770 Da (Mass error 0 Da) | >95% |
| | | | | Obs deglycosylated HC-3d: 55136 Da (Mass error 1 Da) | |
| **FARLE_LALA-VA-2600** | DL-VA-2600 | Farletuzumab_LALA | 8 | Obs LC-1d: 25731 Da (Mass error 0 Da) | >95% |
| | | | | Obs deglycosylated HC-3d: 55013 Da (Mass error 1 Da) | |
| **FARLE_LALA-VA-2605** | DL-VA-2605 | Farletuzumab_LALA | 8 | Obs LC-1d: 25745 Da (Mass error 0 Da) | >95% |
| | | | | Obs deglycosylated HC-3d: 55056 Da (Mass error 2 Da) | |
| **FARLE_LALA-VA-2636** | DL-VA-2636 | Farletuzumab_LALA | 8 | Obs LC-1d: 25785 Da (Mass error 0 Da) | >95% |
| | | | | Obs deglycosylated HC-3d: 55177 Da (Mass error 3 Da) | |
| **FARLE_LALA-VA-2655** | DL-VA-2655 | Farletuzumab_LALA | 8 | Obs LC-1d: 25743 Da (Mass error 0 Da) | >95% |
| | | | | Obs deglycosylated HC-3d: 55051 Da (Mass error 1 Da) | |
| **FARLE_LALA-VA-2668** | DL-VA-2668 | Farletuzumab_LALA | 8 | Obs LC-1d: 25758 Da (Mass error 0 Da) | >95% |
| | | | | Obs deglycosylated HC-3d: 55092 Da (Mass error 3 Da) | |
| **FARLE_LALA-VA-2671** | DL-VA-2671 | Farletuzumab_LALA | 8 | Obs LC-1d: 25770 Da (Mass error 0 Da) | >95% |
| | | | | Obs deglycosylated HC-3d: 55135 Da (Mass error 2 Da) | |
| **huMy9-6-VA-2384** | DL-VA-2384 | huMy9-6 | 8 | Obs LC-1d: 26287 Da (Mass error 0 Da) | >9 5% |
| | | | | Obs HC-3d: 56492 Da (Mass error 4 Da) | |
| **MILA-VA-2384** | DL-VA-2384 | Milatuzumab | 8 | Obs LC-1d: 26017 Da (Mass error 0 Da) | >95% |
| | | | | Obs HC-3d: 56690 Da (Mass error 2 Da) | |
| **NEG-VA-2384** | DL-VA-2384 | Neg. control IgG Sino Bio. #HG1K | 8 | Obs LC-1d: 25428 Da (Mass error 0 Da) | >95% |
| | | | | Obs deglycosylated HC-3d: 54223 Da (Mass error 1 Da) | |
| **TRA-GLUCU-2384** | DL-GLUCU-2384 | Trastuzumab | 8 | Obs LC-1d: 25467 Da (Mass error 0 Da) | >95% |
| | | | | Obs deglycosylated HC-3d: 55583 Da (Mass error 1 Da) | |
| **FARLE_LALA-GLUCU-2384** | DL-GLUCU-2384 | Farletuzumab_LALA | 8 | Obs LC-1d: 25780 Da (Mass error 0 Da) | >95% |
| | | | | Obs deglycosylated HC-3d: 55162 Da (Mass error 2 Da) | |
| **BRENT_LALA-GLUCU-2384** | DL-GLUCU-2384 | Brentuximab_LALA | 8 | Obs LC-1d: 25754 Da (Mass error 0 Da) | >95% |
| | | | | Obs deglycosylated HC-3d: 55227 Da (Mass error 1 Da) | |
| **huMy9-6-VA-2388** | DL-VA-2388 | huMy9-6 | 8 | Obs LC-1d: 26273 Da (Mass error 0 Da) | >95% |
| | | | | Obs HC-3d: 56447 Da (Mass error 1 Da) | |
| **MILA-VA-2388** | DL-VA-2388 | Milatuzumab | 8 | Obs LC-1d: 26004 Da (Mass error 0 Da) | >95% |
| | | | | Obs HC-3d: 56652 Da (Mass error 2 Da) | |
| **NEG-VA-2388** | DL-VA-2388 | Neg. control IgG Sino Bio. #HG1K | 8 | Obs LC-1d: 25416 Da (Mass error 0 Da) | >95% |
| | | | | Obs HC-3d: 55277 Da (Mass error 3 Da) | |
| **TRA-VA-2420** | DL-VA-2420 | Trastuzumab | 8 | Obs LC-1d: 25421 Da (Mass error 0 Da) | >95% |
| | | | | Obs HC-3d: 56542 Da (Mass error 1 Da) | |
| **huMy9-6-VA-2420** | DL-VA-2420 | huMy9-6 | 8 | Obs LC-1d: 26250 Da (Mass error 0 Da) | >95% |
| | | | | Obs HC-3d: 56382 Da (Mass error 1 Da) | |
| **MILA-VA-2420** | DL-VA-2420 | Milatuzumab | 8 | Obs LC-1d: 25981 Da (Mass error 1 Da) | >95% |
| | | | | Obs HC-3d: 56583 Da (Mass error 2 Da) | |
| **TRA-VA-2428** | DL-VA-2428 | Trastuzumab | 8 | Obs LC-1d: 25407 Da (Mass error 0 Da) | >95% |
| | | | | Obs HC-3d: 56500 Da (Mass error 1 Da) | |
| **huMy9-6-VA-2428** | DL-VA-2428 | huMy9-6 | 8 | Obs LC-1d: 26236 Da (Mass error 0 Da) | >95% |
| | | | | Obs HC-3d: 56339 Da (Mass error 1 Da) | |
| **MILA-VA-2428** | DL-VA-2428 | Milatuzumab | 8 | Obs LC-1d: 25967 Da (Mass error 1 Da) | >95% |
| | | | | Obs HC-3d: 56540 Da (Mass error 1 Da) | |

| | | | | | |
|---|---|---|---|---|---|
| *LC-0d: light chain ; LC-1d: light chain with 1 drug-linker ; HC-0d: heavy chain ; HC-1d: heavy chain with 1 drug-linker ; HC-2d: heavy chain with 2 drug-linkers ; HC-3d: heavy chain with 3 drug-linkers. Major glycoform is reported for HC.* | | | | | |

### Example 4: General cell culture practices and animal experiments

Human cancer cell lines used in the present project were purchased from either the American Type Culture Collection (ATCC), The Leibniz Institute DSMZ German Collection of Microorganisms and Cell Cultures GmbH (DSMZ) or the European Collection of Authenticated Cell Cultures (ECACC). For use in experiments, cells were cultivated following good and established cell culture practices, following instructions from the original ATCC/DSMZ/ECACC supplier for cell culture media & supplement, cryopreservation, and subculturing procedures. Cells were incubated under a 5% CO₂ atmosphere at 37°C for no longer than 2 months.

All animal procedures were performed in accordance with the European Union directive 86/609/EEC. Experiments were performed under individual permit and in accredited animal care facilities.

### Example 5: In vitro cytotoxicity assay of 2-substituted 8-halogeno-N4-(4-(pyridin-2-yl) benzyl) pyrazolo[1,5-a][1,3,5] triazine-2,4-diamine family of compounds

In vitro cytotoxicity of compounds of the disclosure was assessed on several cancerous human cell lines. Cells were plated in 96-well plates at an appropriate density depending on the cell line (between 1000 and 10 000 cells/well in 100µL of appropriate culture media) and incubated at 37°C for 24 hours. Serial dilutions of the tested compound previously dissolved in culture media (50µL) were added, and incubation was carried at 37°C out for 6 days. MTT (5mg/mL in PBS, 20*µ*L, Sigma-Aldrich) was added into the wells, and incubation was continued for 1 to 4 hours at 37°C. Culture media was then carefully removed, and well content was homogeneously dissolved with acidified isopropanol. Absorbance values were measured on a microplate reader using a wavelength of 570 nm (with a reference wavelength of 690 nm). The IC₅₀ concentration values compared to untreated control cells were determined using inhibition dose response curve fitting (GraphPad Prism 9). Exatecan mesylate (cat#HY-13631A) and CR8 (CAS# 294646-77-8; cat#HY-18340) were purchased from MedChemExpress (China).

Table 3 below shows the observed in vitro cytotoxicity IC₅₀ values of the compounds of the disclosure in a panel of human cancer cell lines. Low nanomolar efficacies were observed with compounds of the invention. Compared to the known CycK molecular glue degrader compound CR8 (see: Stabicki, M., Kozicka, Z., Petzold, G. et al. The CDK inhibitor CR8 acts as a molecular glue degrader that depletes cyclin K. Nature 585, 293-297, 2020), compounds of the invention are 2-100 fold more potent.

**Table 3: summary of in vitro cytotoxicity IC₅₀ values of compounds of the disclosure (in nanomolar)**

| | **Cell line** | | | | | | | | | | | | | |
|---|---|---|---|---|---|---|---|---|---|---|---|---|---|---|
| **Compound** | **BT-474** | **NCI-N87** | **MDA-MB-453** | **SKBR-3** | **MDA-MB-231** | **JIMT-1** | **MCF-7** | **HT-29** | **BXPC3** | **OE-19** | **A549** | **KB-1** | **HCT-116** | **Caco-2** |
| **2384** | 0.94 | 0.76 | 0.53 | 0.73 | 0.38 | 0.25 | 3.42 | 0.22 | 1.46 | 2.24 | 0.56 | 0.38 | 1.19 | 6.64 |
| **2388** | 1.64 | 1.40 | 1.27 | 1.68 | 0.76 | 0.64 | 5.40 | 0.23 | 1.97 | 3.77 | 0.65 | | 1.66 | 4.46 |
| **2420** | 3.41 | 4.93 | 2.73 | 3.25 | 0.70 | 0.61 | 7.78 | 1.74 | | 6.91 | 1.99 | | 4.40 | 56.15 |
| **2428** | 3.73 | 4.50 | 3.11 | 1.94 | 2.31 | 0.92 | 9.12 | 0.83 | | 7.84 | 2.22 | | 5.31 | 24.20 |
| **2478** | 1.72 | 6.88 | 1.34 | 17.59 | 1.64 | 1.18 | | | | | | | | |
| **2533** | >100 | >100 | >100 | >100 | >100 | >100 | | | | | | | | |
| **2538** | 6.54 | 21.59 | 8.12 | 39.15 | 8.75 | 5.13 | | | | | | | | |
| **2554** | >100 | >100 | >100 | >100 | >100 | >100 | | | | | | | | |
| **2559** | 2.63 | 1.32 | 1.52 | 3.73 | 1.35 | 0.41 | 12.85 | 0.21 | 1.63 | | | | | |
| **2560** | 5.92 | 3.29 | 1.61 | 4.33 | 1.92 | 0.60 | | | | | | | | |
| **2568** | 1.75 | 2.22 | 1.67 | 4.36 | 0.98 | 0.62 | 11.57 | 0.46 | 4.86 | | | 4.95 | | |
| **2569** | 15.22 | 19.70 | 12.81 | 62.61 | 11.24 | 6.70 | | | | | | | | |
| **2571** | 2.68 | 4.27 | 3.55 | 7.75 | 2.22 | 1.53 | 79.63 | 1.12 | 5.43 | | | | | |
| **2584** | 9.72 | 9.44 | 12.33 | 25.91 | 7.17 | 7.61 | | | | | | | | |
| **2585** | 1.77 | 15.41 | 26.75 | 38.89 | 24.72 | 7.86 | | | | | | | | |
| **2586** | 1.73 | 3.14 | 2.47 | 6.59 | 1.44 | 1.13 | 33.90 | 0.45 | 2.44 | | | | | |
| **2590** | 1.33 | 2.42 | 1.76 | 2.55 | 0.74 | 0.29 | | | | | | | | |
| **2600** | 1.18 | 1.32 | 1.17 | 1.69 | 0.29 | 0.19 | 12.19 | 0.28 | 1.16 | | | 1.48 | | |
| **2601** | 1.86 | 2.70 | 1.92 | 3.61 | 0.86 | 0.45 | 15.36 | 0.13 | 1.72 | | | | | |
| **2605** | 2.76 | 4.50 | 3.66 | 7.46 | 1.54 | 1.22 | 3.70 | 0.33 | 2.91 | | | | | |
| **2606** | 0.92 | 0.77 | 0.64 | 1.23 | 0.46 | 0.88 | 6.88 | 0.13 | 1.16 | | | | | |
| **2622** | 13.47 | 3.00 | 21.14 | 2.24 | 12.72 | 4.84 | >100 | 8.25 | 36.13 | | | | | |
| **2629** | 1.75 | 0.94 | 0.77 | 0.27 | 0.66 | 0.11 | 3.91 | 0.36 | 1.17 | | | | | |
| **2635** | 23.12 | 15.88 | 17.94 | 0.83 | 12.87 | 3.52 | >100 | 8.93 | 39.70 | | | | | |
| **2636** | 3.68 | 2.46 | 1.97 | 0.17 | 1.46 | 0.12 | 33.12 | 1.28 | 4.68 | | | 8.10 | | |
| **2659** | 0.72 | 0.34 | 0.40 | 0.72 | 0.26 | | | | | | | | | |
| **2660** | 1.25 | 0.49 | 0.68 | 0.11 | 0.43 | | | | | | | | | |
| **2638** | 0.78 | 0.42 | 0.88 | 0.82 | 0.30 | | | | | | | | | |
| **2658** | 4.57 | 6.43 | 7.84 | 0.77 | 1.98 | | | | | | | | | |
| **2655** | 1.57 | 1.95 | 0.97 | 0.14 | 0.54 | | | | | | | 1.66 | | |
| **2656** | 2.52 | 1.42 | 1.13 | 0.17 | 0.65 | | | | | | | | | |
| **2657** | 1.71 | 0.43 | 0.77 | 0.13 | 0.56 | | | | | | | | | |
| **2668** | 2.16 | 0.84 | 1.78 | 0.20 | 0.82 | | | | | | | 2.99 | | |
| **2669** | 1.75 | 0.39 | 0.59 | 0.67 | 0.44 | | | | | | | | | |
| **2670** | 5.77 | 2.53 | 9.19 | 0.72 | 2.67 | | | | | | | | | |
| **2671** | 3.34 | 1.47 | 2.22 | 0.42 | 1.74 | | | | | | | 7.60 | | |
| **2672** | 2.12 | 0.55 | 0.76 | 0.24 | 0.46 | | | | | | | | | |
| **CR8*** | 18.4 | 62.2 | 40.3 | 30.6 | 10.4 | 25.2 | | | | | | | | |

| | | | | | | | | | | | | | | |
|---|---|---|---|---|---|---|---|---|---|---|---|---|---|---|
| *CR8 is a known molecular glue degrader of CycK (**see: S** 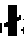 **abicki**, M., Kozicka, Z., Petzold, G. et al. The CDK inhibitor CR8 acts as a molecular glue degraderthat depletes cyclin K. Nature 585, 293-297, 2020), but with limited efficacy (high nM IC₅₀ potency range) | | | | | | | | | | | | | | |

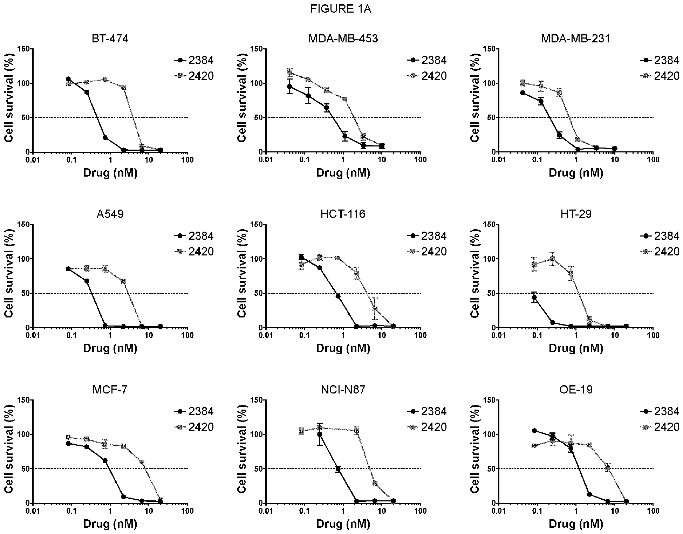

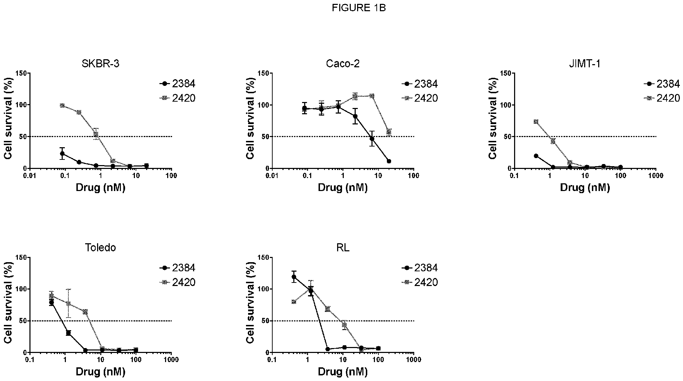

Figures 1A and 1B show side-by-side comparison of halogenated compound 2384 to its isopropyl counterpart 2420. The halogenated compound 2384 is more potent compared to its isopropyl counterpart. This shows that inclusion of a halogen atom in this position increases compound efficacy.

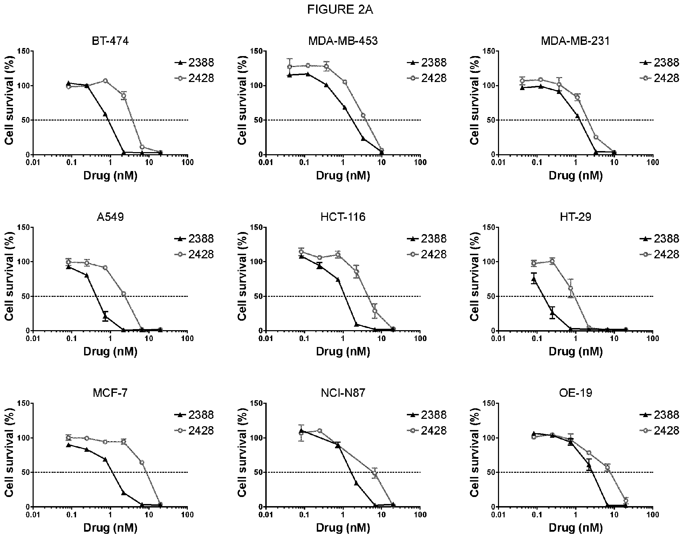

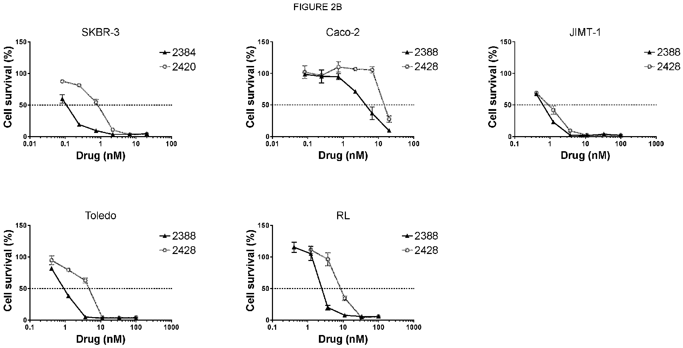

Figures 2A and 2B show side-by-side comparison of halogenated compound 2388 of the disclosure to its isopropyl counterpart 2428. The halogenated compound 2384 is more potent compared to its isopropyl counterpart. This shows that inclusion of a halogen atom in this position increases compound efficacy.

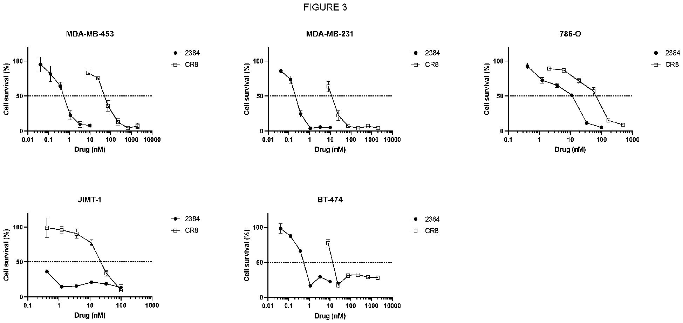

Figure 3 shows side-by-side comparison of compound 2384 of the disclosure to the known CycK molecular glue degrader compound CR8 (see: S 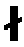 abicki, M., Kozicka, Z., Petzold, G. et al. The CDK inhibitor CR8 acts as a molecular glue degrader that depletes cyclin K. Nature 585, 293-297, 2020). The halogenated compound 2384 shows drastically improved low nanomolar potencies, whereas CR8 shows potencies in the high nanomolar range.

This example shows that the series of halogenated compounds of the invention are more potent than their isopropyl counterpart and than the known molecular glue degrader CR8.

### Example 6: In vivo mice tolerability assessment of 2-substituted 8-halogeno-N4-(4-(pyridin-2-yl) benzyl) pyrazolo[1,5-a][1,3,5] triazine-2,4-diamine family of compounds

Mouse tolerability experiments were performed with selected compounds of the disclosure. Female CD-1 mice (n=5 mice per group) were treated with a single intravenous 1mg/kg dose of compound. Vehicle was PBS pH 6.0. Mice were carefully monitored for mortality, external clinical findings, weight loss, behavior and apparent signs of toxicity over the course of 21 days.

Table 4 below shows the average weight change over time and animal deaths for each individual compound. Some compounds of the disclosure were comparatively better tolerated than others, potentially supporting an improved therapeutic index.

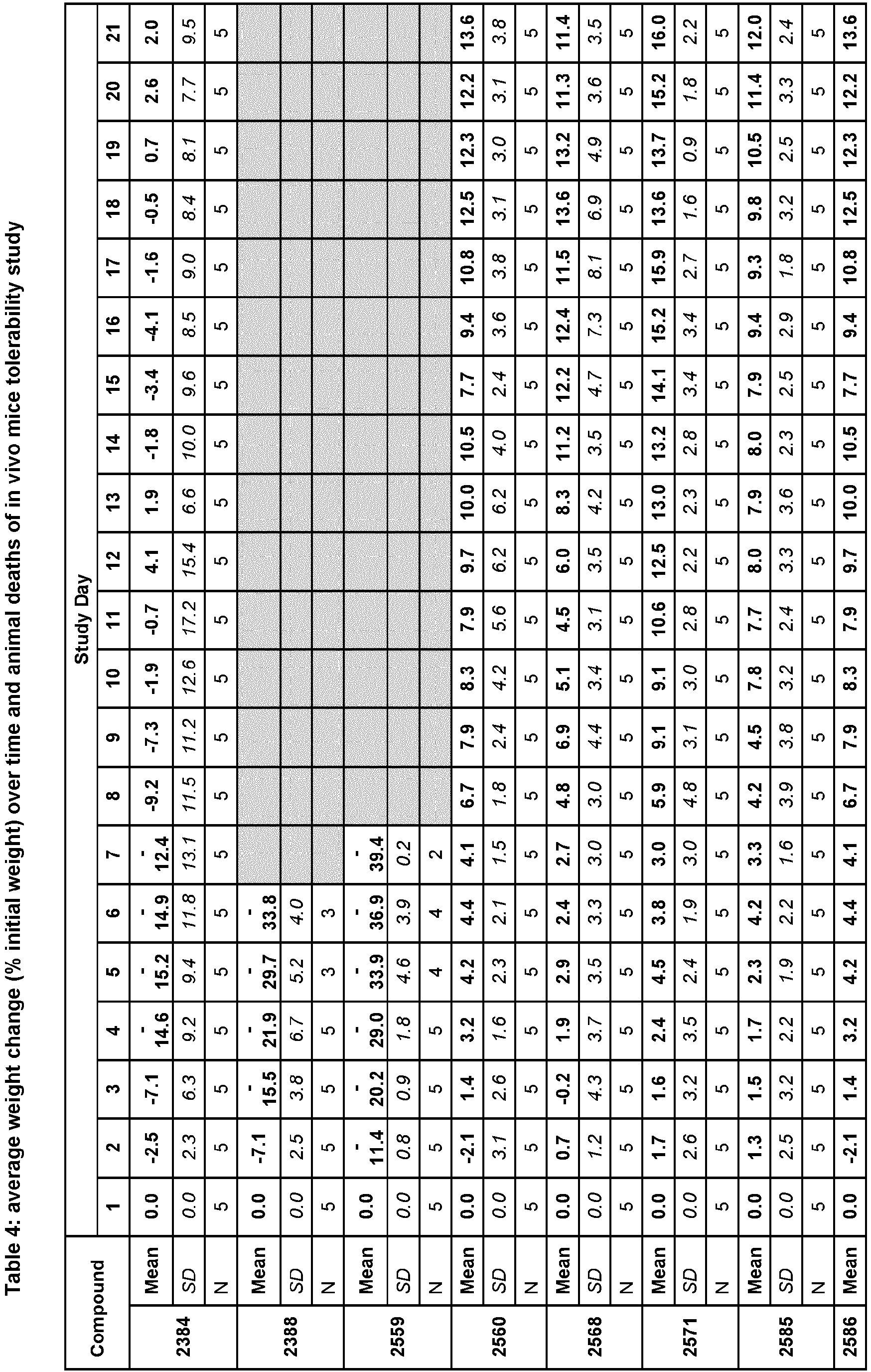

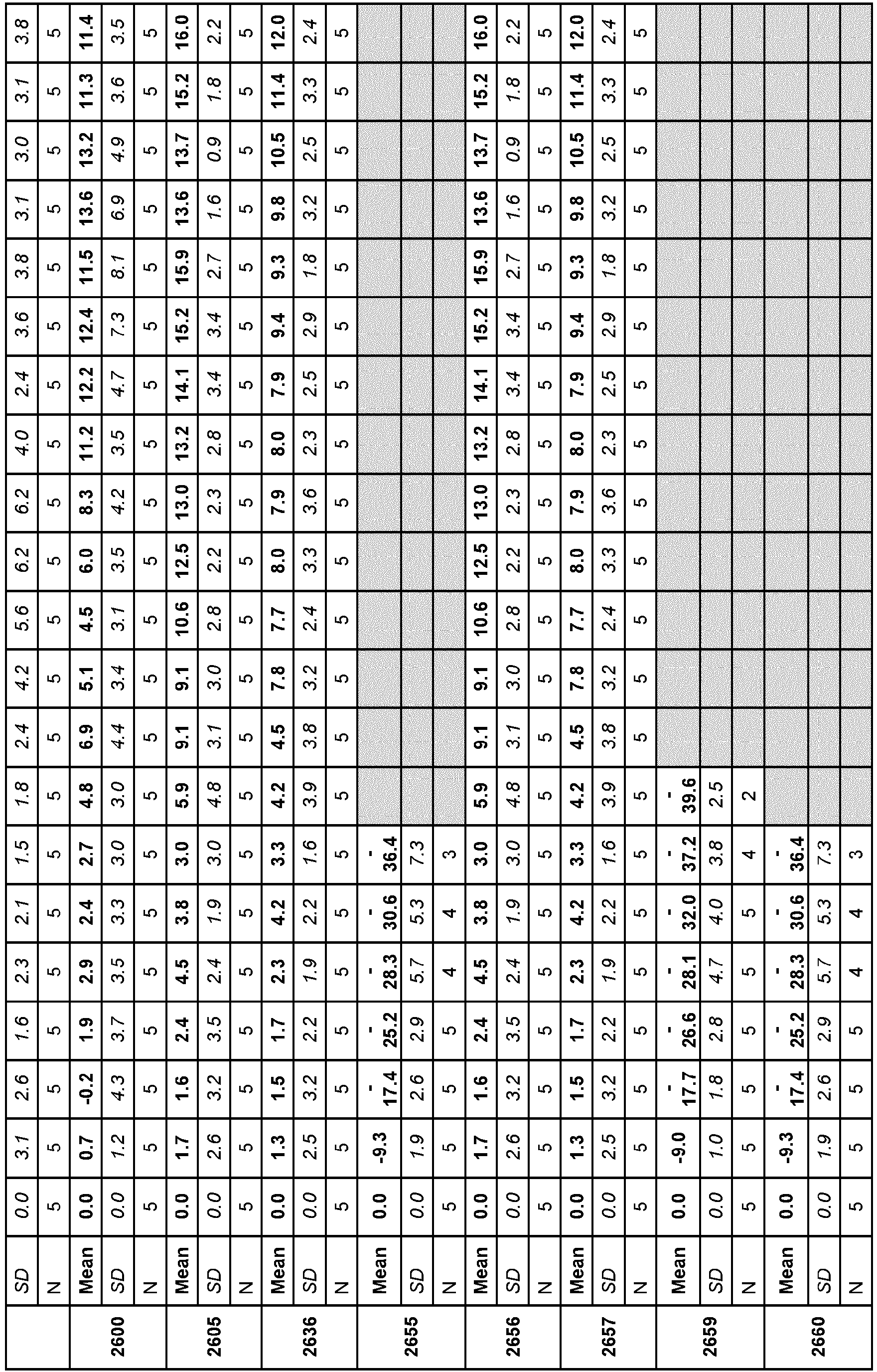

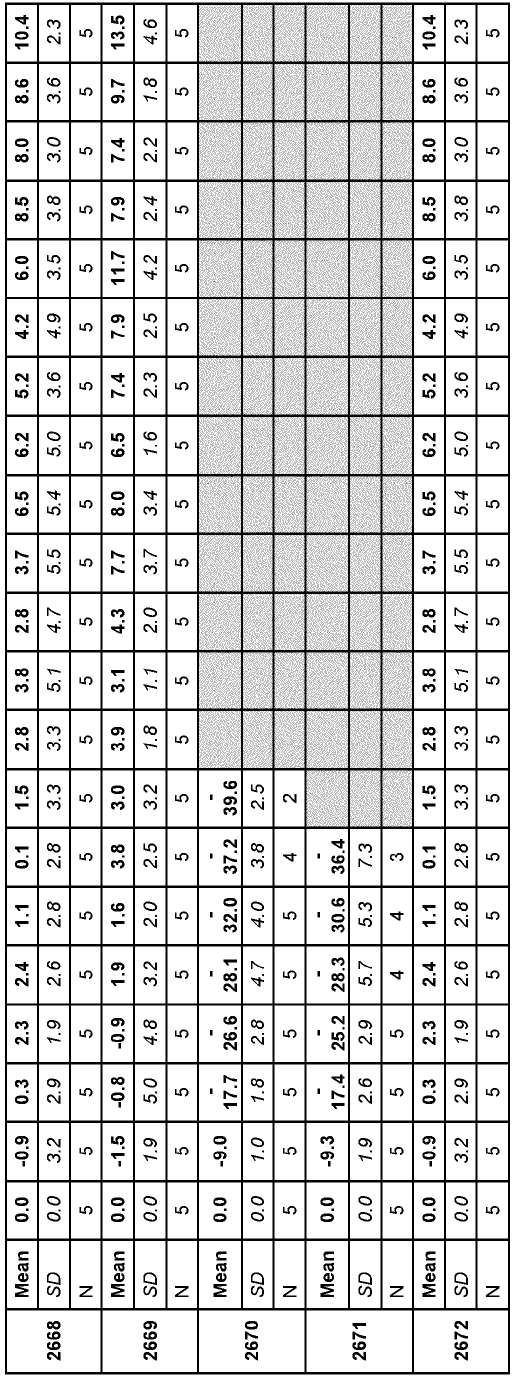

### Example 7: kinase screening assays

Compound 2384 according to the disclosure was subjected to kinase screening assays (performed at Reaction Biology, Germany). The screening assay was performed at a final compound concentration of 10 µM. A radiometric protein kinase assay (33PanQinase^{®} ActivityAssay) was used for measuring the kinase activity of 340 protein kinases. All kinase assays were performed in 96-well FlashPlates^{™} from Perkin Elmer (Boston, MA, USA) in a 50 µL reaction volume. The reaction cocktail was pipetted in 4 steps in the following order: (1) 10 µL of non-radioactive ATP solution (in H₂O); (2) 25 µL of assay buffer/ [γ- 33P]-ATP mixture; (3) 5 µL of test sample in 10% DMSO and (4) 10 µL of enzyme/substrate mixture The assay for all protein kinases contained 70 mM HEPES-NaOH pH 7.5, 3 mM MgCl2, 3 mM MnCl2, 3 µM Na-orthovanadate, 1.2 mM DTT, 50 µg/ml PEG₂₀₀₀₀, ATP (variable amounts, corresponding to the apparent ATP-Km of the respective kinase), [γ- 33P]-ATP (approx. 8 × 10⁵ cpm per well), protein kinase, and substrate. All PKC assays (except the PKC-mu and the PKC-nu assay) additionally contained 1 mM CaCl2, 4 mM EDTA, 5 µg/m! Phosphatidylserine and 1 µg/m! 1,2-Dioleyl-glycerol. The CAMK1D, CAMK2A, CAMK2B, CAMK2D, CAMK2G, CAMK4, CAMKK1, CAMKK2, DAPK2, EEF2K, MYLK, MYLK2 and MYLK3 assays additionally contained 1 µg/ml Calmodulin and 0.5 mM CaCl2. The PRKG1 and PRKG2 assays additionally contained 1 µM cGMP. The DNA-PK assay additionally contained 2.5 µg/ml DNA. The protein kinase reaction cocktails were incubated at 30°C for 60 minutes. The reaction was stopped with 50 µl of 2 % (v/v) H₃PO₄, plates were aspirated and washed two times with 200 µL 0.9 % (w/v) NaCl. Incorporation of 33Pi (counting of "cpm") was determined with a microplate scintillation counter (Microbeta, Wallac). All protein kinase assays were performed with a BeckmanCoulter Biomek 2000/SL robotic system. All protein kinases provided by RBE were expressed in Sf9 insect cells or in E.coli as recombinant GST-fusion proteins or His-tagged proteins, either as full-length or enzymatically active fragments. All kinases were produced from human cDNAs and purified by either GSH-affinity chromatography or immobilized metal. Affinity tags were removed from a number of kinases during purification. The purity of the protein kinases was examined by SDS-PAGE/Coomassie staining, the identity was checked by mass spectroscopy. Kinases from external vendors were expressed, purified and quality-controlled. Staurosporine was used as positive control.

An IC₅₀ profiling (titration from 0.1 nM to 10 000 nM) on top 25 protein kinases was also performed on the 2384 compound, following the same procedure. Staurosporine was used as positive control.

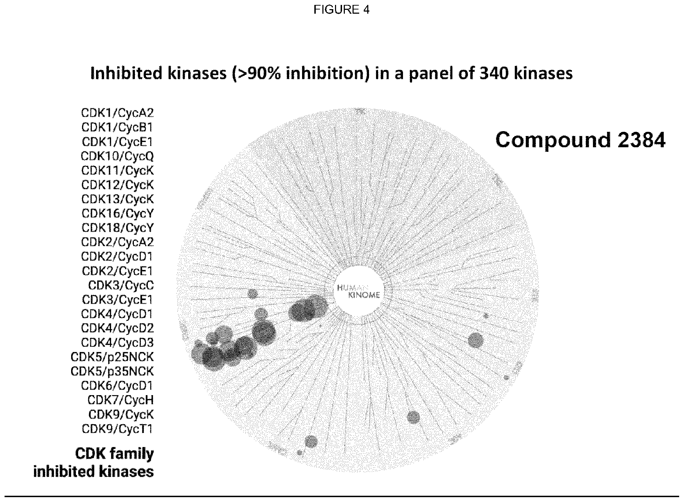

Figure 4 shows the result of the kinase screening assay of the 2384 compound according to the disclosure. Kinases inhibited at more than 90% are presented in the map as circles whose size is representative of percentage of inhibition. Inhibited CDKs at more than 90% are named in the lefthand side of the figure. Most inhibited kinases (more than 90 % inhibition) were found to belong to the CMGC kinase family, and particularly to the cyclin-dependent kinase (CDK) family, as pictured in the kinase map. Specifically, 23 out of the 30 most inhibited kinases were CDK/Cyclin complexes.

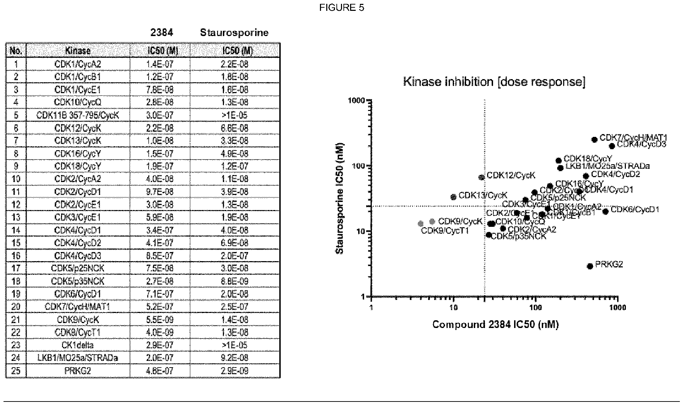

Figure 5 shows the dose-response inhibition on top 25 selected kinases. This allowed to identify CDK9/CycT1, CDK9/CycK, CDK13/CycK and CDK12/CycK as the main targets of compound 2384, with IC₅₀'s below 25 nM (bottom left quadrant).

### Example 8: in vitro cytotoxicity assays in presence of the NEDDylation inhibitor pevonedistat

In vitro cytotoxicity of compounds of the disclosure was assessed in breast cancer cell line BT-474, in presence or absence of the non-cytotoxic cullin NEDDylation inhibitor pevonedistat. NEDDylation is the process by which the ubiquitin-like protein NEDD8 drives degradation of the CycK protein by a process which is analogous to ubiquitination.

8000 cells were plated in 96-well plates and incubated at 37°C for 24 hours. Serial dilutions of the tested compound previously dissolved in culture media were added, and incubation was carried at 37°C out for 5 days in the presence or absence of 400nM of pevonedistat. MTT (5mg/mL in PBS, 20*µ*L, Sigma-Aldrich) was added into the wells, and incubation was continued for 1 to 4 hours at 37°C. Culture media was then carefully removed, and well content was homogeneously dissolved with acidified isopropanol. Absorbance values were measured on a microplate reader using a wavelength of 570 nm (with a reference wavelength of 690 nm). The IC₅₀ concentration values compared to untreated control cells were determined using inhibition dose response curve fitting (GraphPad Prism 9). Pevonedistat (cat#HY-70062) was purchased from MedChemExpress (China).

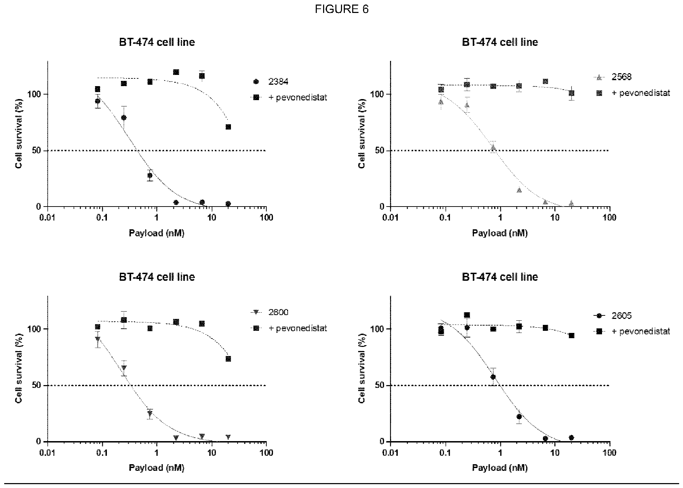

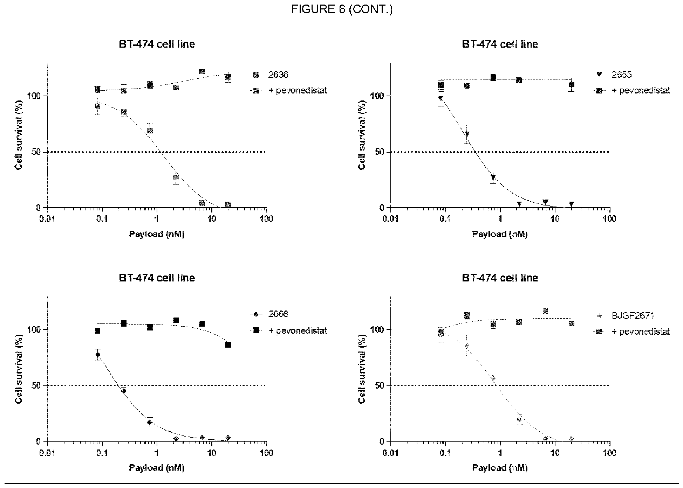

Figure 6 shows that the cell-killing property of compounds of the invention is strongly inhibited by pevonedistat, thereby confirming that main mechanism of action of cytotoxicity is molecular glue degradation of CycK.

### Example 9: in vitro mechanistic studies of compound 2384 of the invention

### Protein quantification by Western Blot

1×10⁶ JIMT-1 cells were plated in 3mL of DMEM/FBS 90:10 (v/v) in 6-well plates and incubated overnight. Cells were treated with the compound 2384 of the invention with or without the proteasome inhibitor MG-132 (MedChemExpress, Cat HY-13259) for 2h. Cells were harvested, and protein were extracted using complete RIPA buffer (RIPA buffer, 1 mM DTT, 1M NaF, 100 mM sodium orthovanadate, phosphatase inhibitor buffer and protease inhibitors) for 1 hour on ice. Lysate were centrifuged 15min at 12 000g at 4°C. and proteins were mixed with 4x Laemmli Sample Buffer (Bio-Rad), heated at 95 °C for 5 mins and separated by SDS PAGE (PROTEAN TGX Stain-Free Gels, Bio-Rad). Proteins were then transferred onto PVDF membrane (iBlot^{®}2 PVDF stacks, Invitrogen) using the iBlot2 device (Invitrogen) and blocked with TBS buffer (Intercept^{®} Blocking Buffer, LI-COR Biosciences). Primary antibodies were incubated overnight at 4°C and secondary antibodies (IRDye Infrared Dyes from LI-COR Biosciences) 1 hour at room temperature. Used primary antibody were: anti-CycK (cat#A301-939A, Bethyl Laboratories, dil. 1:1000) and anti-β-actin (cat#A5441, Sigma-Aldrich, dil. 1:5000). Membranes were scanned using a c500 Imaging System (Azure Biosystems) and densitometric quantification were performed using imaged software. Protein levels were normalized against β-actin.

### RT-qPCR

1×10⁶ JIMT-1 cells were plated in 3mL of DMEM/FBS 90:10 (v/v) in 6-well plates and incubated overnight. Cells were treated with the compound 2384 of the invention, with CR8 (a known molecular glue degrader that depletes CycK) or with the proteasome inhibitor MG-132 (MedChemExpress, Cat HY-13259) for 2h. RNA was extracted using the QIAamp RNeasy Mini Kit (cat#74106, Qiagen) according to the manufacturer's protocol. Reverse transcription was performed using SuperScripr^{™} IV reverse transcriptase (cat#LT02241, Invitrogen). Quantitative PCR was performed using a No ROX Sybr Master Mix in the LightCycler 480 Real-Time PCR system (Roche Life Science).

Cyclin K forward (CACTATGATACCCTGGCAACTGG) and reverse (CAGAAAGAGGCAACAGGCTCCT) primers were purchased from Eurogentec. Gene expression was normalized using ribosomal 28S as the housekeeping gene.

### Cell cycle arrest assessment

1×10⁶ JIMT-1 cells were plated in 3mL of DMEM/FBS 90:10 (v/v) in 6-well plates and incubated overnight. Cells were treated with the compound 2384 of the invention or with CR8 for 24h. Cells were then harvested and washed three times with PBS. Cells were suspended into 300µL of propidium iodide working solution (0.05mg/mL) and incubated for 45 minutes at 4°C in the dark. Analysis was performed using a BD Fortessa flow cytometer controlled by BD FACSDiva software (BD Biosciences) and data were analyzed using FlowJo software (BD Bioscience).

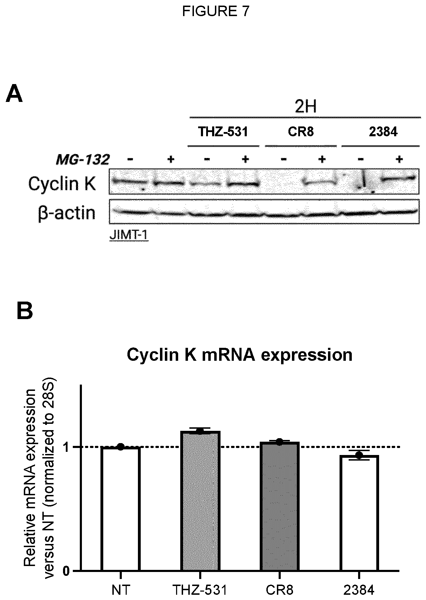

Figure 7A and 7B below shows that exposure of cells to compound 2384 of the invention or known comparator CycK molecular glue degrader (1µM final concentration) led to decreased CycK protein content (Figure 7A) but not at the CycK mRNA level (Figure 7B). THZ-531, a CDK12 inhibitor, had no impact on CycK protein content at this 1µM concentration (Figure 7A). Combination with the proteasome inhibitor MG-132 restored the content in cycK protein (Figure 7A).

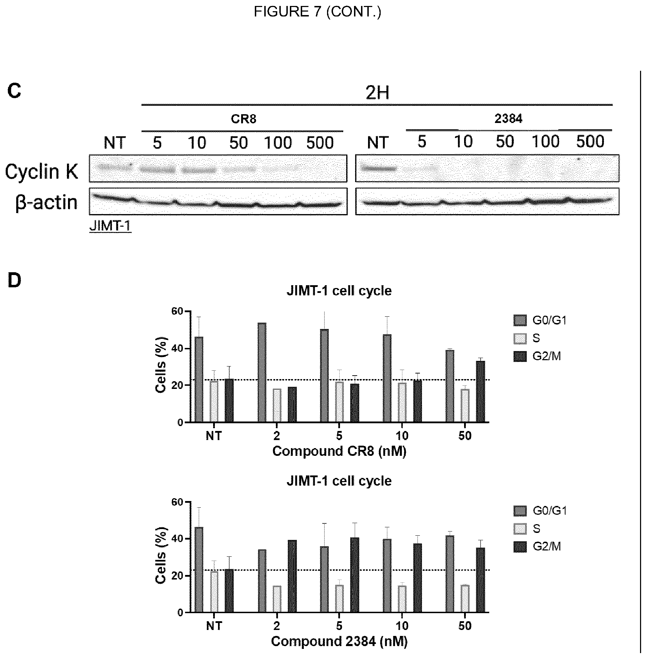

CycK degradation was also assessed using decreasing doses of either compounds 2384 of the invention or CR8 comparator (Figure 7C). 2h treatment of JIMT-1 cells with 10nM compound 2384 induced complete CycK degradation whereas no CycK was observed using CR8 at this dose. Complete CycK degradation by CR8 required concentrations between 100 and 500nM, showing that the 2384 minimum effective dose for CycK degradation is at approximately 10-times lower than that of CR8, thus explaining, its superior in vitro cytotoxicity.

Cell cycle analysis of cells using propidium iodide (PI) demonstrated an arrest in the G2/M phase upon compounds 2384 treatment for 24h at low and high doses (2, 5, 10, and 50nM), while CR8 treatment at 2, 5, and 10 nM had no impact on cell cycle (Figure 7D). Treatment with CR8 at 50 nM was required to induce an arrest in G2/M phase. This observation again contributes to explain the superior cytotoxicity of compound 2384 compared to that of CR8.

These examples above show that the compounds of the disclosure act as molecular glue degraders of CycK.

### Example 10: passive permeability assessment (PAMPA assay)

Parallel artificial membrane permeability assay (PAMPA) studies were conducted using the PAMPA Explorer kit (pION Inc., USA) and the double sink protocol (Avdeef et al., 2005). Stock solutions of compounds of the invention were prepared in dimethyl sulfoxide (DMSO) to a final concentration of 10 mM. Each stock solution was diluted to 50 µM in pH 7.4 Prisma HT buffer (pION) in the presence of 10% MeOH (v/v). 200 µL were added to each well of the donor plate in quintuplicate. The polyvinylidene fluoride (PVDF, 0.45 µM) filter membrane on the acceptor plate was coated with 5 µL of the gastrointestinal tract lipid formulation (GIT-0, pION) and to each well of the acceptor plate, 200 µL of acceptor sink buffer (ASB, pION), supplemented with 10% (v/v) MeOH, was added. The acceptor filter plate was carefully placed on top of the donor plate to form a "sandwich." The sandwich was incubated at 25°C for 5 h, at 300 rpm. UV-vis spectra of the solutions in the blank, reference, acceptor, and donor plates were measured using a Tecan Infinite 200 PRO M Nano Plus microplate reader. For each compound, apparent permeability values Pₐₚₚ (10⁻⁶ cm/s) were calculated using the PAMPA Explorer software v.3.6 (pION). Ketoprofen was used as negative impermeable control and verapamil was used as positive permeable control.

Table 5 below shows the apparent permeability values Pₐₚₚ (10⁻⁶ cm/s) of tested compounds of the disclosure. All compounds exhibited high passive permeabilities values (higher than exatecan and DXd, two ADC payloads known to have good passive permeability properties and good "bystander effect"), enabling efficient "bystander effect" (a desirable property to treat heterogeneous solid tumors with different levels of target expression).

**Table 5: apparent permeability values Pₐₚₚ (10⁻⁶ cm/s) of compounds of the invention**

| **Compound** | **Pₐₚₚ (10⁻⁶ cm/s)** | **Standard error** | **"Bystander effect" potential** |
|---|---|---|---|
| **2384** | 84 | 5 | +++ |
| **2560** | 135 | 18 | +++ |
| **2568** | 88 | 16 | +++ |
| **2571** | 149 | 13 | ++++ |
| **2585** | <0.3 | N/A | - |
| **2600** | 90 | 4 | +++ |
| **2605** | 112 | 6 | +++ |
| **2636** | 67 | 4 | +++ |
| **2655** | 125 | 5 | +++ |
| **2668** | 164 | 7 | ++++ |
| **2670** | 132 | 6 | +++ |
| **2671** | 176 | 2 | ++++ |
| **DXd** | 13 | 0.3 | ++ |
| **Exatecan** | 58 | 5 | +++ |
| **Verapamil** | 141 | 9 | N/A |
| **Ketoprofen** | 0.4 | 0.1 | N/A |

### Example 11: MDR1/BCRP substrate

MDR1 and BCRP efflux substrate assays were conducted in MDCK-MDR1 and MDCK-BCRP in vitro models (Preadyport^{™}, Solvo Biotech) at Symeres (Nederland). Compound of the invention (donor well concentration of 5µM) were incubated in cell monolayer 24-transwell Preadyport system, using Preadyport recommended pH 7.4 assay buffer (donor and receiver wells). After 60 min of incubation (no shaking), samples are collected and suspended into triple volume of 66% cold acetonitrile. Samples are stored at -20°C until thawed, centrifuged and analyzed by LC-MS. Quinidine was used a positive control for the MDR1 assay, and prazosin was used as a positive control for the BCRP assay. Exatecan was used as a control, known not to be an MDR1 substrate. Residual DMSO content from compounds stock solutions was below 1% (v/v). The assay was performed in duplicate. The quantification of test compounds in donor or receptor compartment is conducted using relative LC/MS peak areas (samples of initial incubation solutions are used as a single point calibrator), without any spiked standard samples. The data is used for estimating apparent permeability (Pₐₚₚ) and calculation of efflux ratio.

Table 6 below shows that compounds of the invention exhibited different levels of MDR1 efflux pump susceptibility. MDR1 is protein of the tumor cell membrane that pumps the anticancer drugs out of cancer cells, thereby rendering the cell drug-resistant. The Pₐₚₚ (10⁻⁶ cm/s) B (acceptor = basolateral) to A (donor = apical) reflects the susceptibility of a compounds to be pumped out of a tumor and as such a low Pₐₚₚ B→A value is advantageous in the context of oncology. The efflux ratio B/A reflects the overall ability of a compounds to enter MDR1-expressing tumor cells (equilibrium between ability to passively enter the cell A→B and susceptibility to be pumped out of a tumor B→A).

Table 7 below shows that compounds of the invention exhibited different levels of BCRP efflux pump susceptibility. BCRP, like MDR1, is a protein of the tumor cell membrane that pumps the anticancer drugs out of cancer cells, thereby rendering the cell drug-resistant. This multidrug-resistant protein is particularly expressed in breast and lung cancers. The efflux ratio B/A reflects the overall ability of a compounds to enter BCRP-expressing tumor cells (equilibrium between ability to passively enter the cell A→B and susceptibility to be pumped out of a tumor B→A).

This example shows that compounds of the invention exhibited different levels of MDR1 and BCRP efflux potential. As an example, compounds 2655, 2600 and 2605 show low level of MDR1 and BCRP efflux potential and as such could be especially well suited to tackle multidrug-resistant tumor types (treat patients that have acquired resistance to first/second lines of chemotherapy).

**Table 6: apparent MDR1 permeability values Pₐₚₚ (10⁻⁶ cm/s) and efflux ratio of compounds of the invention**

| **Compound** | **Pₐₚₚ (10⁻⁶ cm/s) A (donor) to B (acceptor)** | **Pₐₚₚ (10⁻⁶ cm/s) B (acceptor) to A (donor)** | **Efflux ratio B/A** |
|---|---|---|---|
| **2655** | 5.13 | 26.9 | 5.25 |
| **Exatecan** | 2.28 | 16.7 | 7.31 |
| **2605** | 4.05 | 54.5 | 13.44 |
| **2600** | 2.05 | 59.3 | 28.86 |
| **2668** | 1.36 | 56.4 | 41.60 |
| **2671** | 0.99 | 43.2 | 43.68 |
| **2670** | < 0.50* | 23.5 | N/A |
| **2560** | < 0.70* | 26.6 | N/A |
| **2571** | 0.59 | 64.2 | 108.24 |
| **2636** | < 12.9* | 30.8 | N/A |
| **2585** | < 3.4* | 34.0 | N/A |
| **2384** | < 0.25* | 36.9 | N/A |
| **2568** | < 0.54* | 40.5 | N/A |

| | | | |
|---|---|---|---|
| **based on LC-MS quantification method lower limit of quantification (LLOQ)* | | | |

**Table 7: apparent BCRP permeability values Pₐₚₚ (10⁻⁶ cm/s) and efflux ratio of compounds of the invention**

| **Compound** | **Pₐₚₚ (10⁻⁶ cm/s) A (donor) to B (acceptor)** | **Pₐₚₚ (10⁻⁶ cm/s) B (acceptor) to A (donor)** | **Efflux ratio B/A** |
|---|---|---|---|
| **2605** | 30.9 | 40.5 | 1.31 |
| **2600** | 22.4 | 47.1 | 2.10 |
| **2655** | 11.1 | 38.7 | 3.47 |
| **2671** | 10.1 | 46.0 | **4.54** |
| **2560** | 7.53 | 37.2 | 4.94 |
| **2670** | 5.02 | 29.8 | 5.93 |
| **2636** | 3.00 | 18.6 | 6.21 |
| **2668** | 10.2 | 71.0 | 6.99 |
| **2384** | 5.49 | 50.7 | **9.24** |
| **Exatecan** | 1.43 | 17.6 | 12.3 |
| **2571** | 7.09 | 91.4 | 12.9 |
| **2568** | 2.52 | 59.5 | 23.6 |
| **2585** | 1.75 | 49.0 | 27.9 |

### Example 12: In vitro cytotoxicity assay of antibody-drug conjugates based on the 2-substituted 8-halogeno-N4-(4-(pyridin-2-yl) benzyl) pyrazolo[1,5-a][1,3,5] triazine-2,4-diamine family of compounds

In vitro cytotoxicity of antibody drug conjugates (ADC) based on of the 2-substituted 8-halogeno-N4-(4-(pyridin-2-yl) benzyl) pyrazolo[1,5-a][1,3,5] triazine-2,4-diamine family of compounds of the disclosure were performed on several cancerous human cell lines, following the procedure described above in example 5.

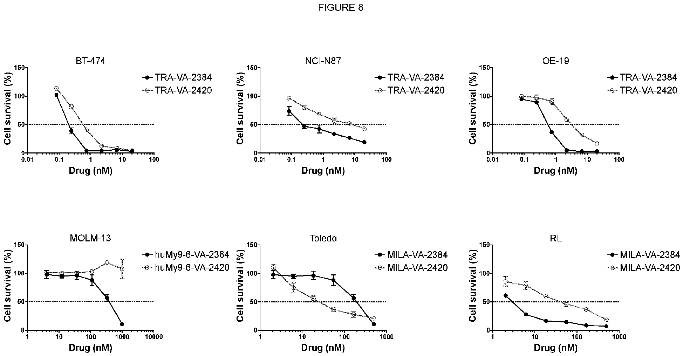

Figure 8 shows side-by-side comparison of antibody drug conjugates based on the lead compound 2384 of the disclosure to antibody drug conjugates based on the isopropyl counterpart compound 2420. ADCs based on payload 2384 are systematically more potent compared to ADCs based on payload 2420.

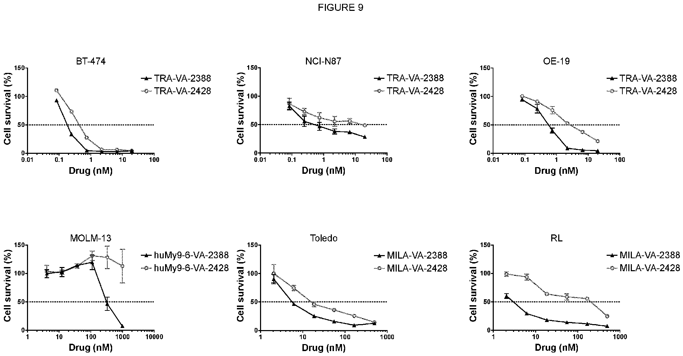

Figure 9 shows side-by-side comparison of antibody drug conjugates based on the lead compound 2388 of the disclosure to antibody drug conjugates based on the isopropyl counterpart compound 2428. ADCs based on payload 2388 are systematically more potent compared to ADCs based on payload 2428.

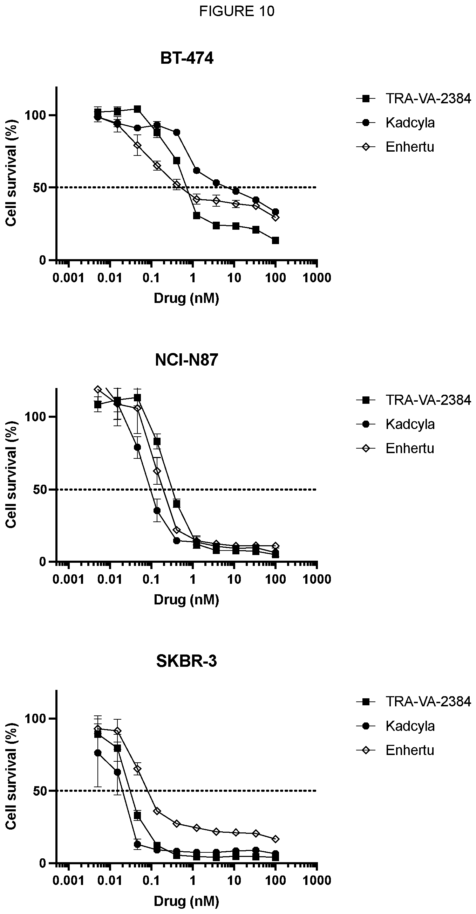

Figure 10 shows side-by-side comparison of trastuzumab-based ADC incorporating the lead compound 2384 of the disclosure (TRA-VA-2384) and trastuzumab-based commercially approved ADCs (Kadcyla^{®} and Enhertu^{®}). TRA-VA-2384 is either more or similarly potent compared to the commercially approved ADCs bearing two different payloads with two different mechanisms of action (tubulin inhibitor for Kadcyla^{®}, topoisomerase I inhibitor for Enhertu^{®}). This shows that compounds of the disclosure are potent enough to generate efficacious ADCs, while proposing a new mechanism of action for cell killing.

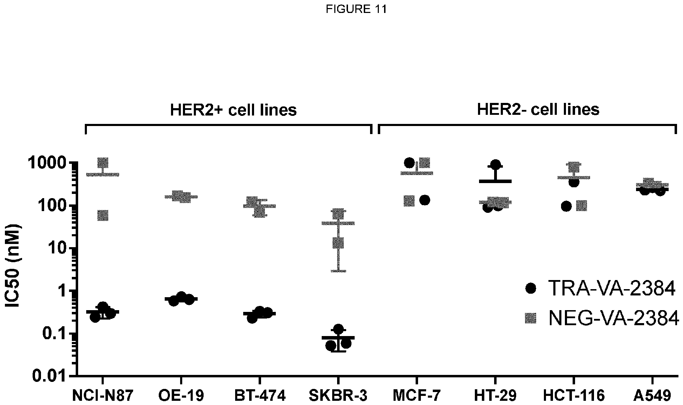

Figure 11 shows trastuzumab (anti-HER2)-based ADC incorporating the lead compound 2384 of the disclosure (TRA-VA-2384) cell killing efficacy, compared to a non-binding isotype control-based ADC (NEG-VA-2384). Those results show that the cell killing activity of the TRA-VA-2384 ADC is target-specific (HER2-specific).

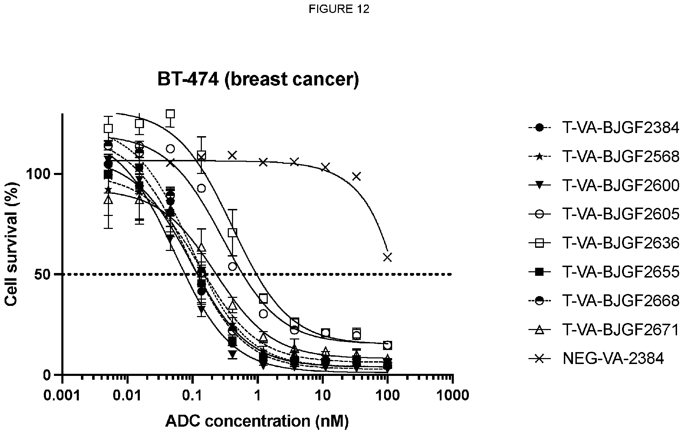

Figure 12 shows trastuzumab (anti-HER2)-based ADCs incorporating lead compounds of the invention cell killing efficacy (in breast cancer model BT-474). Those results show that ADCs based on the compounds of the invention have high level of potency (low nanomolar range).

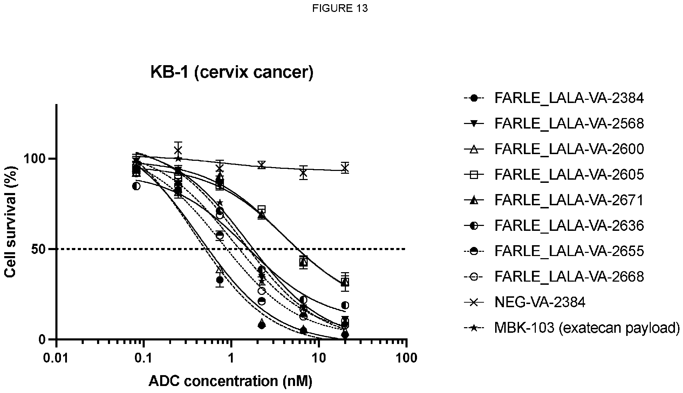

Figure 13 shows farletuzumab_LALA (anti-FRα)-based ADCs incorporating lead compounds of the invention cell killing efficacy (in cervical cancer model KB-1). Those results show that ADCs based on the compounds of the invention have high level of potency (low nanomolar range) and outperform previously known ADC MBK-103 (positive control analogous ADC comprising exatecan as payload instead of compound of the invention - MBK-103 structure has been disclosed in PCT/EP2023/056097).

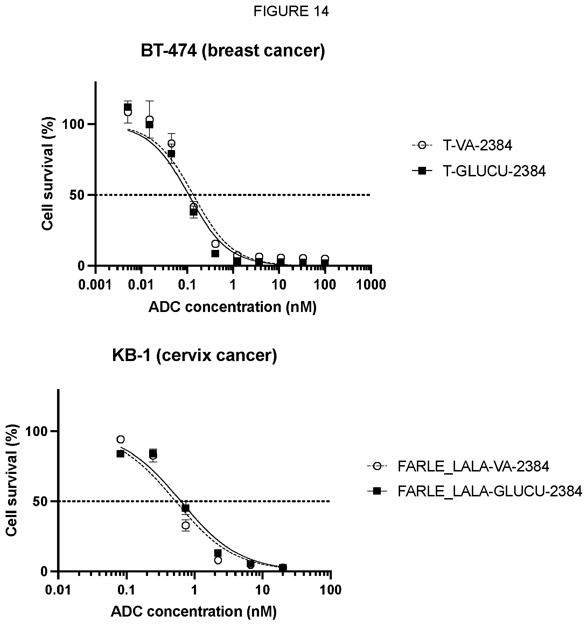

Figure 14 shows that trastuzumab (anti-HER2) and farletuzumab_LALA (anti-FRa)-based ADCs incorporating compound 2384 of the invention and based either on a dipeptide (Val-Ala) or glucuronidase sensitive cleavable linker moiety shows comparable low nanomolar efficacy results. Comparable cell killing efficacies were observed, showing that payloads of the invention can be paired with different cleavable modalities without affecting efficacy of the ADC.

### Example 13: In vitro bystander cell killing assay of antibody-drug conjugates based on the 2-substituted 8-halogeno-N4-(4-(pyridin-2-yl) benzyl) pyrazolo[1,5-a][1,3,5] triazine-2,4-diamine family of compounds

Capacity of trastuzumab (anti-HER2)-based ADC incorporating the lead compound 2384 to exert in vitro bystander killing properties was assessed in a co-culture cell killing assay. BT-474 (HER2-positive) and MDA-MB-231 (HER2-negative) cells were seeded in single or co-culture (2:1 ratio respectively) in 96-well plate at final density of 10 000 cells per well and incubated overnight at 37 °C under a 5% CO₂ atmosphere. Cells were then treated with 25 nM of Enhertu^{®} (positive control ADC know to exert bystander cell killing) or TRA-VA-2384 for 6 days. Cells were then collected by trypsinization and transferred into a round-bottom 96-well plate suitable for high-throughput flow cytometry. Cells were washed three times with PBS, resuspended in 50 µL PBS, and incubated with an anti-HER2 APC-conjugated antibody (BD Bioscience, Cat#340554) for 30 min at 4°C in the dark. Analysis was performed using a BD Fortessa flow cytometer controlled by BD FACSDiva software (BD Bioscience) and data were analyzed using FlowJo software (BD Bioscience).

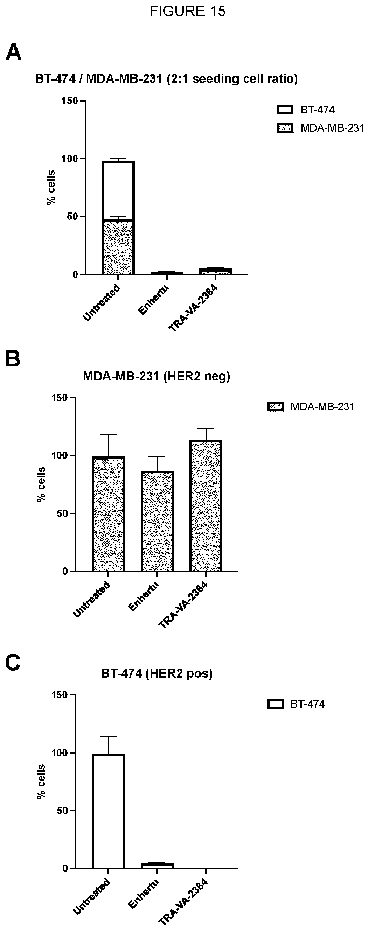

Figure 15A shows that TRA-VA-2384 ADC can efficiently kill both the BT-474 HER2-positive and the MDA-MB-231 HER2-negative cell populations *via* a bystander cell killing. Enhertu is here used as a positive control ADC also able to exert bystander cell killing. Figure 15B is the control experiment showcasing the fact that both TRA-VA-2384 and Enhertu's ADCs are not non-specifically cytotoxic by themselves for the MDA-MB-231 HER2-negative cell line. Figure 15C shows that both ADCs are cytotoxic to the BT-474 HER2-positive cell line.

Those results show that once released into antigen-positive cells (after ADC internalization and digestion), payloads of the disclosure can exert bystander killing effect by passive diffusion into antigen-negative neighboring cells. This property is especially desirable to treat heterogeneous solid tumors.

### Example 14: In vivo efficacy of antibody-drug conjugates based on the 2-substituted 8-halogeno-N4-(4-(pyridin-2-yl) benzyl) pyrazolo[1,5-a][1,3,5] triazine-2,4-diamine family of compounds in cancer xenograft models

Four to five-week-old female CB-17 severe combined immunodeficient (SCID) mice are obtained and quarantined for 7 days prior to study initiation. Mice are inoculated subcutaneously with cells (5-10 × 10⁶ cells per flank) resuspended in PBS. When average tumor volume reached about 120-150 mm3 mice are randomized (typically 6 mice per group) and treated by a single (unless stated otherwise) intravenous injection of PBS (negative control) or investigated antibody-drug conjugate. Tumor volumes are measured every 3-5 days using a caliper device (length × width) and calculated using the following formula V = 4/3 × π × R³, where R represents the radius. Mice are sacrificed when the tumor volume exceeded 1500 mm³ or in case of ulceration of the tumor.

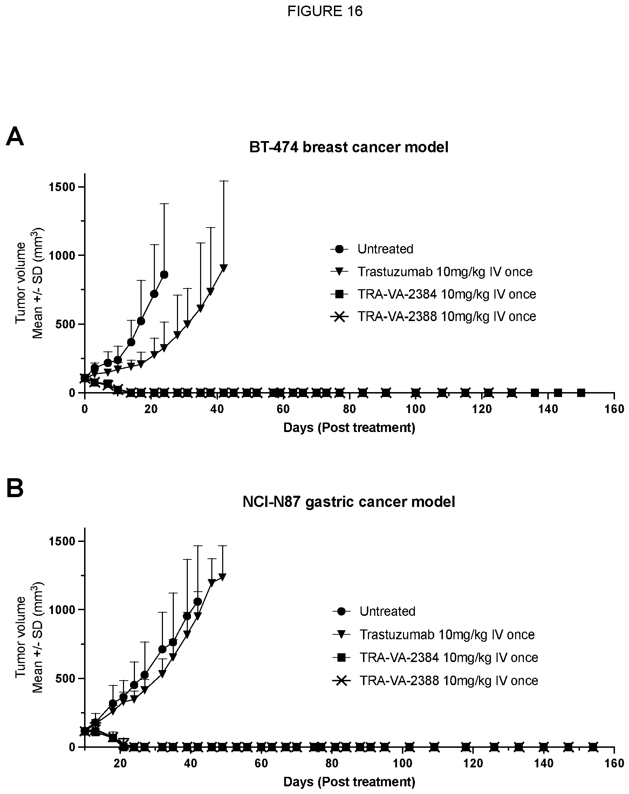

Figure 16A shows a tumor xenograft experiment in the BT-474 HER2+ breast cancer model. Naked trastuzumab, TRA-VA-2384 and TRA-VA-2388 were injected IV once at 10 mg/kg. TRA-VA-2384 and TRA-VA-2388 were highly efficacious and led to complete tumor regression in all animals. At the same dose, naked trastuzumab showed very slight antitumor efficacy.

Figure 16B shows a tumor xenograft experiment in the NCI-N87 HER2+ gastric cancer model. Naked trastuzumab, TRA-VA-2384 and TRA-VA-2388 were injected IV once at 10 mg/kg. TRA-VA-2384 and TRA-VA-2388 were highly efficacious and led to complete tumor regression in all animals. Naked trastuzumab showed no antitumor efficacy at the same dose.

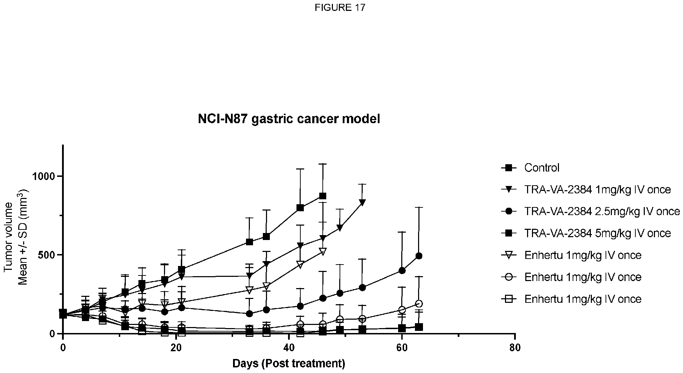

Figure 17 shows a dose-response tumor xenograft experiment in the NCI-N87 HER2+ breast cancer model. TRA-VA-2384 and Enhertu were dosed IV once at 1, 2.5 or 5mg/kg. Similar dose-dependent efficacies were observed between both ADCs.

Those results show that ADCs based on payloads of the invention exhibit excellent in vivo efficacy.

### Example 15: in vivo mouse tolerability of antibody-drug conjugates based on the 2-substituted 8-halogeno-N4-(4-(pyridin-2-yl) benzyl) pyrazolo[1,5-a][1,3,5] triazine-2,4-diamine family of compounds

Mouse tolerability experiments were performed with selected antibody-drug conjugates based on the payloads of the disclosure. Female SCID mice (n=5 mice per group) were treated with a single intravenous 20, 50 or 100mg/kg dose of ADC TRA-VA-2384 or ADC TRA-VA-2388. Vehicle was PBS. Mice were carefully monitored for mortality, external clinical findings, weight loss, behavior and apparent signs of toxicity over the course of 14 days.

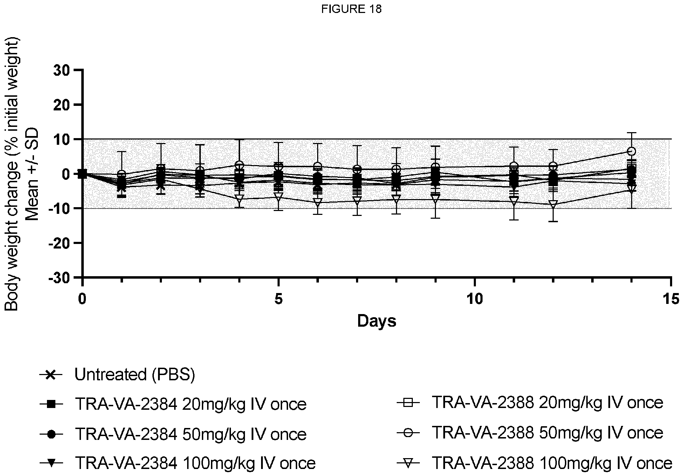

Figure 18 shows that both TRA-VA-2384 and TRA-VA-2388 ADCs are well tolerated at doses up to 100mg/kg.

Taken collectively, results of example 14 and 15 shows that ADCs based on payloads of the invention exhibit a favorable preclinical rodent therapeutic window.

## Claims

1. An antibody-drug conjugate of formula (I):
Ab-[L-D]p (I),
wherein :
Ab is an antibody or an antibody fragment,
L is a cleavable linker moiety bonded to Ab and D,
p is 1 or more, preferably from 1 to 8
D is a cytotoxic drug moiety of formula (II) bonded to L, 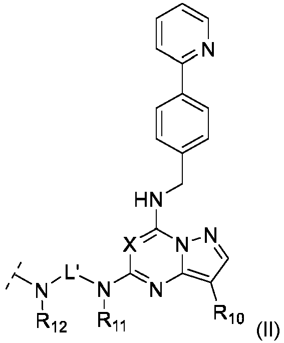 wherein
X is C or N, preferably N;
R10 is selected from the group consisting of H, Halogen, C₁-C₁₂ alkyl, C₁-C₁₂ haloalkyl, C₁-C₁₂ heteroalkyl, aryl having 6 to 10 ring atoms, C₃-C₈ cycloalkyl, heterocycloalkyl having 3 to 10 ring atoms, heteroaryl having 5 to 10 ring atoms, and combination thereof, said alkyl, heteroalkyl, aryl, cycloalkyl, heteroaryl and heterocycloalkyl being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl, preferably R10 is a halogen or a C₁-C₁₂ alkyl;
R11 is selected from the group consisting of H and C₁-C₆ alkyl, said alkyl being optionally substituted;
Or R11 is linked to an atom from the linker L' and forms a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl;
L' is a bond or a linker, preferably selected from C₁-C₂₀ alkylene, C₁-C₂₀ heteroalkylene, C₃-C₁₂ cycloalkylene, heterocyclylene having 5 to 10 ring atoms, arylene having 6 to 10 ring atoms, heteroarylene having 5 to 10 ring atoms, and any combination thereof; said alkylene, heteroalkylene, arylene, cycloalkylene, heteroarylene and heterocycloalkylene being optionally substituted, preferably by one or more substituent selected from the group consisting of -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, and -NR'R"; R', and R" being independently selected from H and C₁-C₆ alkyl;
R12 is selected from the group consisting of H, and C₁-C₁₂ alkyl;
Or R12 is linked to an atom from the linker L' and forms a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl;
Or R11 and R12, together with the N atoms and the linker L' to which they are bonded, form a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl.

2. The antibody-drug conjugate according to claim 1, wherein D is a moiety of formula (III) 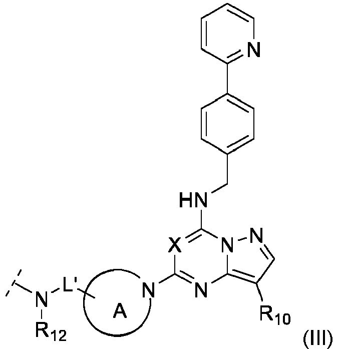 wherein
X is C or N, preferably N;
Ring A is a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl,
L' is a bond or a linker, preferably selected from C₁-C₁₂ alkylene, C₁-C₁₂ heteroalkylene, C₃-C₆ cycloalkylene, heterocyclylene having 5 to 10 ring atoms, arylene having 6 to 10 ring atoms, heteroarylene having 5 to 10 ring atoms, and any combination thereof, and R12 is selected from the group consisting of H, and C₁-C₁₂ alkyl.

3. The antibody-drug conjugate according to claim 1, wherein D is a moiety of formula (IV) 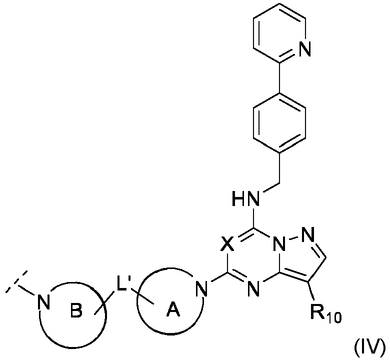 Wherein
X is C or N, preferably N;
Ring A is a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl; and
Ring B is a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl, and
L' is a bond or a linker, preferably selected from C₁-C₁₂ alkylene, C₁-C₁₂ heteroalkylene, C₃-C₆ cycloalkylene, heterocyclylene having 5 to 10 ring atoms, arylene having 6 to 10 ring atoms, heteroarylene having 5 to 10 ring atoms, and any combination thereof.

4. The antibody-drug conjugate according to claim 1, wherein D is a moiety of formula (V) 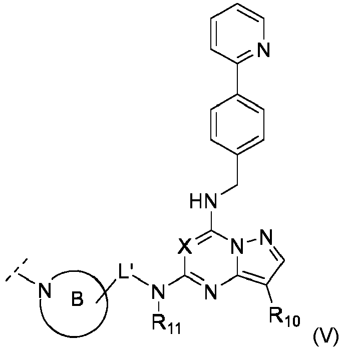 Wherein
X is C or N, preferably N;
R11 is selected from the group consisting of H, and C₁-C₁₂ alkyl,
L' is a bond or a linker, preferably selected from C₁-C₁₂ alkylene, C₁-C₁₂ heteroalkylene, C₃-C₆ cycloalkylene, heterocyclylene having 5 to 10 ring atoms, arylene having 6 to 10 ring atoms, heteroarylene having 5 to 10 ring atoms, and any combination thereof, and Ring B is a heterocycloalkyl having 3 to 10 ring atoms, or a heteroaryl having 5 to 10 ring atoms, said heterocycloalkyl and heteroaryl being optionally substituted, preferably by one or more substituent selected from -OH, =O, C₁-C₁₂ alkyl, C₁-C₁₂ heteroalkyl, C₃-C₆ cycloalkyl, heterocyclyl having 5 to 10 ring atoms, aryl having 6 to 10 ring atoms, heteroaryl having 5 to 10 ring atoms, -(C₁-C₁₂ alkyl)-(C₃-C₈ cycloalkyl), -(C₁-C₁₂ alkyl)-(heterocycloalkyl having 3 to 10 ring atoms), -(C₁-C₁₂ alkyl)-(heteroaryl having 5 to 10 ring atoms), -(C₁-C₁₂ alkyl)-(aryl having 6 to 10 ring atoms), and -NR'R"; R', and R" being independently selected from H and C₁-C₆ alkyl.

5. The antibody-drug conjugate of claim 1, wherein D is selected from

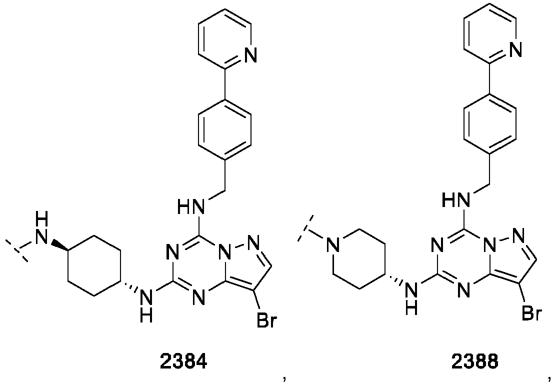

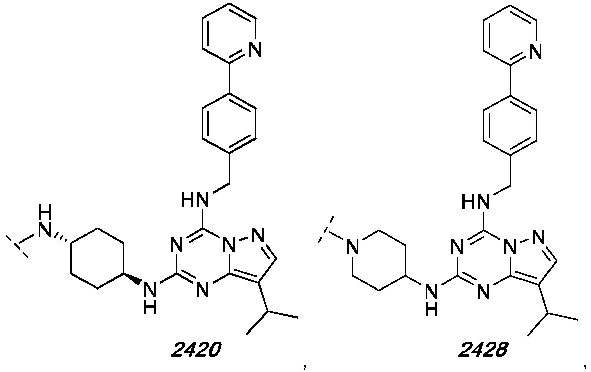

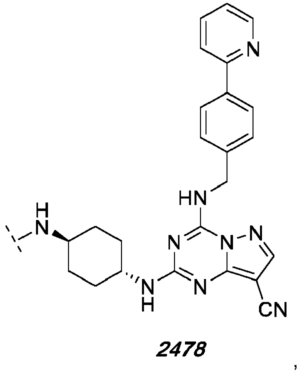

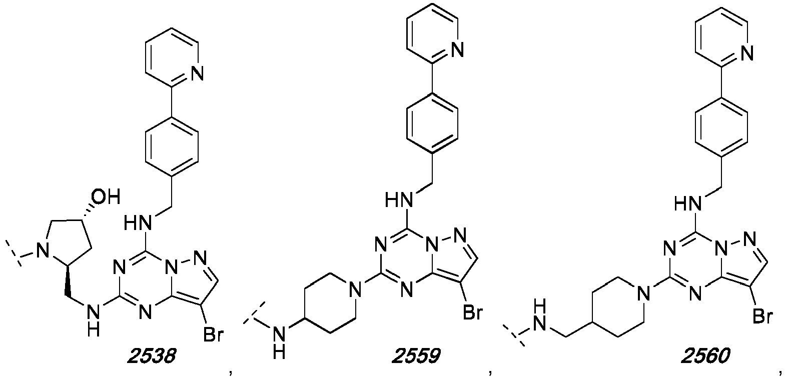

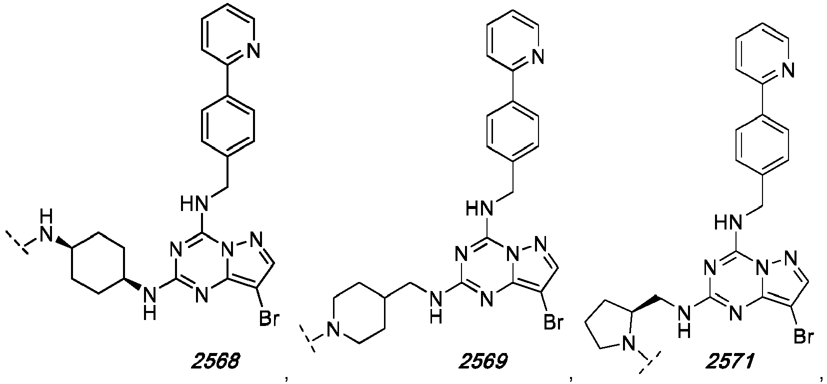

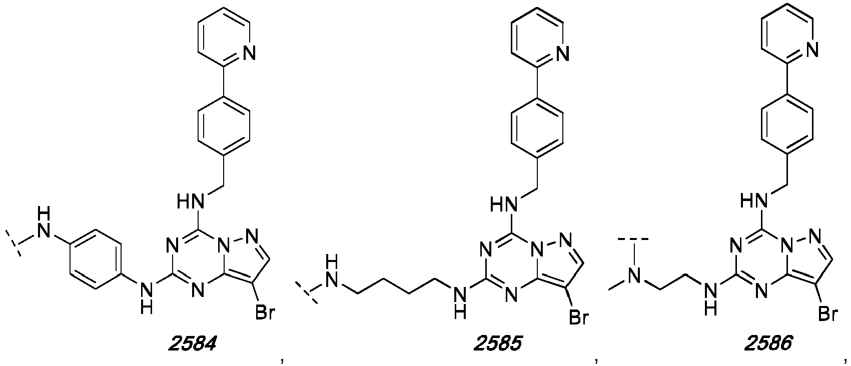

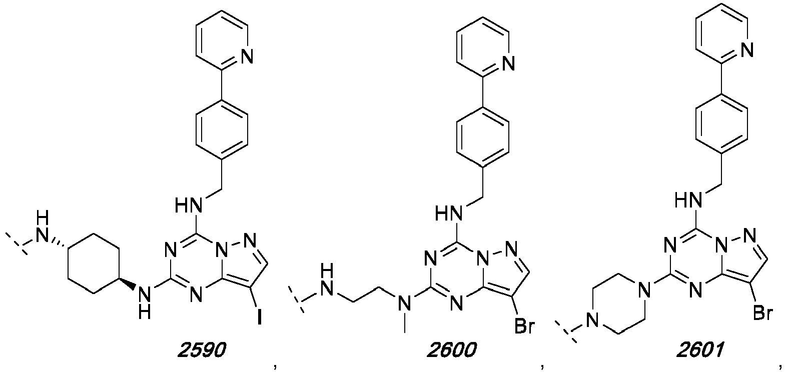

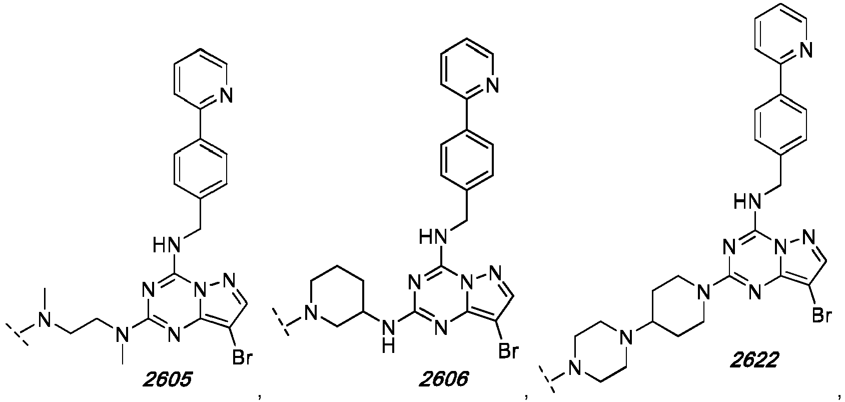

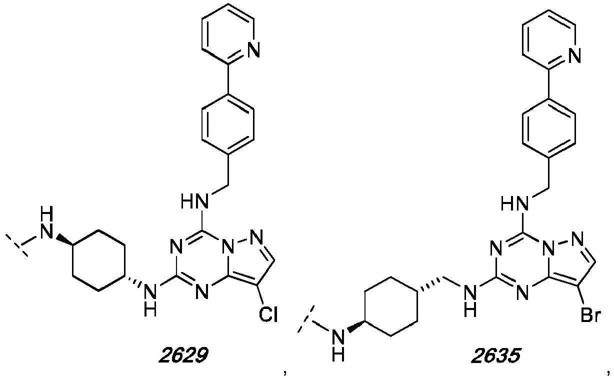

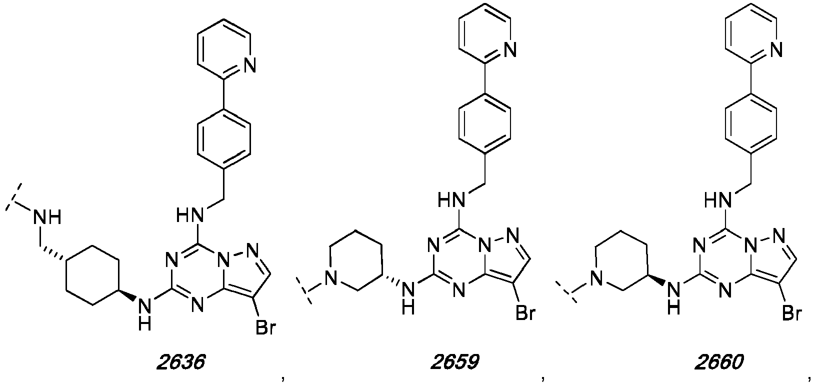

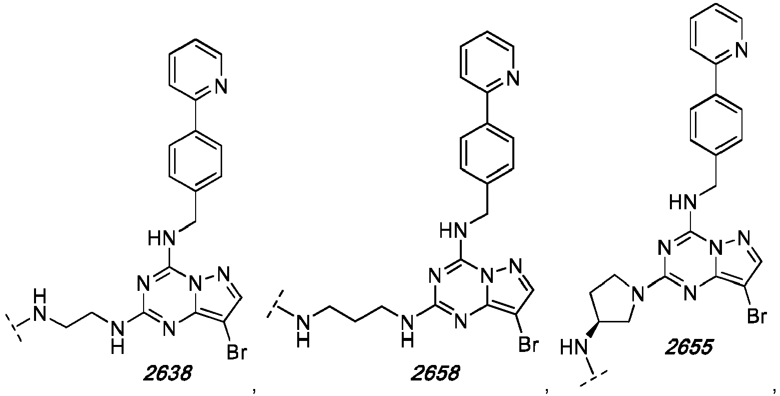

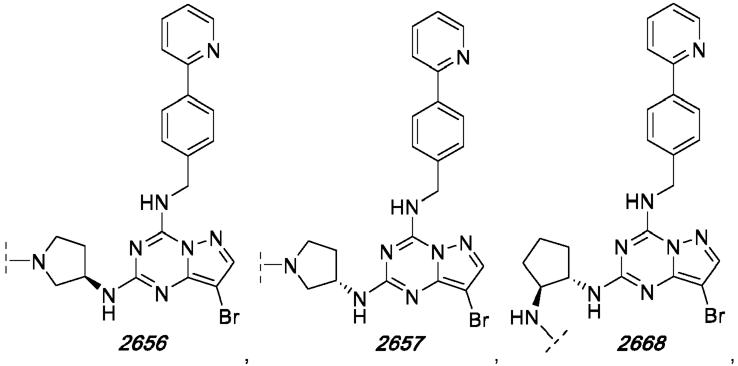

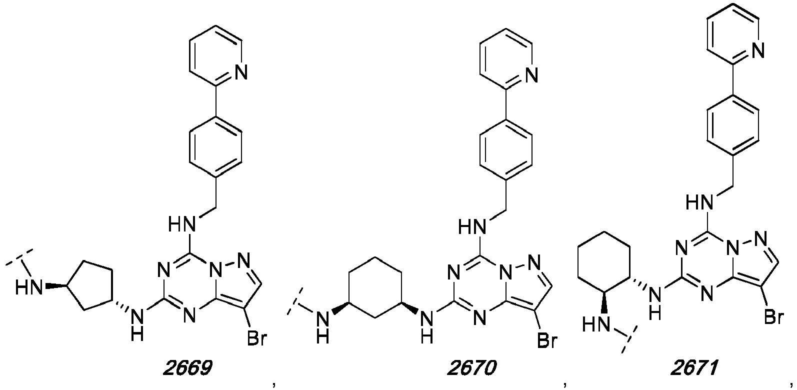

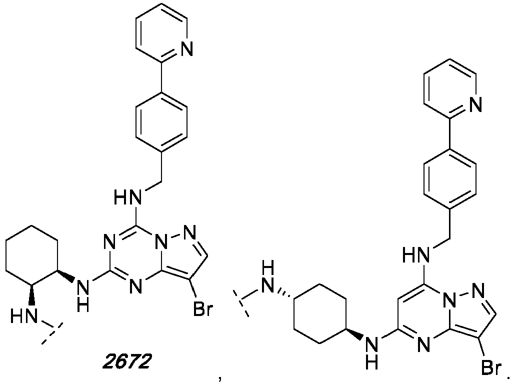

6. The antibody-drug conjugate according to any one of claims 1-5 wherein L is a cleavable linker moiety of formula -A-W-, wherein A is an optional stretcher unit linked to Ab, and W is a cleavable moiety linked to D.

7. The antibody-drug conjugate according to claim 6, wherein W is of the following formula (VI) 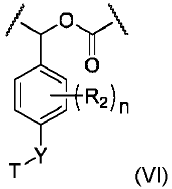 wherein
each R₂ is independently selected from the group consisting electron-withdrawing groups and C1-C4 alkyl;
n is 0, 1 or 2 ;
T is a sugar cleavable unit or a polypeptide cleavable unit;
Y is O when T is a sugar cleavable unit, or NR₃ when T is a polypeptide cleavable unit; and
R3 is selected from the group consisting of H, C1-C24 alkyl, C2-C6 alkenyl; optionally substituted polyether, aryl having 6 to 10 ring atoms, C3-C8 cycloalkyl, heterocycloalkyl having 3 to 10 ring atoms, heteroaryl having 5 to 10 ring atoms, and any combination thereof,
said alkyl and alkenyl being optionally interrupted by one or more heteroatom or chemical groups selected from -O-, -S-, -C(O)-, -NR"-, -C(O)NR"-, -NR"-C(O)-, -NR"-C(O)-NR‴-, -NR"-C(O)-O-, -O-C(O)NR"- and triazole,
R" and R‴ being independently selected from H and C1-C6 alkyl,
and pharmaceutically acceptable salts thereof.

8. The antibody-drug conjugate according claim 7, wherein L corresponds to a linker -A-W-of formula (VII) 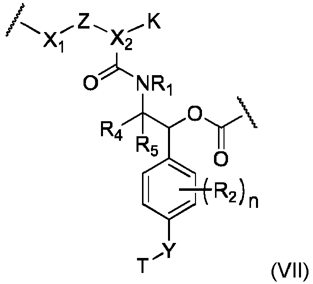 wherein
X₁ is a connector unit;
Z is an optional spacer;
X₂ is a connector unit;
K is an optional hydrophobicity masking entity, preferably selected from polysarcosine and polyethylene glycol;
R₁ is selected from the group consisting of H, C1-C24 alkyl, C₂-C₆ alkenyl; optionally substituted polyether, aryl having 6 to 10 ring atoms, C3-C8 cycloalkyl, heterocycloalkyl having 3 to 10 ring atoms, heteroaryl having 5 to 10 ring atoms, and any combination thereof,
said alkyl and alkenyl being optionally interrupted by one or more heteroatom or chemical groups selected from -O-, -S-, -C(O)-, -NR"-, -C(O)NR"-, -NR"-C(O)-, -NR"-C(O)-NR‴-, -NR"-C(O)-O-, -O-C(O)NR"- and triazole;
R₄ is selected from the group consisting H, C1-C6 alkyl and C2-C6 alkenyl, said alkyl and alkenyl being optionally interrupted by one or more heteroatom or chemical groups selected from -O-, -S-, -C(O)-, and -NR"-; and
R₅ is selected from the group consisting H, C₁-C₆ alkyl and C₂-C₆ alkenyl, said alkyl and alkenyl being optionally interrupted by one or more heteroatom or chemical groups selected from -O-, -S-, -C(O)-, and -NR"-.

9. The antibody-drug conjugate according to claim 8, wherein K is a polysarcosine, preferably of the following formula (VIII) 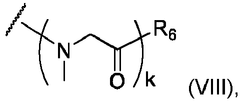 wherein k is an integer between 2 and 50, preferably between 4 and 30, and R₆ corresponds to OH or NH₂.

10. The antibody-drug conjugate according to any one of claims 7-9 wherein T is a sugar cleavable unit which is a glucuronide or a galactoside.

11. The antibody-drug conjugate according to any of claims 7-9, wherein T is a dipeptide, preferably selected from Val-Cit, Val-Ala and Phe-Lys.

12. Pharmaceutical composition comprising an antibody-drug conjugate compound according to any of claims 1 to 11 and a pharmaceutically acceptable carrier.

13. The antibody-drug conjugate according to any of claims 1 to 11, or the pharmaceutical composition according to claim 12, for use as a drug.

14. The antibody-drug conjugate according to any of claims 1 to 11, or the pharmaceutical composition according to claim 12, for use in the treatment of cancer.
